# EUROPEAN PATENT APPLICATION

(11) **EP 3 623 471 A1**
(43) Date of publication of application: **18.03.2020**
(21) Application number: 18797661.8
(22) Date of filing: 11.05.2018
(51) Int. Cl.: C12N 15/09, C12N 1/21, C12P 17/12

(54) **RECOMBINANT MICROORGANISM, AND PRODUCTION METHOD OF PYRIDOXAMINE, OR SALT THEREOF, USING SAID RECOMBINANT MICROORGANISM**

(30) Priority: 12.05.2017 JP 2017095572
(71) Applicant: Mitsui Chemicals, Inc., Minato-ku Tokyo 105-7122 (JP); Renascience Co., Ltd., Tokyo 105-7117 (JP)
(72) Inventor: TATENO, Toshihiro, Mobara-shi Chiba 297-0017 (JP); HIDESAKI, Tomonori, Omuta-shi Fukuoka 836-8610 (JP); ARAKI, Tadashi, Mobara-shi Chiba 297-0017 (JP); SHINDO, Atsunori, Tokyo 105-7122 (JP); MATSUMOTO, Yoshiko, Mobara-shi Chiba 297-0017 (JP); MIYATA, Toshio, Nagoya-shi Aichi 464-0827 (JP); HONJO, Masaru, Mobara-shi Chiba 297-0017 (JP)
(74) Representative: Mewburn Ellis LLP
(86) International application number: PCT/JP2018/018412
(87) International publication number: WO 2018/207930

(57) **Abstract**

Provided are a recombinant microorganism comprising: a gene encoding a pyridoxine oxidase; and a gene encoding a pyridoxamine synthetase having an enzymatic activity of synthesizing pyridoxamine from pyridoxal, in which each of the gene encoding a pyridoxine oxidase and the gene encoding a pyridoxamine synthetase is introduced from outside of a bacterial cell, or is endogenous to the bacterial cell and has an enhanced expression, and a method of producing pyridoxamine or a salt thereof from pyridoxine or a salt thereof using the recombinant microorganism.

## Description

### Technical Field

The present disclosure relates to a recombinant microorganism that can produce pyridoxamine or a salt thereof, and a method of producing pyridoxamine or a salt thereof using the recombinant microorganism.

### Background Art

Pyridoxamine and a salt thereof belong to vitamin B₆ family, and are known to have a glycation reaction inhibitory action. Pyridoxamine and a salt thereof, for example, inhibit accumulation of advanced glycation end products (AGEs) in the body which is involved in various aging processes. AGE is a generic term for substances produced by glycation of proteins, and AGE accumulation is believed to aggravate a disease such as diabetes mellitus, atherosclerosis, chronic renal failure, or Alzheimer's dementia. For this reason, pyridoxamine and a salt thereof are promising substances that are expected to make it possible to prevent or treat such a disease by preventing AGE accumulation.

Pyridoxamine and a salt thereof are also known to have activity as a schizophrenia drug, and various studies for their practical use have been made. In addition, development of health foods and cosmetics utilizing various physiological activities of pyridoxamine and a salt thereof is also in progress.

Pyridoxamine and a salt thereof can be synthesized chemically. For example, International Publication No. WO 2006/066806 discloses a method of chemically synthesizing pyridoxamine dihydrochloride using alanine and formic acid as starting materials. WO 2005/077902 discloses a method of chemically synthesizing pyridoxamine from pyridoxine.

On the other hand, biological synthesis of pyridoxamine is also being studied. WO 2007/142222 discloses a method of obtaining pyridoxamine from pyridoxal using a specific microorganism such as *Achromobacter.* WO 2007/142222 also discloses an experiment in which, by culturing *Acremonium fusidioides* in the presence of pyridoxine, a certain amount of the pyridoxine was converted to pyridoxal.

Japanese Patent Application Laid-Open (JP-A) No. H09-107985 discloses a method of producing vitamin B₆, in which a microorganism belonging to the genus *Rhizobium* and capable of producing vitamin B₆ is cultured under aerobic conditions in a culture medium, and produced vitamin B₆ is obtained from a culture solution.

As a study on vitamin B₆ synthesis, WO 2004/035010 discloses a method of producing vitamin B₆ by culturing an organism in which at least one of the activity of yaaD or yaaE of *Bacillus subtilis* is enhanced compared to the parent organism. Japanese Patent Application Laid-Open (JP-A) No. 2000-23690 describes a production of a vitamin B₆ mixture by using cell-free extract derived from *Rhizobium meliloti* IFO 14782. Journal of Molecular Catalysis B: Enzymatic, 2010, vol. 67, p. 104-110 discloses a pyridoxamine-pyruvate aminotransferase (PPAT) that is enabled to use L-glutamate by modifying its sequence. It is described that pyridoxamine was able to be produced by incubating *E. coli* expressing PPAT having such an modified sequence in the presence of a saturated amount of pyridoxal.

### SUMMARY OF INVENTION

### Technical Problem

However, high reaction yields have not been achieved by pyridoxamine synthesis by chemical synthesis. For example, in a method described in WO 2006/066806, pyridoxamine dihydrochloride is synthesized through many chemical reactions, which results in lower reaction yields. In a method described in WO 2005/077902, a dimer and a trimer of pyridoxamine are produced, and the reaction yield is not significantly high.

On the other hand, in a method described in WO 2007/142222, in which a specific kind of microorganism is used, the conversion efficiency from pyridoxal to pyridoxamine is varied widely among microbial species and is not significantly high in all. In a culture experiment of *Acremonium fusidioides* in the presence of pyridoxine described in WO 2007/142222, most of a product is pyridoxal rather than pyridoxamine. JP-A No. H09-107985 describes that most of produced vitamin B₆ was pyridoxol (pyridoxine), and production of pyridoxamine was slight.

WO 2004/035010 describes that vitamin B₆ is obtained by culturing in a culture medium a microorganism highly expressing the genes for yaaD and yaaE; however, the product is a mixture of pyridoxine, pyridoxal, pyridoxamine, pyridoxamine phosphate, and the like and pyridoxamine is not selectively obtained.

As described above, chemical synthesis of pyridoxamine or a salt thereof (such as WO 2006/066806 or WO 2005/077902) involves many reaction and purification steps, and high yields have not been achieved. Although some microorganisms have pyridoxamine-synthesizing ability (for example, WO 2007/142222 and JP-ANo. H09-107985), it takes time and effort to newly discover such microorganisms. In addition, selective synthesis of pyridoxamine or a salt thereof among the vitamin B₆ family has not been realized and high pyridoxamine production efficiency has not been achieved. The approach of screening specific microbial species from naturally occurring microbial species as described above does not provide molecular biological information as to which enzyme or gene is important for a high production of vitamin B₆.

For example, a method of producing vitamin B₆ using recombinant microorganisms produced by genetic modification technology is described (WO 2004/035010 and Journal of Molecular Catalysis B: Enzymatic, 2010, vol. 67, p. 104-110). However, in production of substances, vitamin B₆ is non-selectively obtained using a common culture medium (WO 2004/035010), or alternatively, a saturated amount of pyridoxal, which is an expensive raw material, is used to produce pyridoxamine (Journal of Molecular Catalysis B: Enzymatic, 2010, vol. 67, p. 104-110). Therefore, inexpensive and selective production of pyridoxamine or a salt thereof has not been achieved.

In view of the above, the disclosure provides a recombinant microorganism capable of inexpensively producing pyridoxamine or a salt thereof from pyridoxine or a salt thereof at high production efficiency, and a method of inexpensively producing pyridoxamine or a salt thereof from pyridoxine or a salt thereof at high production efficiency using the recombinant microorganism.

### Solution to Problem

The disclosure includes the following aspects.
<1> A recombinant microorganism, including:
   a gene encoding a pyridoxine oxidase, and a gene encoding a pyridoxamine synthetase having an enzymatic activity of synthesizing pyridoxamine from pyridoxal,
   wherein each of the gene encoding the pyridoxine oxidase and the gene encoding the pyridoxamine synthetase is introduced from outside of a bacterial cell, or is endogenous to the bacterial cell and has an enhanced expression.
<2> The recombinant microorganism according to <1>, wherein the pyridoxamine synthetase is a pyridoxamine-pyruvate transaminase, a pyridoxamine-oxaloacetate transaminase, an aspartate transaminase, or a pyridoxamine phosphate transaminase.
<3> The recombinant microorganism according to <1> or <2>, wherein the pyridoxine oxidase is represented by the enzyme number EC 1.1.3.12.
<4> The recombinant microorganism according to any one of <1> to <3>, wherein the gene encoding the pyridoxine oxidase is derived from *Microbacterium luteolum.*
<5> The recombinant microorganism according to any one of <1> to <4>, wherein the gene encoding a pyridoxine oxidase:
   (a) has a nucleotide sequence of SEQ ID NO:5,
   (b) has a nucleotide sequence that hybridizes with DNA having a nucleotide sequence complementary to the nucleotide sequence of SEQ ID NO:5 under a stringent condition, and that encodes a protein having pyridoxine oxidase activity,
   (c) has a nucleotide sequence encoding a protein that has an amino acid sequence of SEQ ID NO:1, or
   (d) has a nucleotide sequence encoding a protein that has an amino acid sequence having 80% or more sequence identity with the amino acid sequence of SEQ ID NO:1 and that has pyridoxine oxidase activity.
<6> The recombinant microorganism according to any one of <1> to <5>, wherein the pyridoxamine synthetase includes at least one of the following partial amino acid sequence (c), partial amino acid sequence (d), partial amino acid sequence (e), partial amino acid sequence (f), partial amino acid sequence (g), or partial amino acid sequence (h), and has an enzymatic activity of synthesizing pyridoxamine from pyridoxal:
   (c) X₈X₉X₁₀X₁₁X₁₂X₁₃ (SEQ ID NO:39)
      wherein X₈ represents L, M, I or V,
      X₉ represents H or Q,
      X₁₀ represents G, C or A,
      X₁₁ represents E or D,
      X₁₂ represents P or A, and
      X₁₃ represents V, I, L or A;
   (d) X₁₄X₁₅TPSGTX₁₆X₁₇ (SEQ ID NO:40)
      wherein X₁₄ represents H or S,
      X₁₅ represents D or E,
      X₁₆ represents I, V, or L, and
      X₁₇ represents N or T;
      (e) X₁₈DX_{19V}SX₂₀X₂₁ (SEQ ID NO:41)
      wherein X₁₈ represents V, I, or A,
      X₁₉ represents A, T, or S,
      X₂₀ represents S, A, or G, and
      X₂₁ represents F, W, or V;
   (f) X₂₂X₂₃X₂₄KCX₂₅GX₂₆X₂₇P (SEQ ID NO:42)
      wherein X₂₂ represents G or S,
      X₂₃ represents P, S, or A,
      X₂₄ represents N, G, S, A, or Q,
      X₂₅ represents L or M,
      X₂₆ represents A, S, C, or G, and
      X₂₇ represents P, T, S, or A;
   (g) X₂₈X₂₉X₃₀X₃₁SX₃₂GX₃₃X₃₄ (SEQ ID NO:43)
      wherein X₂₈ represents G or D,
      X₂₉ represents V or I,
      X₃₀ represents V, T, A, S, M, I, or L,
      X₃₁ represents F, M, L, I, or V,
      X₃₂ represents S, G, A, T, I, L, or H,
      X₃₃ represents R, M, or Q, and
      X₃₄ represents G, R, A, D, H, or K; and
   (h) X₃₅X₃₆RX₃₇X₃₈HMGX₃₉X₄₀A (SEQ ID NO:44)
      wherein X₃₅ represents L or V,
      X₃₆ represents T, I, V, or L,
      X₃₇ represents I, V, or L,
      X₃₈ represents G or S,
      X₃₉ represents P, A, or R, and
      X₄₀ represents T, V, or S.
<7> The recombinant microorganism according to any one of <1> to <6>, wherein the pyridoxamine synthetase is represented by the enzyme number EC 2.6.1.30.
<8> The recombinant microorganism according to any one of <1> to <7>, wherein the gene encoding a pyridoxamine synthetase is derived from *Mesorhizobium loti.*
<9> The recombinant microorganism according to any one of <1> to <8>, wherein the gene encoding a pyridoxamine synthetase:
   (a) has a nucleotide sequence of any one of SEQ ID NO:6 or SEQ ID NO:25 to SEQ ID NO:31, or
      has a region between an 18th nucleotide and a 3' end in a nucleotide sequence of SEQ ID NO:10, or a region between an 18th nucleotide and a 3' end in a nucleotide sequence of any one of SEQ ID NO:32 to SEQ ID NO:38;
   (b) has a nucleotide sequence that hybridizes with DNA having a nucleotide sequence complementary to a nucleotide sequence of any one of SEQ ID NO:6 or SEQ ID NO:25 to SEQ ID NO:31, or with DNA having a nucleotide sequence complementary to a region between a 18th nucleotide and a 3' end in a nucleotide sequence of SEQ ID NO:10 or a region between a 18th nucleotide and a 3' end in a nucleotide sequence of any one of SEQ ID NO:32 to SEQ ID NO:38 under a stringent condition, and that encodes a protein having an enzymatic activity of synthesizing pyridoxamine from pyridoxal;
   (c) has a nucleotide sequence encoding a protein that has an amino acid sequence of any one of SEQ ID NO:2 or SEQ ID NO:18 to SEQ ID NO:24; or
   (d) has a nucleotide sequence encoding a protein that has an amino acid sequence having 80% or more sequence identity with at least one amino acid sequence selected from the group consisting of SEQ ID NO:2 and SEQ ID NO:18 to SEQ ID NO:24, and that has an enzymatic activity of synthesizing pyridoxamine from pyridoxal.
<10> The recombinant microorganism according to any one of <1> to <9>, wherein the gene encoding a pyridoxamine synthetase:
   (a) has a nucleotide sequence of SEQ ID NO:6;
   (b) has a nucleotide sequence that hybridizes with DNA having a nucleotide sequence complementary to the nucleotide sequence of SEQ ID NO:6 under a stringent condition, and that encodes a protein having an enzymatic activity of synthesizing pyridoxamine from pyridoxal;
   (c) has a nucleotide sequence encoding a protein that has an amino acid sequence of SEQ ID NO:2; or
   (d) has a nucleotide sequence encoding a protein that has an amino acid sequence having 80% or more sequence identity with the amino acid sequence of SEQ ID NO:2, and that has an enzymatic activity of synthesizing pyridoxamine from pyridoxal.
<11> The recombinant microorganism according to any one of <1> to <5>, wherein the pyridoxamine synthetase is represented by the enzyme number EC 2.6.1.31 or EC 2.6.1.1.
<12> The recombinant microorganism according to any one of <1> to <5> or <11>, wherein the gene encoding a pyridoxamine synthetase is derived from *Escherichia coli.*
<13> The recombinant microorganism according to any one of <1> to <5> or <11> to <12>, wherein the gene encoding the pyridoxamine synthetase:
   (a) has a nucleotide sequence of SEQ ID NO:8;
   (b) has a nucleotide sequence that hybridizes with DNA having a nucleotide sequence complementary to the nucleotide sequence of SEQ ID NO:8 under a stringent condition, and that encodes a protein having an enzymatic activity of synthesizing pyridoxamine from pyridoxal;
   (c) has a nucleotide sequence encoding a protein that has an amino acid sequence of SEQ ID NO:4; or
   (d) a nucleotide sequence encoding a protein that has an amino acid sequence having 80% or more sequence identity with the amino acid sequence of SEQ ID NO:4, and that has an enzymatic activity of synthesizing pyridoxamine from pyridoxal.
<14> The recombinant microorganism according to any one of <1> to <13>, further including a gene encoding a hydrogen peroxide-degrading enzyme that has an enzymatic activity of generating oxygen from hydrogen peroxide.
<15> The recombinant microorganism according to <14>, wherein the gene encoding the hydrogen peroxide-degrading enzyme is introduced from outside of a bacterial cell, or is endogenous to a bacterial cell and has an enhanced expression.
<16> The recombinant microorganism according to <14> or <15>, wherein the hydrogen peroxide-degrading enzyme is represented by the enzyme number EC 1.11.1.6.
<17> The recombinant microorganism according to any one of <14> to <16>, wherein the gene encoding a hydrogen peroxide-degrading enzyme:
   (a) has a nucleotide sequence of SEQ ID NO:7;
   (b) hybridizes with DNA having a nucleotide sequence complementary to the nucleotide sequence of SEQ ID NO:7 under a stringent condition, and has an enzymatic activity of generating oxygen from hydrogen peroxide;
   (c) has a nucleotide sequence encoding a protein that has an amino acid sequence of SEQ ID NO:3; or
   (d) has a nucleotide sequence encoding a protein that has an amino acid sequence having 80% or more sequence identity with the amino acid sequence of SEQ ID NO:3, and that has an enzymatic activity of generating oxygen from hydrogen peroxide.
<18> The recombinant microorganism according to any one of <1> to <17>, including a recombinant *E. coli.*
<19> A method of producing pyridoxamine or a salt thereof, the method including bringing the recombinant microorganism according to any one of <1> to <18>, a culture of the recombinant microorganism, or a treated product of the recombinant microorganism or the culture, into contact with pyridoxine or a salt thereof to produce pyridoxamine or a salt thereof in the presence of oxygen.
<20> The production method according to <19>, wherein the recombinant microorganism, the culture of the recombinant microorganism, or the treated product of the recombinant microorganism or the culture, includes the pyridoxine oxidase and the pyridoxamine synthetase.
<21> The production method according to <20>, wherein the recombinant microorganism, the culture of the recombinant microorganism, or the treated product of the recombinant microorganism or the culture, further includes a hydrogen peroxide-degrading enzyme.
<22> The production method according to any one of <19> to <21>, wherein the treated product of the recombinant microorganism or the culture is a product treated by treatment including one or more selected from the group consisting of heat treatment, cooling treatment, mechanical destruction of a cell, ultrasonic treatment, freeze-thaw treatment, drying treatment, pressurized or reduced pressure treatment, osmotic pressure treatment, cell autolysis, surfactant treatment, enzyme treatment, cell separation treatment, purification treatment, and extraction treatment.
<23> The production method according to any one of <19> to <22>, the method including either or both of the following (A) and (B):
   (A) adding pyridoxine or a salt thereof continuously or in several batches to a solution including the recombinant microorganism, or the culture of the recombinant microorganism, or the treated product of the recombinant microorganism or the culture; and
   (B) controlling a molar concentration of an amino acid consumed by the pyridoxamine synthetase so as to be 1 or more times a molar concentration of pyridoxine or a salt thereof, in a solution including the recombinant microorganism, the culture of the recombinant microorganism, or the treated product of the recombinant microorganism or the culture.
<24> The production method according to <23>, wherein the amino acid consumed by the pyridoxamine synthetase is L-alanine, D-alanine, L-glutamic acid, or D-glutamic acid. Advantageous Effects of Invention

According to the disclosure, a recombinant microorganism capable of inexpensively producing pyridoxamine or a salt thereof from pyridoxine or a salt thereof at high production efficiency, and a method of inexpensively producing pyridoxamine or a salt thereof from pyridoxine or a salt thereof at high production efficiency using the recombinant microorganism are provided.

### BRIEF DESCRIPTION OF DRAWINGS

Fig. 1 shows results of measurement of pyridoxine hydrochloride concentration, pyridoxal hydrochloride concentration, and pyridoxamine dihydrochloride concentration in Test 7.
Fig. 2 shows results of measurement of pyridoxine hydrochloride concentration, pyridoxal hydrochloride concentration, and pyridoxamine dihydrochloride concentration in Test 8.
Fig. 3 shows results of measurement of pyridoxine hydrochloride concentration, pyridoxal hydrochloride concentration, and pyridoxamine dihydrochloride concentration in Test 9.
Fig. 4-1 shows alignment of sequences of SEQ ID NO:2 and SEQ ID NO:18 to SEQ ID NO:24.
Fig. 4-2 shows alignment of sequences of SEQ ID NO:2 and SEQ ID NO:18 to SEQ ID NO:24.

### DESCRIPTION OF EMBODIMENTS

The disclosure provides a recombinant microorganism (hereinafter referred to as a recombinant microorganism according to the disclosure), including:
a gene encoding a pyridoxine oxidase; and a gene encoding a pyridoxamine synthetase having an enzymatic activity of synthesizing pyridoxamine from pyridoxal,
wherein each of the gene encoding the pyridoxine oxidase and the gene encoding the pyridoxamine synthetase is introduced from outside of a bacterial cell, or is endogenous to the bacterial cell and has an enhanced expression. In the disclosure, "introduction" refers to introduction in such a manner to allow expression in a bacterial cell.

Until now, it has not been known a method for inexpensively producing pyridoxamine or a salt thereof from pyridoxine or a salt thereof at high production efficiency either by a chemical method or a biological method. The structure of pyridoxamine is shown below.

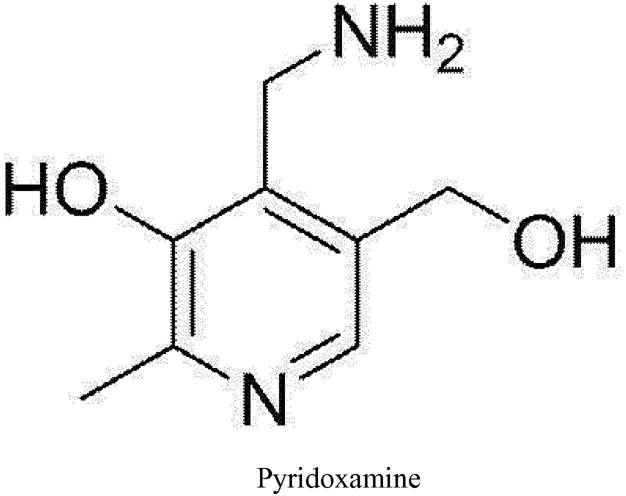

However, the present inventors have surprisingly found that pyridoxamine or a salt thereof can be inexpensively produced from pyridoxine or a salt thereof at high production efficiency by using a recombinant microorganism having above described constitution or a culture of the recombinant microorganism, or a treated product of the recombinant microorganism or the culture. Although the reason for this is not necessarily clear, it is assumed that since each of the genes encoding the above two kinds of enzymes is introduced from outside of a bacterial cell, or is endogenous to the bacterial cell and has an enhanced expression, the enzyme can be expressed at a high expression level from the introduced or enhanced gene. Further, it is assumed that due to the cooperative action of the above two kinds of enzymes, equilibrium of each reaction in a process of producing pyridoxamine or a salt thereof from pyridoxine or a salt thereof and the amount of a by-product or the like produced in this process or the amount of a raw material or the like consumed in this process advantageously affect the production of pyridoxamine or a salt thereof from pyridoxine or a salt thereof.

Furthermore, in a case in which the recombinant microorganism, the culture of the recombinant microorganism, or the treated product of the recombinant microorganism or the culture according to the disclosure is used, it is assumed that pyridoxal is temporarily produced as an intermediate product. However, since pyridoxal is an aldehyde and is highly reactive, pyridoxal spontaneously reacts with an amino group donor such as alanine at a near-neutral pH even in the absence of a catalyst such as an enzyme to produce pyridoxamine and a by-product. In a spontaneous reaction of pyridoxal, it is assumed that the production efficiency of pyridoxamine is not to be high because a by-product is produced and the selectivity of pyridoxamine production is not high. In contrast, in a case in which the recombinant microorganism, the culture of the recombinant microorganism, or the treated product of the recombinant microorganism or the culture according to the disclosure is used, pyridoxal an intermediate is rapidly converted to pyridoxamine by a pyridoxamine synthetase, accumulation of pyridoxal is suppressed, and production of a by-product is also suppressed. As a result, high pyridoxamine production efficiency can be achieved.

Such a cooperative action of the above-described two kinds of enzymes, which has not been found so far, allows the production of pyridoxamine or a salt thereof with high production efficiency by using pyridoxine or a salt thereof, without the need to carry out a multi-step reaction as in a chemical synthesis method. Moreover, by using the recombinant microorganism according to the disclosure, it is possible to avoid by-production of large amounts of other substances included in the vitamin B₆ complex. Pyridoxine is a raw material which is industrially available at a low cost compared to pyridoxal. By using pyridoxine as a starting material, pyridoxamine or a salt thereof can be inexpensively produced.

### <Pyridoxine Oxidase>

Pyridoxine oxidase is an enzyme also called pyridoxine-4-oxidase. The pyridoxine oxidase may be an enzyme represented by the enzyme number EC1.1.3.12. Pyridoxine oxidase is an enzyme having an enzymatic activity that catalyzes a conversion reaction to pyridoxal by oxidizing pyridoxine with oxygen. Although pyridoxal or pyridoxine may also exist as a salt depending on a surrounding environment; however, in the disclosure, a description of enzymatic activity is given with the omission of the mention of a salt in order to simplify an expression. Pyridoxine oxidase consumes oxygen to produce hydrogen peroxide when oxidizing pyridoxine. Due to production of hydrogen peroxide which is harmful to organisms, it has been considered that it is possible to achieve a high yield when pyridoxine oxidase is used to produce another substance from pyridoxine. However, when the recombinant microorganism according to the disclosure is used, an unexpected effect of achieving the production of pyridoxamine or a salt thereof from pyridoxine or a salt thereof as a raw material at high production efficiency can be obtained.

The pyridoxine oxidase of EC1.1.3.12 may be a pyridoxine oxidase derived from, for example, *Enterobacter cloacae, Mesorhizobium loti, Microbacterium luteolum, Ochrobactrum anthropi, Pseudomonas sp.* MA-1, or the like. The pyridoxine oxidase from *Microbacterium luteolum* has the amino acid sequence of SEQ ID NO:1.

### <Pyridoxamine Synthetase>

The pyridoxamine synthetase used in the disclosure refers to any enzyme having an enzymatic activity of synthesizing pyridoxamine from pyridoxal. Pyridoxal or pyridoxamine may also exist as a salt depending on a surrounding environment; however, in the disclosure, a description of enzymatic activity is given with the omission of the mention of a salt in order to simplify an expression. Examples of the pyridoxamine synthetase include, for example, a pyridoxamine-pyruvate transaminase represented by the enzyme number EC 2.6.1.30, a pyridoxamine-oxaloacetate transaminase represented by EC 2.6.1.31, an aspartate transaminase represented by EC 2.6.1.1, and a pyridoxamine phosphate transaminase represented by EC 2.6.1.54.

The aspartate transaminase represented by EC 2.6.1.1 is a holoenzyme using pyridoxal phosphate as a coenzyme, and has an enzymatic activity of produce glutamic acid and oxaloacetic acid by transferring the amino group of aspartic acid to 2-oxoglutaric acid. The aspartate transaminase in a state of an apoenzyme not bound to pyridoxal phosphate is known synthesize pyridoxamine by transferring the amino group of glutamic acid or aspartic acid to pyridoxal (Journal of Biological Chemistry, January 1962, Vol. 237, No. 1, p. 127-132). In other words, an apoenzyme form of the aspartate transaminase represented by EC 2.6.1.1 is the pyridoxamine-oxaloacetate transaminase of EC 2.6.1.31. For this reason, aspartate transaminase exists as an apoenzyme when a coenzyme pyridoxal phosphate is absent or present in a small amount, and synthesizes pyridoxamine.

The pyridoxamine synthetase has an enzymatic activity of producing pyridoxamine or a salt thereof by oxidizing an amino group moiety of a specific amino acid to (=O) and transferring the amino group in the synthesis of pyridoxamine or a salt thereof from pyridoxal or a salt thereof. For example, the pyridoxamine-pyruvate transaminase can use both L-alanine and D-alanine, each of the pyridoxamine-oxaloacetate transaminase and the aspartate transaminase in an apoenzyme state can use D-aspartic acid, L-aspartic acid, D-glutamic acid, and L-glutamic acid, and the pyridoxamine phosphate transaminase can use D-glutamic acid.

The pyridoxamine-pyruvate transaminase may be derived from, for example, a microorganism belonging to the phylum *Proteobacteria,* a microorganism belonging to the phylum *Actinobacteria,* a microorganism belonging to the phylum *Spirocheta,* or a microorganism belonging to the phylum *Filmictes.* The pyridoxamine-pyruvate transaminase may be a pyridoxamine-pyruvate transaminase derived from, for example, *Mesorhizobium loti, Ochrobactrum anthropi,* or the genus *Pseudomonas* (such as *Pseudomonas* sp. MA-1). Here, the pyridoxamine-pyruvate transaminase from *Mesorhizobium loti* has the amino acid sequence of SEQ ID NO:2, for example.

The pyridoxamine-pyruvate transaminase may also be, for example, a pyridoxamine-pyruvate transaminase (having the amino acid sequence of SEQ ID NO:18) derived from *Mesorhizobium* sp. YR577, a pyridoxamine-pyruvate transaminase (having the amino acid sequence of SEQ ID NO:19) derived from *Pseudaminobacter salicylatoxidans,* a pyridoxamine-pyruvate transaminase (having the amino acid sequence of SEQ ID NO:20) derived from *Bauldia litoralis,* a pyridoxamine-pyruvate transaminase (having the amino acid sequence of SEQ ID NO:21) derived from *Skermanella stibiiresistens,* a pyridoxamine-pyruvate transaminase (having the amino acid sequence of SEQ ID NO:22) derived from *Rhizobium* sp. AC44/96, a pyridoxamine-pyruvate transaminase (having the amino acid sequence of SEQ ID NO:23) derived from *Erwinia toletana,* or a pyridoxamine-pyruvate transaminase (having the amino acid sequence of SEQ ID NO:24) derived from *Herbiconiux ginsengi.*

The pyridoxamine-oxaloacetate transaminase may be, for example, a pyridoxamine-oxaloacetate transaminase derived from *Escherichia coli, Oryctolagus cuniculus,* or *Rattus norvegicus.* For example, the pyridoxamine-oxaloacetate transaminase from *Escherichia coli* has the amino acid sequence of SEQ ID NO:4. The aspartate transaminase may be an aspartate transaminase derived from, for example, *Escherichia coli, Trichoderma viride,* or the like. The pyridoxamine phosphate transaminase may be a pyridoxamine phosphate transaminase derived from, for example, *Clostridium butyricum.*

### <Hydrogen Peroxide-Degrading Enzyme>

The recombinant microorganism according to the disclosure may further include a gene encoding a hydrogen peroxide-degrading enzyme having an enzymatic activity of degrading hydrogen peroxide. The hydrogen peroxide-degrading enzyme used in the disclosure refers to any enzyme having an enzymatic activity of degrading hydrogen peroxide generated when the pyridoxine oxidase oxidizes pyridoxine. In the case of further including the gene encoding the hydrogen peroxide-degrading enzyme, accumulation of hydrogen peroxide that is harmful to organisms and enzymatic activity can be further reduced, which is advantageous in terms of further improving the production efficiency of pyridoxamine or a salt thereof and prolonging the duration of a reaction. The hydrogen peroxide-degrading enzyme may have an enzymatic activity of regenerating oxygen. The presence of the enzymatic activity of regenerating oxygen can increase the concentration of oxygen in a reaction system, particularly when producing pyridoxamine or a salt thereof in a low oxygen environment, and is advantageous from the viewpoint of further improving the production efficiency of pyridoxamine or a salt thereof.

Examples of such hydrogen peroxide-degrading enzymes include an enzyme represented by the enzyme number (EC) 1.11.1.1, 1.11.1.2, 1.11.1.3, 1.11.1.5, 1.11.1.6, 1.11.1.7, 1.11.1.8, 1.11.1.9, 1.11.1.10, 1.11.1.11, 1.11.1.13, 1.11.1.14, 1.11.1.16, 1.11.1.17, 1.11.1.18, 1.11.1.19, 1.11.1.21, or 1.11.1.23. Among them, a catalase represented by the enzyme number EC 1.11.1.6 and a catalase peroxidase represented by EC 1.11.1.21 are preferable from the viewpoint of the oxygen regenerating ability, and a catalase represented by the enzyme number EC 1.11.1.6 is more preferable.

The hydrogen peroxide-degrading enzyme may be a catalase derived from, for example, *Listeria seeligeri, Escherichia coli*) or *Saccharomyces cerevisiae.* Alternatively, the hydrogen peroxide-degrading enzyme may be a catalase peroxidase derived from, for example, *Escherichia coli.* For example, the catalase from *Listeria seeligeri* has the amino acid sequence of SEQ ID NO:3.

Each of the pyridoxine oxidase, the pyridoxamine synthetase, and the hydrogen peroxide-degrading enzyme may be a protein that has a known amino acid sequence (such as an amino acid sequence encoded by a gene naturally occurring in an organism, or an amino acid sequence encoded by a gene possessed by a naturally occurring microorganism such as the above-described microorganism) having the enzymatic activity and that is unmodified, or may be a protein that has an amino acid sequence obtained by modifying such an amino acid sequence as long as the activity of the enzymatic activity (such as the above-described enzymatic activity) is not lost. Examples of the modification include insertion, deletion, substitution of an amino acid residue, and addition of an additional amino acid residue to either or both of the N-terminus and the C-terminus of an amino acid sequence. In a case in which there is one or more of the insertion, deletion, and substitution of the amino acid residue, the number of each of insertion, deletion, and substitution, if present, may be, for example, from 1 to 30 amino acid residues, from 1 to 20 amino acid residues, from 1 to 10 amino acid residues, or from 1 to 5 amino acid residues; and the total number of insertion, deletion, and substitution of the amino acid residue may be, for example, from 1 to 50 amino acid residues, from 1 to 30 amino acid residues, from 1 to 10 amino acid residues, or from 1 to 5 amino acid residues. The number of amino acid residue added to the terminus, if present, may be, for example, from 1 to 50 amino acid residues, from 1 to 30 amino acid residues, from 1 to 10 amino acid residues, or from 1 to 5 amino acid residues per one terminus. The additional amino acid residue may form a signal sequence for extracellular secretion or the like. Examples of the signal sequence include an *E. coli* OmpA signal sequence.

Alternatively, each of the enzymes may be a protein having a known amino acid sequence (such as an amino acid sequence encoded by a gene naturally occurring in an organism) having the enzymatic activity per se, or a protein having an amino acid sequence that has 80% or more, 85% or more, 90% or more, or 95% or more sequence identity with a known amino acid sequence (such as an amino acid sequence encoded by a gene naturally occurring in an organism) having the enzymatic activity and that has a desired enzymatic activity (such as the above-mentioned enzymatic activity). Here, the sequence identity can be evaluated by using, for example, a BLAST (registered trademark, National Library of Medicine) program with default parameters.

For example, the pyridoxine oxidase may be, for example, a protein having the amino acid sequence of SEQ ID NO:1, or may be a protein having an amino acid sequence in which one or more of the substitution, deletion, or the insertion of an amino acid residue, or addition of an additional amino acid residue to either or both of the N-terminus and C-terminus of the amino acid sequence are performed in the amino acid sequence of SEQ ID NO:1. Examples of the degree of the substitution, deletion, and insertion of an amino acid residue, and the addition of an additional amino acid residue to either or both of the N-terminus and the C-terminus of the amino acid sequence are as described above.

Alternatively, the pyridoxine oxidase may be a protein having the amino acid sequence of SEQ ID NO:1, or may be a protein having an amino acid sequence having, for example, 80% or more, 85% or more, 90% or more, or 95% or more sequence identity with the amino acid sequence of SEQ ID NO:1.

In a case of using such a protein having an amino acid sequence similar to the amino acid sequence of SEQ ID NO:1 as described above, it is necessary that the protein has an activity for a pyridoxine oxidase. The pyridoxine oxidase activity can be measured by, for example, adding a protein to be tested to an aqueous solution including pyridoxine as a substrate in the presence of oxygen, and quantifying the amount of produced pyridoxal by high performance liquid chromatography, or forming a Schiff base between produced pyridoxal and an amine such as trishydroxymethylaminomethane and quantifying the amount of the Schiff base with absorbance measurement at 415 nm or the like.

Alternatively, the pyridoxamine synthetase may be a protein having the amino acid sequence of any one of SEQ ID NO:2 or SEQ ID NO:18 to SEQ ID NO:24, or a protein having an amino acid sequence having, for example, 80% or more, 85% or more, 90% or more, or 95% or more sequence identity with at least one of the amino acid sequence of SEQ ID NO:2, the amino acid sequence of SEQ ID NO:18, the amino acid sequence of SEQ ID NO:19, the amino acid sequence of SEQ ID NO:20, the amino acid sequence of SEQ ID NO:21, the amino acid sequence of SEQ ID NO:22, the amino acid sequence of SEQ ID NO:23, or the amino acid sequence of SEQ ID NO:24. It is necessary that the protein has an activity for a pyridoxamine synthetase. In this case, the enzymatic activity of synthesizing pyridoxamine from pyridoxal can be measured by, for example, adding a protein to be tested to an aqueous solution including pyridoxal as a substrate and L-alanine, and quantifying the amount of produced pyridoxamine by high performance liquid chromatography or the like.

Alternatively, the pyridoxamine synthetase may be a pyridoxamine synthetase including at least one of the following partial amino acid sequence (c), partial amino acid sequence (d), partial amino acid sequence (e), partial amino acid sequence (f), partial amino acid sequence (g), or partial amino acid sequence (h), and having an enzymatic activity of synthesizing pyridoxamine from pyridoxal. In this case, the enzymatic activity of synthesizing pyridoxamine from pyridoxal can be measured by, for example, adding a protein to be tested to an aqueous solution including pyridoxal and L-alanine as substrates, and quantifying the amount of produced pyridoxamine by high performance liquid chromatography or the like.
(c) X₈X₉X₁₀X₁₁X₁₂X₁₃ (SEQ ID NO:39)
   wherein X₈ represents L, M, I or V,
   X₉ represents H or Q,
   X₁₀ represents G, C or A,
   X₁₁ represents E or D,
   X₁₂ represents P or A, and
   X₁₃ represents V, I, L or A;
(d) X₁₄X₁₅TPSGTX₁₆X₁₇ (SEQ ID NO:40)
   wherein X₁₄ represents H or S,
   X₁₅ represents D or E,
   X₁₆ represents I, V, or L, and
   X₁₇ represents N or T;
(e) X₁₈DX₁₉VSX₂₀X₂₁ (SEQ ID NO:41)
   wherein X₁₈ represents V, I, or A,
   X₁₉ represents A, T, or S,
   X₂₀ represents S, A, or G, and
   X₂₁ represents F, W, or V;
(f) X₂₂X₂₃X₂₄KCX₂₅GX₂₆X₂₇P (SEQ ID NO:42)
   wherein X₂₂ represents G or S,
   X₂₃ represents P, S, or A,
   X₂₄ represents N, G, S, A, or Q,
   X₂₅ represents L or M,
   X₂₆ represents A, S, C, or G, and
   X₂₇ represents P, T, S, or A;
(g) X₂₈X₂₉X₃₀X₃₁SX₃₂GX₃₃X₃₄ (SEQ ID NO:43)
   wherein X₂₈ represents G or D,
   X₂₉ represents V or I,
   X₃₀ represents V, T, A, S, M, I, or L,
   X₃₁ represents F, M, L, I, or V,
   X₃₂ represents S, G, A, T, I, L, or H,
   X₃₃ represents R, M, or Q, and
   X₃₄ represents G, R, A, D, H, or K; and
(h) X₃₅X₃₆RX₃₇X₃₈HMGX₃₉X₄₀A (SEQ ID NO:44)
   wherein X₃₅ represents L or V,
   X₃₆ represents T, I, V, or L,
   X₃₇ represents I, V, or L,
   X₃₈ represents G or S,
   X₃₉ represents P, A, or R, and
   X₄₀ represents T, V, or S.

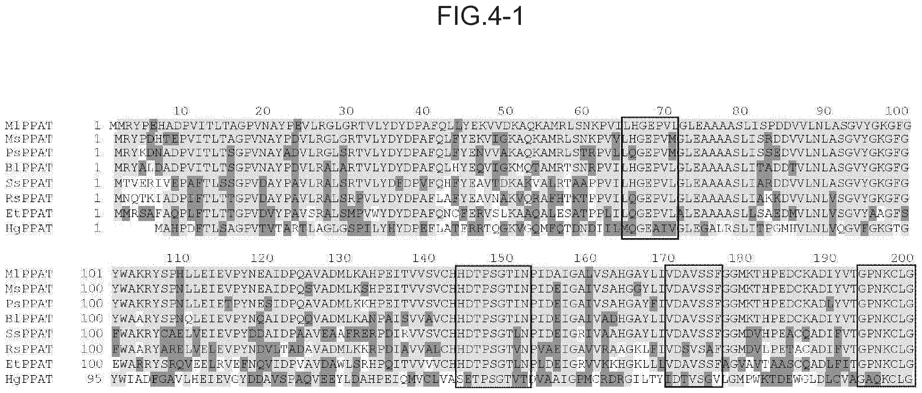

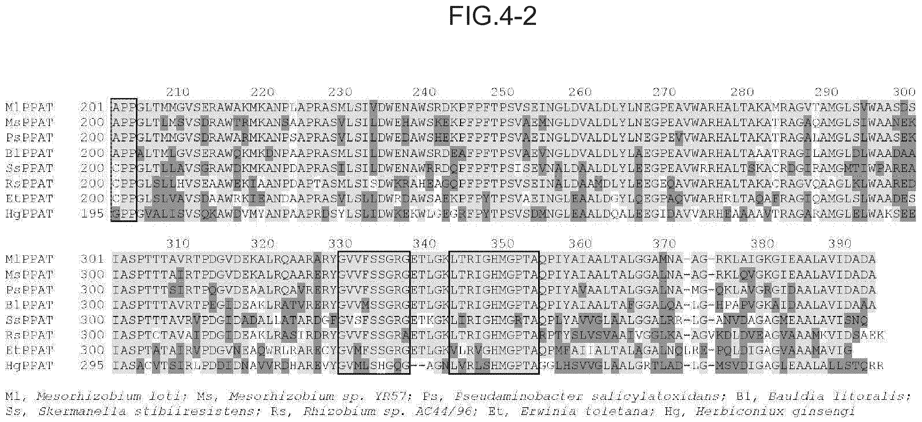

Fig. 4-1 and Fig 4-2 illustrate alignments of the sequences of SEQ ID NO:2 and SEQ ID NO:18 to SEQ ID NO:24, respectively. In Fig. 4-1 and Fig.4-2, M1PPAT represents a pyridoxamine-pyruvate transaminase from *Mesorhizobium loti,* MsPPAT represents a pyridoxamine-pyruvate transaminase from *Mesorhizobium* sp. YR577, PsPPAT represents a pyridoxamine-pyruvate transaminase from *Pseudaminobacter salicylatoxidans,* B1PPAT represents a pyridoxamine-pyruvate transaminase from *Bauldia litoralis,* SsPPAT represents a pyridoxamine-pyruvate transaminase from *Skermanella stibiiresistens,* RsPPAT represents a pyridoxamine-pyruvate transaminase from *Rhizobium* sp. AC44/96, EtPPAT represents a pyridoxamine-pyruvate transaminase from *Erwinia toletana,* and HgPPAT represents a pyridoxamine-pyruvate transaminase from *Herbiconiux ginsengi.* The partial amino acid sequence (c) corresponds to amino acid residues corresponding to the 65th to 70th amino acid residues from the N-terminus of the pyridoxamine-pyruvate transaminase from *Mesorhizobium loti* in alignment, the partial amino acid sequence (d) corresponds to amino acid residues corresponding to the 144th to 152nd amino acid residues from the N-terminus of the pyridoxamine-pyruvate transaminase from *Mesorhizobium loti* in alignment, the partial amino acid sequence (e) corresponds to amino acid residues corresponding to the 170th to 176th amino acid residues from the N-terminus of the pyridoxamine-pyruvate transaminase from *Mesorhizobium loti* in alignment, the partial amino acid sequence (f) corresponds to amino acid residues corresponding to the 194th to 203rd amino acid residues from the N-terminus of the pyridoxamine-pyruvate transaminase from *Mesorhizobium loti* in alignment, the partial amino acid sequence (g) corresponds to the amino acid residues corresponding to the 329th to 337th amino acid residues from the N-terminus of the pyridoxamine-pyruvate transaminase from *Mesorhizobium loti* in alignment, and the partial amino acid sequence (h) corresponds to the amino acid residues corresponding to the 343rd to 353rd amino acid residues from the N-terminus of pyridoxamine-pyruvate transaminase from *Mesorhizobium loti* in alignment. The partial amino acid sequence (c) preferably exists in the region of the 55th to 80th amino acid residues from the N-terminus of the protein, and more preferably exists in the region of the 56th to 75th amino acid residues from the N-terminus. The partial amino acid sequence (d) preferably exists in the region of the 134th to 162nd amino acid residues from the N-terminus of a protein, and more preferably exists in the region of the 139th to 157th amino acid residues from the N-terminus. The partial amino acid sequence (e) preferably exists in the region of the 160th to 186th amino acid residues from the N-terminus of a protein, and more preferably exists in the region of the 165th to 181st amino acid residues from the N-terminus. The partial amino acid sequence (f) preferably exists in the region of the 184th to 213rd amino acid residues from the N-terminus of a protein, and more preferably exists in the region of the 189th to 208th amino acid residues from the N-terminus. The partial amino acid sequence (g) preferably exists in the region of the 319th to 347th amino acid residues from the N-terminus of a protein, and more preferably exists in the region of the 324th to 342nd amino acid residues from the N-terminus. The partial amino acid sequence (h) preferably exists in the region of the 333rd to 363rd amino acid residues from the N-terminus of a protein, and more preferably exists in the region of the 338th to 358th amino acid residues from the N-terminus. In the disclosure, alignment between sequences can be performed by using, for example, a BLAST (registered trademark, National Library of Medicine) program with default parameters.

In the disclosure, " amino acid residue corresponding to the Xth amino acid residue from N-terminus of enzyme A" in the amino acid sequence of enzyme B refers to an amino acid residue on the amino acid sequence of the enzyme B, corresponding to the Xth amino acid residue from the N-terminus of the amino acid sequence of enzyme A in a case in which the amino acid sequence of enzyme A is aligned with the amino acid sequence of the enzyme B.

As can be seen from Fig. 4-1 and Fig. 4-2, the partial amino acid sequence (c), the partial amino acid sequence (d), the partial amino acid sequence (e), the partial amino acid sequence (f), the partial amino acid sequence (g), and the partial amino acid sequence (h) are regions highly conserved in a group of pyridoxamine-pyruvate transaminases. Therefore, a variation of the pyridoxamine-pyruvate transaminases including at least one of the partial amino acid sequence (c), the partial amino acid sequence (d), the partial amino acid sequence (e), the partial amino acid sequence (f), the partial amino acid sequence (g), or the partial amino acid sequence (h) is considered to be highly probable to achieve functioning as the pyridoxamine synthetase in the disclosure. In the pyridoxamine-pyruvate transaminase from *Mesorhizobium loti,* an amino acid residue corresponding to the 197th lysine residue from the N-terminus on the alignment is important for binding to pyridoxal, an amino acid residue corresponding to the 68th glutamate residue from the N-terminus on the alignment is important for catalytic activity, an amino acid residue corresponding to the 171st aspartate residue from the N-terminus on the alignment and an amino acid residue corresponding to the 146th threonine residue from the N-terminus on the alignment assist binding to pyridoxal, and an amino acid residue corresponding to the 336th arginine residue from the N-terminus on the alignment and an amino acid residue corresponding to the 345th arginine residue from the N-terminus on the alignment are considered to be important for recognizing an amino acid (Journal of Biological Chemistry, 2008, vol. 283, No. 2, pp 1120-1127), and the amino acid residues are important residues in view of the function of the pyridoxine synthetase. In a case in which these residues are conserved, it is considered to be highly probable to achieve functioning as the pyridoxine synthetase in the disclosure. However, an amino acid residue corresponding to the 68th glutamate residue from the N-terminus of the pyridoxamine-pyruvate transaminase from *Mesorhizobium loti* on the alignment may be not only glutamate but also aspartate. An amino acid residue corresponding to the 336th arginine residue from the N-terminus of the pyridoxamine-pyruvate transaminase from *Mesorhizobium loti* on the alignment may be not only arginine but also methionine or glutamine.

Examples of other expressions of a region including the amino acid residue corresponding to the 68th glutamate residue from the N-terminus of the pyridoxamine-pyruvate transaminase from *Mesorhizobium loti* on the alignment include the following partial amino acid sequence (c-1). Instead of the partial amino acid sequence (c), the pyridoxamine synthetase may include the partial amino acid sequence (c-1).
(c-1) X₁X₂X₃X₄X₅X₆X₇X₈X₉X₁₀X₁₁X₁₂X₁₃X₁₄X₁₅X₁₆X₁₇X₁₈X₁₉ (SEQ ID NO:45)
wherein X₁ represents V, L, I, or M,
X₂ represents I, L, or V,
X₃ represents L, M, I, or V,
X₄ represents H or Q,
X₅ represents G, C, or A,
X₆ represents E or D,
X₇ represents P or A,
X₈ represents V, I, A, or L,
X₉ represents L, M, P, or V,
X₁₀ represents G or A,
X₁₁ represents L or I,
X₁₂ represents E or Q,
X₁₃ represents A or G,
X₁₄ represents A or V,
X₁₅ represents A or L,
X₁₆ represents A, L, H, or Y,
X₁₇ represents S, G, or A,
X₁₈ represents L, F, V, or A, and
X₁₉ represents I, F, V, or L.

The partial amino acid sequence (c-1) corresponds to amino acid residues corresponding to the 63rd to 81st amino acid residues from the N-terminus of the pyridoxamine-pyruvate transaminase from *Mesorhizobium loti* on the alignment. The partial amino acid sequence (c-1) preferably exists in the region of the 53rd to 91st amino acid residues from the N-terminus of a protein, and more preferably exists in the region of the 58th to 86th amino acid residues from the N-terminus.

Examples of other expressions of a region including the amino acid residue corresponding to the 146th threonine residue from the N-terminus of a pyridoxamine-pyruvate transaminase from *Mesorhizobium loti* on the alignment include the following partial amino acid sequence (d-1). Instead of the partial amino acid sequence (d), the pyridoxamine synthetase may include the partial amino acid sequence (d-1).
(d-1) X₁X₂X₃X₄X₅X₆X₇X₈TPSGTX₉X₁₀X₁₁X₁₂X₁₃X₁₄X₁₅X₁₆ (SEQ ID NO:46)
wherein X₁ represents V, I, L, or M,
X₂ represents V or I,
X₃ represents S, A, V, C, or F,
X₄ represents V, I, A, L, or T,
X₅ represents C or V,
X₆ represents H, N, or A,
X₇ represents H or S,
X₈ represents D or E,
X₉ represents I, V, or L,
X₁₀ represents N or T,
X₁₁ represents P or D,
X₁₂ represents I, V, L, or A,
X₁₃ represents D, N, E, A, G, Q, V, R, or P,
X₁₄ represents A, E, Q, or D,
X₁₅ represents I or L, and
X₁₆ represents G or A.

The partial amino acid sequence (d-1) corresponds to the amino acid residues corresponding to the 138th to 158th amino acid residues from the N-terminus of the pyridoxamine-pyruvate transaminase from *Mesorhizobium loti* on the alignment. The partial amino acid sequence (d-1) preferably exists in the region of the 128th to 168th amino acid residues from the N-terminus of a protein, and more preferably exists in the region of the 133rd to 163rd amino acid residues from the N-terminus.

Examples of other expressions of a region including the amino acid residue corresponding to the 171st aspartate residue from the N-terminus of a pyridoxamine-pyruvate transaminase from *Mesorhizobium loti* include the following partial amino acid sequence (e-1). Instead of the partial amino acid sequence (e), the pyridoxamine synthetase may include the partial amino acid sequence (e-1).
(e-1) X₁X₂X₃X₄X₅X₆DX₇VSX₈X₉X₁₀X₁₁X₁₂ (SEQ ID NO:47)
in which X₁ represents G, D, or A,
X₂ represents A, G, K, T, Q, R, or E,
X₃ represents Y, N, L, or F,
X₄ represents L, F, M, or V,
X₅ represents I, L, or Y,
X₆ represents V, A, or I,
X₇ represents A, S, or T,
X₈ represents S, A, or G,
X₉ represents F, W, or V,
X₁₀ represents G, A, or L,
X₁₁ represents G or S, and
X₁₂ represents M, V, or L.

The partial amino acid sequence (e-1) corresponds to the amino acid residues corresponding to the 165th to 179th amino acid residues from the N-terminus of the pyridoxamine-pyruvate transaminase from *Mesorhizobium loti* on the alignment. The partial amino acid sequence (e-1) preferably exists in the region of the 155th to 189th amino acid residues from the N-terminus of the protein, and more preferably exists in the region of the 160th to 184th amino acid residues from the N-terminus.

Examples of other expressions of a region including the amino acid residue corresponding to the 197th lysine residue from the N-terminus of a pyridoxamine-pyruvate transaminase from *Mesorhizobium loti* on the alignment include the following partial amino acid sequence (f-1). Instead of the partial amino acid sequence (f), the pyridoxamine synthetase may include the partial amino acid sequence (f-1).
(f-1) X₁X₂X₃X₄X₅X₆X₇X₈X₉KCX₁₀GX₁₁X₁₂PX₁₃X₁₄X₁₅X₁₆X₁₇X₁₈X₁₉S (SEQ ID NO:48)
wherein X₁ represents A, S, V, or I,
X₂ represents D, G, or A,
X₃ represents I, L, F, V, or M,
X₄ represents Y, F, L, or C,
X₅ represents V or I,
X₆ represents T or A,
X₇ represents G or S,
X₈ represents P, S, or A,
X₉ represents N, G, S, Q, or A,
X₁₀ represents L or M,
X₁₁ represents A, S, C, or G,
X₁₂ represents P, T, S, or A,
X₁₃ represents G, A, or S,
X₁₄ represents L or V,
X₁₅ represents T, S, or A,
X₁₆ represents M, I, L, V, or F,
X₁₇ represents M, L, V, A, or I,
X₁₈ represents G, A, H, or S, and
X₁₉ represents V, I, or A.

The partial amino acid sequence (f-1) corresponds to the amino acid residues corresponding to the 188th to 211st amino acid residues from the N-terminus of the pyridoxamine-pyruvate transaminase from *Mesorhizobium loti* on the alignment. The partial amino acid sequence (f-1) preferably exists in the region of from the 178th to 221st amino acid residues from the N-terminus of the protein, and more preferably exists in the region of from the 183rd to 216th amino acid residues from the N-terminus.

Examples of other expressions of a region including the amino acid residue corresponding to the 336th arginine residue from the N-terminus of the pyridoxamine-pyruvate transaminase from *Mesorhizobium loti* on the alignment include the following partial amino acid sequence (g-1). Instead of the partial amino acid sequence (g), the pyridoxamine synthetase may include the partial amino acid sequence (g-1).
(g-1) X₁X₂X₃X₄X₅SX₆GX₇X₈ (SEQ ID NO:49)
wherein X₁ represents Y, F, H, or S,
X₂ represents G or D,
X₃ represents V or I,
X₄ represents V, T, A, S, M, I, or L,
X₅ represents F, M, L, I, or V,
X₆ represents S, G, A, T, I, L, or H,
X₇ represents R, M, or Q, and
X₈ represents G, R, A, D, H, or K.

The partial amino acid sequence (g-1) corresponds to the amino acid residues corresponding to the 328th to 337th amino acid residues from the N-terminus of the pyridoxamine-pyruvate transaminase from *Mesorhizobium loti* on the alignment. The partial amino acid sequence (g-1) preferably exists in the region of the 318th to 347th amino acid residues from the N-terminus of the protein, and more preferably exists in the region of the 323rd to 342nd amino acid residues from the N-terminus.

Examples of other expressions of a region including the amino acid residue corresponding to the 345th arginine residue from the N-terminus of the pyridoxamine-pyruvate transaminase from *Mesorhizobium loti* on the alignment include the following partial amino acid sequence (h-1). Instead of the partial amino acid sequence (h), the pyridoxamine synthetase may include the partial amino acid sequence (h-1).
(h-1) X₁X₂X₃X₄X₅RX₆X₇HMGX₈X₉AX₁₀X₁₁ (SEQ ID NO:50)
wherein X₁ represents L, Q, K, A, F, Y, or W,
X₂ represents G, N, H, or D,
X₃ represents K, R, or N,
X₄ represents L or V,
X₅ represents T, I, V, or L,
X₆ represents I, V, or L,
X₇ represents G or S,
X₈ represents P, A, or R,
X₉ represents T, V, or S,
X₁₀ represents Q, R, E, K, H, Y, or G, and
X₁₁ represents P or G.

The partial amino acid sequence (h-1) corresponds to the amino acid residues corresponding to the 340th to 355th amino acid residues from the N-terminus of the pyridoxamine-pyruvate transaminase from *Mesorhizobium loti* on the alignment. The partial amino acid sequence (h-1) preferably exists in the region of the 330th to 365th amino acid residues from the N-terminus of a protein, and more preferably exists in the region of the 335th to 360th amino acid residues from the N-terminus.

The pyridoxamine synthetase may be, for example, a protein having the amino acid sequence of SEQ ID NO:2, or may be a protein having an amino acid sequence in which one or more of the substitution, deletion, or insertion of an amino acid residue, or the addition of an additional amino acid residue to either or both of the N-terminus and C-terminus of the amino acid sequence are performed in the amino acid sequence of SEQ ID NO:2. Examples of the degrees of the substitution, deletion, and insertion of the amino acid residue, and the addition of an additional amino acid residue to either or both of the N-terminus and the C-terminus of the amino acid sequence are as described above.

Alternatively, the pyridoxamine synthetase may be a protein having the amino acid sequence of SEQ ID NO:2, or may be a protein having an amino acid sequence having, for example, 80% or more, 85% or more, 90% or more, or 95% or more sequence identity with the amino acid sequence of SEQ ID NO:2.

In the case of using such a protein having an amino acid sequence similar to the amino acid sequence of SEQ ID NO:2 as described above, it is necessary that the protein has an activity for pyridoxamine synthetase (an enzymatic activity of synthesizing pyridoxamine from pyridoxal, also referred to as pyridoxamine synthetase activity in the disclosure). The enzymatic activity (pyridoxamine synthetase activity) of synthesizing pyridoxamine from pyridoxal can be measured by, for example, adding a protein to be tested to an aqueous solution including pyridoxal as a substrate, and necessary amino acid (for example, L-alanine in the case of a protein having an amino acid sequence similar to the amino acid sequence of SEQ ID NO:2), and quantifying the amount of produced pyridoxamine by high performance liquid chromatography or the like.

Alternatively, the pyridoxamine synthetase may be, for example, a protein having an amino acid sequence of SEQ ID NO:4, or may be a protein having an amino acid sequence in which one or more of the substitution, deletion, or the insertion of an amino acid residue, or the addition of an additional amino acid residue to either or both of the N-terminus and C-terminus are performed in the amino acid sequence of SEQ ID NO:4. Examples of the degrees of the substitution, deletion, and insertion of an amino acid residue, and the addition of an additional amino acid residue to either or both of the N-terminus andr the C-terminus of the amino acid sequence are as described above.

Alternatively, the pyridoxamine synthetase may be a protein having the amino acid sequence of SEQ ID NO:4, or may be a protein having an amino acid sequence having, for example, 80% or more, 85% or more, 90% or more, or 95% or more sequence identity with the amino acid sequence of SEQ ID NO:4.

In a case of using such a protein having an amino acid sequence similar to the amino acid sequence of SEQ ID NO:4 as described above, it is necessary that the protein has an activity for a pyridoxamine synthetase (an enzymatic activity of synthesizing pyridoxamine from pyridoxal, also referred to as pyridoxamine synthetase activity in the disclosure). The enzyme (pyridoxamine synthetase activity) of synthesizing pyridoxamine from pyridoxal can be measured by, for example, adding a protein to be tested to an aqueous solution including pyridoxal as a substrate, and necessary amino acid (for example, L-glutamic acid, L-aspartic acid, or a salt thereof in the case of a protein having an amino acid sequence similar to the amino acid sequence of SEQ ID NO:4), and quantifying the amount of produced pyridoxamine by high performance liquid chromatography or the like.

The hydrogen peroxide-degrading enzyme may be, for example, a protein having the amino acid sequence of SEQ ID NO:3, or may be a protein having an amino acid sequence in which one or more of the substitution, deletion, or the insertion of an amino acid residue, or addition of an additional amino acid residue to either or both of the N-terminus and C-terminus of the amino acid sequence are performed in the amino acid sequence of SEQ ID NO:3. Examples of the degrees of the substitution, deletion, and insertion of the amino acid residue, and the addition of an additional amino acid residue to either or both of the N-terminus and the C-terminus of the amino acid sequence are as described above.

Alternatively, the hydrogen peroxide-degrading enzyme may be a protein having the amino acid sequence of SEQ ID NO:3, or may be a protein having an amino acid sequence having, for example, 80% or more, 85% or more, 90% or more, or 95% or more sequence identity with the amino acid sequence of SEQ ID NO:3.

In a case of using such a protein having an amino acid sequence similar to the amino acid sequence of SEQ ID NO:3 as described above, it is necessary that the protein has an activity for a hydrogen peroxide-degrading enzyme. The activity of the hydrogen peroxide-degrading enzyme can be measured by, for example, adding a protein to be tested to an aqueous solution of hydrogen peroxide as a substrate and quantifying decrease in the amount of hydrogen peroxide using decrease in absorbance at 240 nm or the like.

### <Gene Encoding Pyridoxine Oxidase, Gene Encoding Pyridoxamine Synthetase, and Gene Encoding Hydrogen Peroxide-Degrading Enzyme>

The gene encoding the pyridoxine oxidase may be any gene encoding the pyridoxine oxidase described above. The gene encoding the pyridoxamine synthetase may be any gene encoding the pyridoxamine synthetase described above. The gene encoding the hydrogen peroxide-degrading enzyme may be any gene encoding the hydrogen peroxide-degrading enzyme described above. Enzymes encoded by these genes are not limited to known amino acid sequences having the enzymatic having the enzymatic activities (for example, an amino acid sequence encoded by a gene naturally occurring in an organism), and may be enzymes having modified amino acid sequences different from the known amino acid sequences.

Such a gene may be a known gene such as a gene naturally possessed by the microorganism exemplified above as the microorganism (microorganism from which the enzyme is derived) having each enzyme, or a gene in which a nucleotide sequence is modified so that the nucleotide sequence encodes a modified amino acid sequence that is modified from the known amino acid sequence of the enzyme as described above as long as the desired enzymatic activity is obtained. Examples of the modified amino acid sequence include an amino acid sequence similar to any one of the amino acid sequences of SEQ ID NO:1 to SEQ ID NO:4 as described above. The nucleotide sequence of a gene encoding a particular amino acid sequence can be varied within degeneracy of a codon. In this case, it is preferable to use a codon frequently used in a microorganism used as a host of the recombinant microorganism from the viewpoint of gene expression efficiency.

It is also possible to design a nucleotide sequence of a gene based on an amino acid sequence to be encoded using the codon table. The designed nucleotide sequence may be obtained by modifying a known nucleotide sequence using genetic recombination technology, or may be obtained by chemically synthesizing the nucleotide sequence.

Examples of the method of modifying a nucleotide sequence include site-directed mutagenesis (Kramer, W. and frita, H.J., Methods in Enzymology, vol.154, P.350 (1987)), recombinant PCR (PCR Technology, Stockton Press (1989)), a method of chemically synthesizing a specific part of DNA, a method of treating a gene with hydroxyamine, a method of treating a strain carrying a gene with ultraviolet irradiation or a chemical agent such as nitrosoguanidine or nitrous acid, and a method of using a commercially available mutagenesis kit.

For example, each of the gene encoding the pyridoxine oxidase, the gene encoding the pyridoxamine synthetase, and the gene encoding the hydrogen peroxide-degrading enzyme may be DNA having an unmodified nucleotide sequence that encodes a polynucleotide having the enzymatic activity (for example, a nucleotide sequence possessed by a gene naturally occurring in an organism such as the above-described microorganism), or DNA having a nucleotide sequence in which a modification is made in the above nucleotide sequence as long as the enzymatic activity (the above-described enzymatic activity) of an enzyme encoded by the nucleotide sequence is not lost. Examples of the modification include insertion, deletion, and substitution of a nucleotide, and addition of an additional nucleotide to either or both of the 5' end and 3' end of the nucleotide sequence. In a case in which there is one or more of the insertion, deletion, and substitution of the nucleotide, the number of each of the insertion, deletion, and substitution, if present, may be, for example, from 1 to 90 nucleotides, from 1 to 60 nucleotides, from 1 to 30 amino acid residues, from 1 to 20 amino acid residues, from 1 to 15 nucleotides, from 1 to 10 nucleotides, or from 1 to 5 nucleotides; and the total number of the insertion, deletion, and substitution of the nucleotide may be, for example from 1 to 100 nucleotides, from 1 to 50 nucleotides, from 1 to 30 nucleotides, from 1 to 10 nucleotides, or from 1 to 5 nucleotides. Insertion or deletion of a nucleotide may occur locally, and it is preferable that the entire nucleotide sequence does not have a large frame shift. The number of nucleotide added to the end if present, may be, for example, from 1 to 150 nucleotides, from 1 to 100 nucleotides, from 1 to 50 nucleotides, from 1 to 30 nucleotides, from 1 to 10 nucleotides, or from 1 to 5 nucleotides per end. Such an additional nucleotide may encode a signal sequence for extracellular secretion or the like.

Alternatively, the gene encoding each of the enzymes may be DNA having a known nucleotide sequence (for example, a nucleotide sequence of a gene naturally occurring in an organism) that encodes a polynucleotide having the enzymatic activity per se, or DNA that has a nucleotide sequence having 80% or more, 85% or more, 90% or more, or 95% or more sequence identity with the known nucleotide sequence and that encodes an enzyme having a desired enzymatic activity (such as the above-mentioned enzymatic activity). Here, the sequence identity can be evaluated by using, for example, a BLAST (registered trademark, National Library of Medicine) program with default parameters.

Alternatively, the gene encoding each of the enzymes may be DNA having a known nucleotide sequence (for example, a nucleotide sequence of a gene naturally occurring in an organism) that encodes a polynucleotide having the enzymatic activity per se, or DNA having a nucleotide sequence that hybridizes under a stringent condition with DNA having a nucleotide sequence complementary to the known nucleotide sequence, and that encodes an enzyme having a desired enzymatic activity (such as the above-described enzymatic activity). Hybridization under a stringent condition can be performed as follows.

The hybridization is performed on DNA of interest using DNA consisting of a nucleotide sequence complementary to a reference nucleotide sequence or a partial sequence thereof as a probe. After washing the resultant under a stringent condition, the significance of the hybridization of the probe to the nucleic acid of interest is confirmed. The length of the probe can be, for example, 20 or more continuous nucleotides, preferably 50 or more nucleotides, more preferably 100 or more nucleotides, and further preferably 200 or more nucleotides. It is also preferable to use DNA as a probe that has the same nucleotide length as the reference nucleotide sequence and that is complementary to the reference nucleotide over the entire length. Examples of the conditions for hybridization may include conditions commonly used by those skilled in the art for detecting a specific hybridization signal. Such conditions preferably mean stringent hybridization conditions and stringent wash conditions. Examples thereof include conditions that a temperature of 55°C is maintained overnight, together with a probe, in a solution including 6 × SSC (saline sodium citrate) (composition of 1 × SSC: 0.15 M NaCl, 0.015 M sodium citrate, pH 7.0), 0.5% SDS, 5 × Denhardt, and 100 mg/ mL herring sperm DNA. Examples thereof may include subsequent washing of a filter in 0.2 × SSC at 42°C. Examples of the stringent conditions may include conditions of 0.1 × SSC and 50°C in the step of washing a filter, and, in addition, examples of the stringent conditions can include 0.1 × SSC and 65°C in the same step.

For example, the gene encoding the pyridoxine oxidase may be, for example, DNA having the nucleotide sequence of SEQ ID NO:5 (the nucleotide sequence of a gene encoding a pyridoxine oxidase from *Microbacterium luteolum*), or DNA having a nucleotide sequence that hybridizes with DNA having a nucleotide sequence complementary to the nucleotide sequence of SEQ ID NO:5 under stringent conditions, and that encodes a protein having pyridoxine oxidase activity.

Alternatively, the gene encoding the pyridoxine oxidase may be, for example, DNA having the nucleotide sequence of SEQ ID NO:5, or DNA having a nucleotide sequence in which one or more of the substitution, deletion, or insertion of a nucleotide, or the addition of an additional nucleotide to either or both of the 5' end and the 3' end of the nucleotide sequence are performed in the nucleotide sequence of SEQ ID NO:5, and which encodes a protein having pyridoxine oxidase activity. Examples of the degrees of the substitution, deletion, and insertion of a nucleotide, and the addition of an additional nucleotide to either or both of the N-terminus and C-terminus of the nucleotide sequence are as described above.

Alternatively, the pyridoxine oxidase may be DNA having the nucleotide sequence of SEQ ID NO:5, or DNA having a nucleotide sequence that has, for example, 80% or more, 85% or more, 90% or more, or 95% or more sequence identity with the nucleotide sequence of SEQ ID NO:5, and that encodes a protein having pyridoxine oxidase activity.

The gene encoding the pyridoxamine synthetase may be, for example, DNA having the nucleotide sequence of SEQ ID NO:6 (the nucleotide sequence of a pyridoxamine-pyruvate transaminase gene from *Mesorhizobium loti)* or the nucleotide sequence of SEQ ID NO:8 (the nucleotide sequence of a pyridoxamine-oxaloacetate transaminase gene from *E. coli*), or may be DNA having a nucleotide sequence that hybridizes with DNA having a nucleotide sequence complementary to the nucleotide sequence of SEQ ID NO:6 or SEQ ID NO:8 under a stringent condition, and that encodes a protein having pyridoxamine synthetase activity.

Alternatively, the gene encoding the pyridoxamine synthetase may be, for example, DNA having the nucleotide sequence of SEQ ID NO:6 or SEQ ID NO:8, or DNA having a nucleotide sequence in which one or more of the substitution, deletion, or insertion of a nucleotide, or the addition of an additional nucleotide to either or both of the 5' end and the 3' end of the nucleotide sequence are performed in the nucleotide sequence of SEQ ID NO:6 or SEQ ID NO:8, and which encodes a protein having pyridoxamine synthetase activity. Examples of the degrees of the substitution, deletion, and insertion of the nucleotide, and the addition of an additional nucleotide to either or both of the N-terminus and C-terminus of the nucleotide sequence are as described above.

Alternatively, the pyridoxamine synthetase may be DNA having the nucleotide sequence of SEQ ID NO:6 or SEQ ID NO:8, or may be DNA having a nucleotide sequence that has, for example, 80% or more, 85% or more, 90% or more, or 95% or more sequence identity with the nucleotide sequence of SEQ ID NO:6 or SEQ ID NO:8, and that encodes a protein having pyridoxamine synthetase activity.

Alternatively, the gene encoding the pyridoxamine synthetase may be, for example, DNA having the nucleotide sequence of any one of SEQ ID NO:6 or SEQ ID NO:25 to SEQ ID NO:31, or may be DNA having a nucleotide sequence that hybridizes with DNA having a nucleotide sequence complementary to the nucleotide sequence of any one of SEQ ID NO:6 or SEQ ID NO:25 to SEQ ID NO:31 under a stringent condition, and that encodes a protein having pyridoxamine synthetase activity.

Alternatively, the gene encoding the pyridoxamine synthetase may be, for example, DNA having the nucleotide sequence of any one of SEQ ID NO:6 or SEQ ID NO:25 to SEQ ID NO:31, or DNA having a nucleotide sequence in which one or more of the substitution, deletion, or insertion of a nucleotide, or the addition of an additional nucleotide to either or both of the 5' end and the 3' end of the nucleotide sequence are performed in the nucleotide sequence of any one of SEQ ID NO:6 or SEQ ID NO:25 to SEQ ID NO:31, and which encodes a protein having pyridoxamine synthetase activity. Examples of the degrees of the substitution, deletion, and insertion of a nucleotide, and the addition of an additional nucleotide either or both of the N-terminus and C-terminus of a nucleotide sequence are as described above.

Alternatively, the pyridoxamine synthetase may be DNA having the nucleotide sequence of any one of SEQ ID NO:6 or SEQ ID NO:25 to SEQ ID NO:31, or may DNA having a nucleotide sequence that has, for example, 80% or more, 85% or more, 90% or more, or 95% or more sequence identity with at least one of the nucleotide sequence of SEQ ID NO:6, the nucleotide sequence of SEQ ID NO:25 (the nucleotide sequence of a gene encoding pyridoxamine-pyruvate transaminase from *Mesorhizobium sp.* YR577), the nucleotide sequence of SEQ ID NO:26 (the nucleotide sequence of a gene encoding pyridoxamine-pyruvate transaminase from *Pseudaminobacter salicylatoxidans*), the nucleotide sequence of SEQ ID NO:27 (the nucleotide sequence of a gene encoding pyridoxamine-pyruvate transaminase from *Bauldia litoralis*), the nucleotide sequence of SEQ ID NO:28 (the nucleotide sequence of a gene encoding a pyridoxamine-pyruvate transaminase from *Skermanella stibiiresistens*), the nucleotide sequence of SEQ ID NO:29 (the nucleotide sequence of gene encoding a pyridoxamine-pyruvate transaminase from *Rhizobium sp.* AC44/96), the nucleotide sequence of SEQ ID NO:30 (the nucleotide sequence of a gene encoding a pyridoxamine-pyruvate transaminase from *Erwinia toletana*), or the nucleotide sequence of SEQ ID NO:31 (the nucleotide sequence of a gene encoding a pyridoxamine-pyruvate transaminase from *Herbiconiux ginsengi*), and that encodes a protein having pyridoxamine synthetase activity.

In the case of expression in a recombinant microorganism in which prokaryote such as *E. coli* is used as a host, a codon may be optimized to facilitate expression. For example, a nucleotide sequence may be modified so that a codon, which is most frequently used, of codons that encodes respective amino acids in a prokaryote as a host is highly frequently used as a codon for the amino acid. From such a viewpoint, as DNA including a gene encoding the pyridoxamine synthetase, for example, DNA having the region between the 18th nucleotide and the 3' end of the nucleotide sequence of SEQ ID NO:10 or the region between the 18th nucleotide and the 3' end of the nucleotide sequence of any one of SEQ ID NO:32 to SEQ ID NO:38 may be used, or DNA having a nucleotide sequence that hybridizes with DNA having a nucleotide sequence complementary to the region between 18th nucleotide and the 3' end in the nucleotide sequence of SEQ ID NO:10 or the region between the 18th nucleotide and the 3' end in the nucleotide sequence of any one of SEQ ID NO:32 to SEQ ID NO:38 under a stringent condition, and that encodes a protein having pyridoxamine synthetase activity may be used. Seventeen nucleotides from the 5' end of the nucleotide sequence of SEQ ID NO:10 are included in an upstream region, and an initiation codon is included in the 18th nucleotide to the 20th nucleotide. Therefore, the region between the 18th nucleotide and the 3' end of this nucleotide sequence may be used as a gene region encoding a pyridoxamine synthetase. Similarly, seventeen nucleotides from the 5' end of each of the nucleotide sequences of SEQ ID NO:32 to SEQ ID NO:38 are included in an upstream, and an initiation codon is included in the 18th nucleotide to the 20th nucleotide. Therefore, the region between the 18th nucleotide and the 3' end of these nucleotide sequences may be used as a gene region encoding a pyridoxamine synthetase.

Alternatively, the gene encoding the pyridoxamine synthetase may be, for example, DNA having the region between the 18th nucleotide and the 3' end of the nucleotide sequence of SEQ ID NO:10 or the region between the 18th nucleotide and the 3' end of the nucleotide sequence of any one of SEQ ID NO:32 to SEQ ID NO:38, or DNA having a nucleotide sequence in which one or more of the substitution, deletion, or insertion of a nucleotide, or the addition of an additional nucleotide to either or both of the 5' end and the 3' end of the are performed in the region between the 18th nucleotide and the 3' end of the nucleotide sequence of the nucleotide sequence of SEQ ID NO:10 or the region between the 18th nucleotide and the 3' end of the nucleotide sequence of any one of SEQ ID NO:32 to SEQ ID NO:38, and which encodes a protein having pyridoxamine synthetase activity. Examples of the degrees of the substitution, deletion, and insertion of a nucleotide, and the addition of an additional nucleotide to either or both of the N-terminus and C-terminus of the nucleotide sequence are as described above.

Alternatively, the pyridoxamine synthetase may be DNA having the region between the 18th nucleotide and the 3' end of the nucleotide sequence of SEQ ID NO:10 or the region between the 18th nucleotide and the 3' end of any one of nucleotide sequences of SEQ ID NO:32 to SEQ ID NO:38, or may be DNA having a nucleotide sequence that has, for example, 80% or more, 85% or more, 90% or more, or 95% or more sequence identity with at least one of the region between the 18th nucleotide and the 3' end of the nucleotide sequence of SEQ ID NO:10 (codon-optimized nucleotide sequence of a gene encoding pyridoxamine-pyruvate transaminase from *Mesorhizobium loti*), the region between the 18th nucleotide and the 3' end of the nucleotide sequence of SEQ ID NO:32 (codon-optimized nucleotide sequence of a gene encoding pyridoxamine-pyruvate transaminase from *Mesorhizobium sp.* YR577), the region between the 18th nucleotide and the 3' end of the nucleotide sequence of SEQ ID NO:33 (codon-optimized nucleotide sequence of a gene encoding pyridoxamine-pyruvate transaminase from *Pseudaminobacter salicylatoxidans*), the region between the 18th nucleotide and the 3' end of the nucleotide sequence of SEQ ID NO:34 (codon-optimized nucleotide sequence of a gene encoding pyridoxamine-pyruvate transaminase from *Bauldia litoralis),* the region between the 18th nucleotide and the 3' end of the nucleotide sequence of SEQ ID NO:35 (codon-optimized nucleotide sequence of a gene encoding pyridoxamine-pyruvate transaminase from *Skermanella stibiiresistens*), the region between the 18th nucleotide and the 3' end of the nucleotide sequence of SEQ ID NO:36 (codon-optimized nucleotide sequence of gene encoding pyridoxamine-pyruvate transaminase from *Rhizobium sp.* AC44/96), the region between the 18th nucleotide and the 3' end of the nucleotide sequence of SEQ ID NO:37 (codon-optimized nucleotide sequence of a gene encoding pyridoxamine-pyruvate transaminase from *Erwinia toletana*), or the region between the 18th nucleotide and the 3' end of the nucleotide sequence of SEQ ID NO:38 (codon-optimized nucleotide sequence of a gene encoding pyridoxamine-pyruvate transaminase from *Herbiconiux ginsengi*), and that encodes a protein having pyridoxamine synthetase activity.

The gene encoding the hydrogen peroxide-degrading enzyme may be, for example, DNA having a nucleotide sequence of SEQ ID NO:7 (the nucleotide sequence of a gene encoding a catalase from *Listeria seeligeri*), or DNA having a nucleotide sequence that hybridizes with DNA having a nucleotide sequence complementary to the nucleotide sequence of SEQ ID NO:7 under a stringent condition, and that encodes a protein having hydrogen peroxide-degrading enzymatic activity.

Alternatively, the gene encoding the hydrogen peroxide-degrading enzyme may be, for example, DNA having the nucleotide sequence of SEQ ID NO:7, or DNA having a nucleotide sequence in which one or more of the substitution, deletion, or insertion of a nucleotide, or the addition of an additional nucleotide to both or either of the 5' end and the 3' end of the nucleotide sequence are performed in the nucleotide sequence of SEQ ID NO:7, and which encodes a protein having hydrogen peroxide-degrading enzymatic activity. Examples of the degrees of the substitution, deletion, and insertion of a nucleotide, and the addition of an additional nucleotide to both or either of the N-terminus and C-terminus of the nucleotide sequence are as described above.

Alternatively, the hydrogen peroxide-degrading enzyme may be DNA having the nucleotide sequence of SEQ ID NO:7, or may be DNA having a nucleotide sequence that has, for example, 80% or more, 85% or more, 90% or more, or 95% or more sequence identity with the nucleotide sequence of SEQ ID NO:7, and that encodes a protein having hydrogen peroxide-degrading enzymatic activity.

### <Recombinant Microorganism Having Gene Encoding Pyridoxine Oxidase and Gene Encoding Pyridoxamine Synthetase>

In the recombinant microorganism according to the disclosure, each of a gene encoding a pyridoxine oxidase and a gene encoding a pyridoxamine synthetase may be endogenous to a bacterial cell and has an enhanced expression, or may be introduced from outside of a bacterial cell into the bacterial cell. Both of a gene endogenous to a bacterial cell and has an enhanced expression and a gene introduced from outside of a bacterial cell into the bacterial cell may exist in a recombinant microorganism. In the recombinant microorganism according to the disclosure, in addition to either or both of a gene that encodes a pyridoxine oxidase and is endogenous to a bacterial cell and has an enhanced expression and a gene that encodes a pyridoxine oxidase and is introduced from outside of a bacterial cell into the bacterial cell, a gene that encodes a non-enhanced (for example, promoter-unmodified) pyridoxine oxidase and is endogenous to a bacterial cell may exist. Similarly, in addition to either or both of a gene that encodes a pyridoxamine synthetase and is endogenous to a bacterial cell and has an enhanced expression and a gene that encodes a pyridoxamine synthetase and is introduced from outside of a bacterial cell into the bacterial cell, a gene that encodes a non-enhanced (for example, promoter-unmodified) pyridoxamine synthetase and is endogenous to the cell may be present. In the disclosure, since one or more of the enhancement of the expression of the gene endogenous to a bacterial cell and the introduction of the gene from outside of a bacterial cell into the bacterial cell are performed for each of the gene encoding a pyridoxine oxidase and the gene encoding a pyridoxamine synthetase, even when a gene whose expression is not enhanced originally exists in a bacterial cell, an expression level significantly higher than the expression level by the original gene whose expression is not enhanced can be obtained, whereby high pyridoxamine production efficiency can be achieved.

In a case in which the recombinant microorganism according to the disclosure further includes a gene encoding a hydrogen peroxide-degrading enzyme, the gene encoding the hydrogen peroxide-degrading enzyme may be endogenous to a bacterial cell, endogenous to a bacterial cell and has an enhanced expression, or introduced from outside of a bacterial cell into the bacterial cell. However, since the amount of hydrogen peroxide produced increases as the reaction rate increases, it is preferable to perform at least one of the enhancement of the expression of a gene that encodes a hydrogen peroxide-degrading enzyme and is endogenous to a bacterial cell or the introduction of a gene that encoding a hydrogen peroxide-degrading enzyme from outside of a bacterial cell into the bacterial cell, in order to increase the expression level of hydrogen peroxide-degrading enzyme.

In other words, each of the gene encoding a pyridoxine oxidase and the gene encoding a pyridoxamine synthetase may be endogenous to the genome of a host microorganism and has an enhanced expression by an operation such as the substitution of a promoter, or may be introduced from outside of a bacterial cell into the bacterial cell using a vector such as a plasmid. In addition to this, the recombinant microorganism may further include a gene endogenous to the genome of a host microorganism prior to the recombination and whose expression is not enhanced. In the disclosure, each of the gene encoding a pyridoxine oxidase and the gene encoding a pyridoxamine synthetase is introduced from outside of a cell in the host microorganism, or expression of the gene endogenous to the cell in the host microorganism is enhanced by the substitution of a promoter, or the like, whereby the expression of each of the genes is increased, and the pyridoxamine production capability due to the combination of the above-described two enzymes is further enhanced. In the case of performing the introduction or expression enhancement of each of the gene encoding a pyridoxine oxidase and the gene encoding a pyridoxamine synthetase, either or both of the introduction from outside of a cell in a host microorganism and the enhancement of the gene expression by the substitution of a promoter, or the like, may be performed.

The recombinant microorganism according to the disclosure may include a gene encoding a hydrogen peroxide-degrading enzyme, but it is not necessarily required. When the recombinant microorganism according to the disclosure further includes a gene encoding a hydrogen peroxide-degrading enzyme, the gene encoding a hydrogen peroxide-degrading enzyme may be endogenous to the genome of a host microorganism prior to recombination, endogenous to the genome of a host microorganism and has an enhanced expression by an operation such as the substitution of a promoter, or introduced from outside of a bacterial cell into the bacterial cell using a vector such as a plasmid. In the case of performing the introduction or expression enhancement of the gene encoding a hydrogen peroxide-degrading enzyme, either or both of the introduction from outside of a cell in a host microorganism and the enhancement of the gene expression by substitution of a promoter or the like may be performed.

It is impossible to obtain a sufficient production capability for highly producing pyridoxamine or a salt thereof unless such increase in expression as described above is performed in the gene encoding a pyridoxine oxidase and the gene encoding a pyridoxamine synthetase. Therefore, in the recombinant microorganism according to the disclosure, at least one of the introduction from outside of a bacterial cell or the enhancement of the expression of a gene endogenous to the bacterial cell is performed in each of the gene encoding a pyridoxine oxidase and the gene encoding a pyridoxamine synthetase. Introduction of an enzyme gene from outside of a bacterial cell is not necessarily limited to the introduction for compensating an enzyme gene which is not present in a host microorganism, and the introduction may be performed for the purpose of increasing the expression of an enzyme gene endogenous to a host microorganism. An enzyme gene which is not present in a host microorganism can be easily confirmed using an enzyme database such as KEGG or BRENDA.

In a case in which the expression of a gene endogenous to a host microorganism is enhanced by the substitution of a promoter of the gene with another promoter, the another promoter (newly introduced promoter) is not particularly limited as long as the promoter can enhance the gene expression (more than before promoter substitution) in the host microorganism, and may be a constitutive promoter or an inducible promoter. Substitution of a promoter can be performed using a general genetic modification technology. The sequence of a promoter endogenous to a host microorganism may be partially or completely left unless the sequence adversely affects expression by a newly introduced promoter in practical use.

When the host microorganism is, for example, a prokaryote, examples of the promoter that can be used as the newly introduced promoter include a trp promoter, a lac promoter, and a GAPDH promoter from *E. coli,* a PL promoter and a PR promoter from lambda phage, and a gluconate synthetase promoter (gnt), an alkaline protease promoter (apr), a neutral protease promoter (npr), and an α-amylase promoter (amy) from *Bacillus subtilis.* An originally modified or designed promoter such as a tac promoter can also be used.

When the host microorganism is, for example, a filamentous fungus, examples of the promoter that can be used as the newly introduced promoter include a cellobiohydrolase (cbh) promoter, an endoglucanase (egl) promoter, a xylanase III (xyn3) promoter, a U6 promoter, and an α-amylase (amy) promoter.

When the host microorganism is, for example, yeast, examples of the promoter that can be used as the newly introduced promoter include an alcohol dehydrogenase (ADH1) promoter, a phosphoglycerate kinase (PGK1) promoter, a peptide chain elongation factor (TEF) promoter, a glycerol 3-phosphate dehydrogenase (GPD) promoter, a galactokinase (GAL1) promoter, a metallothionein (CUP1) promoter, a repressive acid phosphatase (PHO5) promoter, and a glyceraldehyde 3-phosphate dehydrogenase (GAPDH) promoter. The sequences of the above-described promoters are not limited to those derived from yeast used as a host microorganism. An exogenous promoter such as a cytomegalovirus (CMV) promoter may also be used.

In the case of introducing a gene (heterogenous gene) from outside of a host microorganism, the method is not particularly limited as long as a gene can be introduced into the inside of a bacterial cell (inside a cell) and an enzyme encoded by the gene can be expressed, and examples thereof include transformation with a plasmid carrying an enzyme gene, introduction of an enzyme gene into a genome, and a combination thereof. When introducing a gene, an expression vector into which the gene is integrated may be introduced into a bacterial cell. The expression vector is not particularly limited as long as the vector is a vector into which the nucleotide sequence of the gene is integrated, and the expression vector is more preferably a plasmid vector or a phage vector having the following constitution, from the viewpoint of improving transformation efficiency or translation efficiency.

The expression vector is not particularly limited as long as the vector includes the nucleotide sequence of the gene and can transform the host microorganism. If necessary, in addition to such a nucleotide sequence, a nucleotide sequence (hereinafter, also simply referred to as "another region") constituting another region may be included. Examples of such a region include a control region required to the production of a desired enzyme by a recombinant microorganism obtained by transformation, and a region required for autonomous replication.

From the viewpoint of facilitating selection of the recombinant microorganism, the vector may further include a nucleotide sequence encoding a selection gene which can be used as a selection marker.

Examples of a control region required to the production of a desired enzyme include a promoter sequence (including an operator sequence for controlling transcription), a ribosome-binding sequence (SD sequence), and a transcription termination sequence.

In the case of using yeast as a host microorganism, from the viewpoint of expression efficiency of the gene, an expression vector that can be used preferably includes a promoter sequence in addition to the nucleotide sequence of the above gene. Any promoter sequence may be used as long as the sequence can express the gene in a transformant obtained using yeast as a host microorganism. Examples of the promoter sequence include the promoters described above as examples of the promoter that can be used as the newly introduced promoter in the case of using yeast as a host microorganism.

The expression vector may include a secretion signal. This allows to, when a recombinant microorganism produces a desired enzyme, extracellular secretion of the enzyme.

The secretion signal is not particularly limited as long as a desired enzyme can be secreted from yeast as a host microorganism. From the viewpoint of secretion efficiency, it is preferable to use an α factor signal sequence, an invertase signal sequence, an acid phosphatase signal sequence, a glucoamylase signal sequence, or the like.

Specific examples of the expression vector having the promoter sequence and the secretion signal as described above include pRS423, pRS424, and YEplac195.

From the viewpoint of expression efficiency of the gene, an expression vector that can be used when a filamentous fungus is used as a host microorganism preferably includes a promoter sequence in addition to the nucleotide sequence of the above gene. Any promoter sequence may be used as long as the sequence can express the gene in a transformant obtained by using a filamentous fungus as a host microorganism. Examples of the promoter include the promoters described above as examples of the promoter that can be used as the newly introduced promoter in the case of using a filamentous fungus as the host microorganism.

Suitable expression vectors for filamentous fungi are described in van den Hondel, C.A.M. J.J.et al.(1991) In: Bennett, J.W. and Lasure, L.L.(eds.) More Gene Manipulations in Fungi. Academic Press, pp.396-428.

Another commonly used expression vector such as pUC18, pBR322, pUC100, pSL1180 (manufactured by Pharmacia Inc.), pFB6 and *Aspergillus* pRAX, or *Trichoderma* pTEX can also be used.

From the viewpoint of the expression efficiency of the gene, an expression vector which can be used when a prokaryote such as *E. coli, Bacillus subtilis,* or Actinomycetes is used as a host microorganism preferably includes a promoter sequence in addition to the nucleotide sequence of the above gene. The expression vector may include a ribosome-binding sequence, a transcription termination sequence, or the like other than a promoter sequence.

Examples of the promoter sequence include the promoters described above as examples of the promoter that can be used as the newly introduced promoter in the case of using a prokaryote as the host microorganism.

Examples of the ribosome-binding sequence include a sequence derived from *E. coli* or *B. subtilis,* and the ribosome-binding sequence is not particularly limited thereto as long as the sequence is a sequence that functions in a desired host microorganism such as *E. coli* or *B. subtilis.*

Examples of the ribosome-binding sequence include a sequence in which a consensus sequence having four or more consecutive nucleotides, of sequences complementary to the 3' end region of 16S ribosomal RNA is produced by DNA synthesis. The transcription termination sequence is not necessarily required, and a p-factor independent transcription termination, for example, as a lipoprotein terminator, a trp operon terminator, or the like can be used.

The order of these sequences on the expression vector of the control region is not particularly limited, and the promoter sequence, the ribosome-binding sequence, the gene encoding a target enzyme, and the transcription termination sequence are desirably arranged in this order from the upstream (5'-end side) in consideration of transcription efficiency.

Specific examples of the expression vector that can be used when a host microorganism is a prokaryote include pBR322, pUC18, Bluescript II SK (+), pKK223-3, or pSC101 including a region that can be autonomously replicated in E. coli, or pUB 110, pTZ4, pC194, ρ11, ϕ1, or ϕ105 including a region that can be autonomously replicated in *Bacillus subtilis.*

Examples of the vector that can be autonomously replicated in two or more kinds of host microorganisms include pHV14, TRp7, YEp7, and pBS7.

In the case of introducing a gene from outside of a cell in the host microorganism, the gene may be a gene having a nucleotide sequence that is not present in the genome of the host microorganism, and may be a gene having a nucleotide sequence that is present in the genome of the host microorganism. Even when the same gene originally exists in the genome of a host microorganism, the introduction of a gene from outside the bacterial cell causes stronger gene expression, and the enzymatic activity is enhanced and the production efficiency of pyridoxamine or a salt thereof can be further enhanced.

Conventional methods well known to those skilled in the art can be used as methods necessary for introducing a gene from outside of a bacterial cell (extracellular) into the bacterial cell (intracellular), such as preparation of a genomic DNA, cleavage and ligation of DNA, transformation, PCR (Polymerase Chain Reaction), and design and synthesis of an oligonucleotide used as a primer. These methods are described in Sambrook, J., et al., "Molecular Cloning A Laboratory Manual, Second Edition", Cold Spring Harbor Laboratory Press, (1989) and the like. For example, a method using competent cells and a method using electroporation are described.

The host microorganism is not particularly limited as long as the microorganism is a microorganism capable of expressing, if present, a gene encoding a pyridoxine oxidase and a gene encoding a pyridoxamine synthetase. Examples of the host microorganism include yeasts, filamentous fungi, and prokaryotes. Examples of the yeasts include a yeast belonging to the genus *Saccharomyces,* such as *Saccharomyces cerevisiae*, a yeast belonging to the genus *Schizosaccharomyces,* such as *Schizosaccharomyces pombe,* a yeast belonging to the genus *Hansenula,* and a yeast belonging to the genus *Pichia.* Examples of the filamentous fungi include a filamentous fungus belonging to the genus *Trichoderma,* such as *Trichoderma reesei* or *viride,* a filamentous fungus belonging to the genus *Aspergillus,* such as *Aspergillus niger* or *oryzae,* a filamentous fungus belonging to the genus *Humicola,* such as *Humicola insolens,* and a filamentous fungus belonging to the genus *Acremonium,* such as *Acremonium cellulolyticus* or *fusidioides.* Examples of the prokaryotes include a bacterium belonging to the genus *Escherichia,* such as *Escherichia coli,* a bacterium belonging to the genus *Schewanella,* such as *Schewanella* sp. AC10, a bacterium belonging to the genus *Mesorhizobium,* such as *Mesorhizobium loti,* a bacterium belonging to the genus *Rhizobium,* such as *Rhizobium meliloti,* a *Bacillus* bacterium belonging to the genus *Bacillus,* such as *Bacillus subtilis,* and an actinomycete such as an actinomycete belonging to the genus *Streptomyces,* such as *Streptomyces lividans.*

A desired gene can be introduced into a host microorganism by, for example, introducing (transforming) into these host microorganisms an expression vector including the desired gene. The introduced gene can be highly expressed in the obtained recombinant microorganism, for example, by the activity of a promoter included in the expression vector.

In a case in which the host microorganism is *E. coli,* for example, a competent cell method by calcium treatment, an electroporation method, or the like can be used as a method of introducing the recombinant DNA such as an expression vector into a cell of the host microorganism. The enzyme encoded by the introduced gene can be stably produced at a high expression level by culturing a recombinant microorganism obtained in such a manner.

The DNA encoding the enzyme can be isolated from the recombinant microorganism, and can be transferred into another microorganism. Using this DNA as a template, the DNA encoding the enzyme can be easily introduced into another host microorganism, by amplifying a DNA fragment encoding an enzyme by PCR, treating the resultant with a restriction enzyme or the like, and then the obtained fragment is ligated with another vector DNA fragment.

### <Method of Producing Pyridoxamine or Salt Thereof>

In one embodiment, a method of producing pyridoxamine or a salt thereof includes bringing the recombinant microorganism, the culture of the recombinant microorganism, or the treated product of the recombinant microorganism or the culture according to the disclosure, into contact with pyridoxine or a salt thereof in the presence of oxygen to produce pyridoxamine or a salt thereof.

Examples of a salt of pyridoxine include a salt of pyridoxine and an acid. Examples of the acid include hydrochloric acid, sulfuric acid, nitric acid, and phosphoric acid. Examples of the salt of pyridoxine include pyridoxine hydrochloride.

Examples of the salt of pyridoxamine include a salt of pyridoxamine with an acid. Examples of the acid include hydrochloric acid, sulfuric acid, nitric acid, acetic acid, and phosphoric acid. From the viewpoint of use as a medicament, the salt of pyridoxamine is preferably pyridoxamine dihydrochloride, which is most advanced for medical applications.

Such a method of producing pyridoxamine or a salt thereof using the recombinant microorganism, the culture of the recombinant microorganism, or the treated product of the recombinant microorganism or the culture is also simply referred to as "method of producing pyridoxamine or a salt thereof using a recombinant microorganism".

The culture of the recombinant microorganism refers to a product obtained by culturing the recombinant microorganism, which is composed of a bacterial cell, a surrounding medium, and the like. In a case in which an enzyme is secreted extracellularly, the enzyme can more easily contact with a substrate when such a culture is used, and the efficiency of producing pyridoxamine or a salt thereof can be improved. The use of the culture is not necessarily required. For example, a dried or frozen recombinant microorganism cell prepared in advance may be added directly to a reaction system.

Any of a synthetic medium and a natural medium can be used as a culture medium in culturing a recombinant microorganism, as long as the culture medium is a culture medium including adequate amounts of a carbon source, a nitrogen source, an inorganic substance, and another nutrient. The culture can be performed using a general culture method, such as shaking culture, aeration stirring culture, continuous culture, or fed-batch culture in a liquid medium including the culture components.

More specifically, the culture condition for the recombinant microorganism is the same as the culture condition for the original host microorganism, and known conditions can be used.

Known components can be used as the components used in the culture medium. For example, organic nutrient sources such as a meat extract, a yeast extract, a malt extract, peptone, NZ amine, and potato, carbon sources such as glucose, maltose, sucrose, starch, and an organic acid, nitrogen sources such as ammonium sulfate, urea, and ammonium chloride, inorganic nutrient sourced such as phosphate, magnesium, potassium, and iron, and vitamins can be used in combination, if appropriate.

In culture of a recombinant microorganism transformed by the expression vector including the selection marker, for example, a culture medium including an agent against the drug resistance is used in a case in which the selection marker has drug resistance, and a culture medium that does not include a nutrient is used in a case in which the selection marker has a requirement for the nutrient.

The culture conditions may be selected, if appropriate, depending on the kinds of the recombinant microorganism, a culture medium, and a culture method, and are not particularly limited as long as the culture conditions are conditions under which the recombinant microorganism grows and can produce a pyridoxine oxidase and a pyridoxamine synthetase.

The pH of the culture medium may be selected, for example, in a range of from pH 4 to pH 8, and may be in a range of from pH 5 to pH 8.

The culture temperature is, for example, from 20°C to 45°C, and preferably from 24°C to 37°C. The culture may be performed aerobically or anaerobically depending on the kinds of a microorganism.

The culture period is, for example, from 1 day to 7 days. The culture period may be set to maximize the production amount of the target enzyme.

The treated product of the recombinant microorganism refers to a product obtained by any treatment of the recombinant microorganism as long as the activities of the pyridoxine oxidase and the pyridoxamine synthetase produced by the recombinant microorganism are not lost. Examples of such treatment include one or more selected from the group consisting of heat treatment, cooling treatment, mechanical destruction, ultrasonic treatment, freeze-thaw treatment, drying treatment, pressurized or reduced pressure treatment, osmotic pressure treatment, autolysis, surfactant treatment, and enzyme treatment (for example, cell lysis treatment). Even in a case in which the recombinant microorganism per se is killed by such treatment, such a treated product can be used for a reaction as long as the activity of the enzyme produced by the microorganism remains.

The treated product of the culture refers to a product obtained by any treatment of the culture of the recombinant microorganism as long as the activities of the pyridoxine oxidase and the pyridoxamine synthetase produced by the recombinant microorganism are not lost. Examples of such treatment include one or more selected from the group consisting of heat treatment, cooling treatment, mechanical destruction of a cell, ultrasonic treatment, freeze-thaw treatment, drying treatment, pressurized or reduced pressure treatment, osmotic pressure treatment, cell autolysis, surfactant treatment, enzyme treatment (for example, cell destruction treatment), cell separation treatment, purification treatment, and extraction treatment. For example, a cell of the recombinant microorganism may be separated from a culture medium or the like, and the separated cell may be added to a reaction system. Means such as filtration or centrifugation can be used for such separation. Alternatively, purification treatment for separating, from a contaminant, the pyridoxine oxidase and the pyridoxamine synthetase, and, if present, the hydrogen peroxide-degrading enzyme, may be performed, and a solution including an enzyme obtained by the purification treatment may be added to the reaction system. Alternatively, an extract obtained by extracting the culture using an organic solvent such as methanol or acetonitrile, or a mixed solvent of an organic solvent and water may be added to the reaction system. It is also acceptable that such a purified product or extract does not include a cell of a recombinant microorganism. Even in a case in which the cell of the microorganism does not exist, such a purified product or extract can be used for a reaction as long as the enzymatic activity remains.

Such disruption or lysis treatment of a cell as described above can be performed by destructing the cell membrane of the recombinant microorganism according to a known method such as lysozyme treatment, freeze-thawing, or ultrasonic disruption.

It is preferable that the recombinant microorganism, the culture of the recombinant microorganism, or the treated product of the recombinant microorganism or the culture according to the disclosure is brought into contact with pyridoxine or a salt thereof under the following conditions.

The contact is preferably performed in a solution including pyridoxine or a salt thereof as a substrate. The pH of the solution is not particularly limited as long as the enzymatic activities of the pyridoxine oxidase and the pyridoxamine synthetase are maintained, and is preferably from pH 6.0 to pH 9.0, and more preferably from pH 7.0 to pH 8.5. The temperature of the solution is also not particularly limited as long as the enzymatic activities of the pyridoxine oxidase and the pyridoxamine synthetase are maintained, and is preferably from 20°C to 70°C, and more preferably from 25°C to 50°C.

As a medium for the solution, water or an aqueous medium, an organic solvent, or a mixed solvent of an organic solvent and water or an aqueous medium may be used. Examples of the aqueous medium include buffers such as a phosphate buffer, a HEPES (N-2-hydroxyethylpiperazine-N-ethanesulfonic acid) buffer, and a tris[tris(hydroxymethyl)aminomethane]hydrochloride buffer. Any organic solvent may be used as long as the solvent does not inhibit a reaction, and examples thereof include acetone, ethyl acetate, dimethyl sulfoxide, xylene, methanol, ethanol, and butanol.

The recombinant microorganism, the culture of the recombinant microorganism, or the treated product of the recombinant microorganism or the culture is brought into contact with pyridoxine or a salt thereof in the presence of oxygen. This is because the pyridoxine oxidase consumes oxygen when oxidizing pyridoxine or a salt thereof. Regarding the oxygen concentration in a reaction system, for example, a reaction can be conducted while a solution including the recombinant microorganism, the culture of the recombinant microorganism, or the treated product of the recombinant microorganism or the culture, and pyridoxine or a salt thereof is opened to the atmosphere, in other words, the solution is brought into contact with the atmosphere, or while the solution is brought into contact with a gas including from 0.1% to 20%, from 0.5% to 10%, or from 1% to 5% of oxygen by volume. The amount of dissolved oxygen in the solution may be from 0.1 mg to 13 mg oxygen/L, from 0.5 mg to 10 mg oxygen/L, or from 1 mg to 8 mg oxygen/L.

In a case in which the recombinant microorganism has a gene encoding a hydrogen peroxide-degrading enzyme capable of generating oxygen, the reaction can be conducted under a condition in which the oxygen concentration is reduced or a condition in which oxygen supply is reduced or blocked. For example, it is also possible to carry out the reaction under a condition in which a reaction system is sealed or under a condition in which a reaction system is purged with nitrogen (nitrogen substituted).

It is preferable that the recombinant microorganism, the culture of the recombinant microorganism, or the treated product of the recombinant microorganism or the culture according to the disclosure is brought into contact with pyridoxine or a salt thereof under a shaking or stirring condition. For example, such contact can be performed in a solution. For example, pyridoxine or a salt thereof may be added to a solution including the recombinant microorganism, the culture of the recombinant microorganism, or the treated product of the recombinant microorganism or the culture in the form of a substrate solution or in the form of a solid. An amino acid to be consumed by a pyridoxamine synthetase may be further added to a solution including the recombinant microorganism, the culture of the recombinant microorganism, or the treated product of the recombinant microorganism or the culture. The amino acid may be added, in a state of being included in a substrate solution containing pyridoxine or a salt thereof, to a solution including the recombinant microorganism, the culture of the recombinant microorganism, or the treated product of the recombinant microorganism or the culture together with the pyridoxine or a salt thereof. Alternatively, the amino acid may be added, in a state of being included in a substrate solution other than the substrate solution containing pyridoxine or a salt thereof or in the form of a solid, to a solution including the recombinant microorganism, the culture of the recombinant microorganism, or the treated product of the recombinant microorganism or the culture.

An acid or an alkali may be added to maintain the pH of a reaction liquid in an appropriate range at the initiation of the reaction or during the reaction. Examples of the alkali that can be added to the reaction liquid include an alkali metal hydroxide such as lithium hydroxide, sodium hydroxide, or potassium hydroxide; and one such as ammonium hydroxide, calcium hydroxide, dipotassium phosphate, disodium phosphate, potassium pyrophosphate, or ammonia which is dissolved in water to make the liquid basic. Examples of the acid that can be added to a reaction liquid include hydrochloric acid, sulfuric acid, nitric acid, acetic acid, and phosphoric acid.

The contact may be performed, for example, under an air atmosphere or under a partial deoxygenation atmosphere. The deoxidizing atmosphere can be achieved by substitution with an inert gas, pressure reduction, boiling, or a combination thereof. It is preferable at least to replace with an inert gas, or to use an inert gas atmosphere. Examples of the inert gas include nitrogen gas, helium gas, argon gas, and carbon dioxide, and nitrogen gas is preferable. Since the pyridoxine oxidase consumes oxygen when oxidizing pyridoxine, an atmosphere in which oxygen is not removed is preferable.

In a preferable embodiment, the recombinant microorganism, the culture of the recombinant microorganism, or the treated product of the recombinant microorganism or the culture to be used includes the pyridoxine oxidase and the pyridoxamine synthetase. For this reason, the pyridoxine oxidase and the pyridoxamine synthetase, which are present together in the reaction liquid, act cooperatively when contacting, and pyridoxamine or a salt thereof is produced with high production efficiency. Although it is not essential that the treated product of the recombinant microorganism or the treated product of the culture includes the recombinant microorganism in a living state, from the viewpoint of continuously supplying the substance involved in the reaction by metabolism, it is preferable to include the recombinant microorganism in a living state.

Regarding the timing of addition, the recombinant microorganism, the culture of the recombinant microorganism, or the treated product of the recombinant microorganism or the culture may be added all at once at the initiation of a reaction, or may be added in batches or continuously during a reaction. Similarly, pyridoxamine, which is a raw material, may be added all at once at the initiation of a reaction, or may be added in batches or continuously during a reaction.

The reaction liquid may include an amino acid to be consumed in the production of pyridoxamine or a salt thereof by a pyridoxamine synthetase. This amino acid may also be added all at once at the initiation of a reaction, or may be added in batches or continuously during a reaction. For example, in a case in which a pyridoxamine-pyruvate transaminase is used as a pyridoxamine synthetase, the reaction liquid may include one or more of L-alanine and D-alanine. In a case in which a pyridoxamine-oxaloacetate transaminase or an aspartate transaminase is used as a pyridoxamine synthetase, the reaction liquid may include at least one of L-aspartic acid, D-aspartic acid, L-glutamic acid, or D-glutamic acid. An amino acid may also exist as a salt depending on a surrounding environment; however, in the disclosure, a description of amino acid is given with inclusion of the mention of a salt. For example, the description of L-glutamic acid includes not only L-glutamic acid which has not formed salt, but also L-glutamic acid which has formed salt (for example, monosodium L-glutamate monohydrate). Examples of counter ions in the case of forming a salt include cations such as sodium ion and potassium ion, and anions such as chloride ion, acetate ion, and nitrate ion.

The concentration of pyridoxine or a salt thereof in the reaction liquid may be, for example, from 0.1 mM to 500 mM, or may be from 0.4 mM to 200 mM, from 0.5 mM to 100 mM, or from 0.8 mM to 50 mM. The enzymatic activity of pyridoxine oxidase tends to be inhibited when the concentration of pyridoxine or a salt thereof is increased. For this reason, it is preferable that the concentration of pyridoxine or a salt thereof in the reaction liquid is not excessively high. In other words, the concentration of pyridoxine or a salt thereof in the reaction liquid may be controlled, for example, to maintain the concentration within the above range. For example, the method of producing pyridoxamine or a salt thereof according to the disclosure may include either or both of the following (A) and (B):
(A) adding pyridoxine or a salt thereof continuously or in several batches to a solution including the recombinant microorganism, the culture of the recombinant microorganism, or the treated product of the recombinant microorganism or the culture; and
(B) controlling a molar concentration of an amino acid consumed by the pyridoxamine synthetase so as to be 1 or more times a molar concentration of pyridoxine or a salt thereof, in a solution including the recombinant microorganism, the culture of the recombinant microorganism, or the treated product of the recombinant microorganism or the culture.

Examples of the amino acids include L-alanine, D-alanine, L-glutamic acid, D-glutamic acid, L-aspartic acid, and D-aspartic acid.

For such control, for example, pyridoxine or a salt thereof may be added in several batches instead of being added all at once to the reaction liquid. Since the concentration of pyridoxine decreases as a reaction proceeds, excessively high concentrations of pyridoxine or a salt thereof can be avoided by adding pyridoxine or a salt thereof at intervals. Examples of such sequential addition include, for example, addition of pyridoxine or a salt thereof twice or more, preferably three or more times, at a time interval in the range of from 0.5 hours to 10 hours. Pyridoxine or a salt thereof may be continuously added to the reaction liquid. In the case of continuous addition, excessively high concentrations of pyridoxine or a salt thereof can be avoided by adjusting the addition rate. For this adjustment, the concentration of pyridoxine or a salt thereof in the reaction liquid may be measured at a specific timing or may be monitored continuously.

The concentration of the amino acid (the amino acid to be consumed in producing pyridoxamine or a salt thereof by a pyridoxamine synthetase) may be, for example, from 0.1 mM to 2,000 mM, or may be from 0.2 mM to 1,000 mM, from 0.4 mM to 500 mM, from 0.5 mM to 400 mM, from 1 mM to 300 mM, or from 2 mM to 250 mM. The method for adding the amino acid is not particularly limited, and the amino acid may be added all at once, may be added in several batches, or may be added continuously. Examples of such sequential addition include, for example, adding an amino acid twice or more, preferably three or more times at a time interval in the range of from 0.5 hours to 10 hours. In a case in which pyridoxine or a salt thereof is added in portions, the amino acid may be added simultaneously with the addition of pyridoxine or a salt thereof, or may be added separately from pyridoxine or a salt thereof.

The concentration (molar concentration) of the amino acid (the amino acid consumed when pyridoxamine or a salt thereof is produced by pyridoxamine synthetase) is preferably maintained higher than the concentration (molar concentration) of pyridoxine or a salt thereof. In a case in which the concentration of the amino acid is maintained higher than the concentration of pyridoxine or a salt thereof, the reaction equilibrium can be shifted in favor of pyridoxamine or a salt thereof, and the production efficiency of pyridoxamine or a salt thereof can be further improved. The molar concentration of the amino acid is preferably 1.0 time or more, more preferably 1.5 times or more, still more preferably 2.0 times or more, and still more preferably 5.0 times or more, and may be 10.0 times or more the molar concentration of pyridoxine or a salt thereof.

Examples of the method of bringing the recombinant microorganism, the culture of the recombinant microorganism, or the treated product of the recombinant microorganism or the culture into contact with pyridoxine or a salt thereof include: a method of adding the recombinant microorganism, the culture of the recombinant microorganism, or the treated product of the recombinant microorganism or the culture to a solution including pyridoxine or a salt thereof and allowing a reaction to proceed while stirring; a method of adding the recombinant microorganism, the culture of the recombinant microorganism, or the treated product of the recombinant microorganism or the culture to a solution including pyridoxine or a salt thereof and allowing a reaction to proceed while shaking; and a method of mixing the recombinant microorganism, the culture of the recombinant microorganism, or the treated product of the recombinant microorganism or the culture and pyridoxine or a salt thereof thoroughly in a solution, then allowing the mixture to stand still for allowing a reaction to proceed. From the viewpoint of reaction efficiency, preferable examples thereof include a method of adding the recombinant microorganism, the culture of the recombinant microorganism, or the treated product of the recombinant microorganism or the culture to a solution including pyridoxine or a salt thereof and allowing a reaction to proceed while stirring.

A reaction vessel that can be used for a reaction is not particularly limited. The reaction vessel is preferably a vessel in which the recombinant microorganism, the culture of the recombinant microorganism, or the treated product of the recombinant microorganism or the culture and the solution including pyridoxine or a salt thereof added thereto can be stirred enough to mix them sufficiently, and which has a temperature control function of keeping the temperature within an optimum temperature range for a pyridoxine oxidase and a pyridoxamine synthetase.

The contact time (reaction time) of the recombinant microorganism, the culture of the recombinant microorganism, or the treated product of the recombinant microorganism or the culture with pyridoxine or a salt thereof is not particularly limited as long as the enzymatic activities of the pyridoxine oxidase and the pyridoxamine synthetase are maintained. For example, the contact time may be from 30 minutes to 100 hours, and may be from 2 hours to 50 hours. The reaction may be performed with a batch method, may be performed with a semi-batch method in which either or both of a substrate, and the microorganism, the culture or the treated product are added in batches during the reaction, or may be performed with a continuous method. In the case of the semi-batch method or the continuous method, the upper limit of the reaction time is not particularly limited since an operation such as supplying either or both of a new raw material, and the recombinant microorganism, the culture, or the treated product is conducted.

In the above method, the use of the recombinant microorganism, the culture of the recombinant microorganism, or the treated product of the recombinant microorganism or the culture according to the disclosure, allows the production of pyridoxamine or a salt thereof inexpensively with high production efficiency by using pyridoxine or a salt thereof as a raw material, without the need to carry out a complicated step as in a chemical synthesis method. Pyridoxamine or a salt thereof obtained by the above-described method can be used, for example, for producing a product that utilizes its physiological activity. For example, the product can be used for an application such as prevention or treatment of a disease or schizophrenia caused by AGE accumulation such as diabetes mellitus, atherosclerosis, chronic renal failure, or Alzheimer's dementia, a health food, or a cosmetic.

The term "step" as used herein includes not only a separate step but also a step that is not clearly distinguished from other steps as long as the desired purpose of the step is achieved therefrom. As used herein, the notation "to" expressing a numerical range including the numerical values before and after "to", as the minimum value and the maximum value, respectively.

Herein, the amount of a component of a composition, when plural substances corresponding to the same component exist in the composition, the total amount of the component in the composition refers to a total amount of the plural substances in the composition, unless otherwise specified.

### EXAMPLES

Hereinafter, the present embodiment is described more specifically by referring to the following examples, but the disclosure is not limited at all to these examples. In addition,"%" indicating the amounts of components in compositions in examples is based on mass standard unless otherwise specified.

### <Analysis Conditions>

Pyridoxine hydrochloride, pyridoxal hydrochloride, and pyridoxamine dihydrochloride were quantified by high performance liquid chromatography. The analysis conditions thereof are as follows.
Column: Shodex (registered trademark) Asahipak ODP-50 6E (SHOWA DENKO K.K.)
Guard column: Shodex (registered trademark) Asahipak ODP-50G 6A (SHOWA DENKO K.K.)
Column temperature: 30°C
Pump flow rate: 1.0 mL/min
Eluent: 50 mM phosphate buffer (pH 2.0)
Detection: UV 254 nm

### Comparative Example 1: Production of pno Expression Strain

A synthetic DNA having the nucleotide sequence of SEQ ID NO:9 was obtained by custom synthesis of the nucleotide sequence of a pyridoxine 4-oxidase gene derived from *Microbacterium luteolum,* codon-optimized for *E. coli,* from GenScript. A DNA fragment including a target gene was amplified by PCR with an oligonucleotide having the nucleotide sequence of SEQ ID NO:12 and an oligonucleotide having the nucleotide sequence of SEQ ID NO:13 as primers using the synthetic DNA as a template. The amplified DNA fragment was treated with BamHI and SalI, and the obtained DNA fragment and a treated product of pUC18 (manufactured by Takara Bio Inc.) with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:9 according to a usual method of determining a nucleotide sequence. The obtained plasmid was named pUC18-pno. Here, pno is the abbreviated name of a pyridoxine-4-oxidase.

### Example 1: Production of ppat-pno Expression Strain

A synthetic DNA having the nucleotide sequence of SEQ ID NO:10 was obtained by custom synthesis of the nucleotide sequence of a pyridoxamine-pyruvate aminotransferase gene derived from *Mesorhizobium loti* MAFF303099, codon-optimized for *E. coli,* from GenScript. A DNA fragment including a target gene was amplified by PCR with an oligonucleotide having the nucleotide sequence of SEQ ID NO:14 and an oligonucleotide having the nucleotide sequence of SEQ ID NO:15 as primers using the synthetic DNA as a template. The amplified DNA fragment was treated with EcoRI and BamHI, and the obtained DNA fragment and a treated product obtained by treating the pUC18-pno produced in Comparative Example 1 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:10 according to a usual method of determining a nucleotide sequence. The obtained plasmid was named pUC 18-ppat-pno. Here, ppat is the abbreviated name of pyridoxamine-pyruvate aminotransferase.

### Example 2: Production of ppat-pno-kat Expression Strain

A synthetic DNA having the nucleotide sequence of SEQ ID NO:11 was obtained by custom synthesis of the nucleotide sequence of a catalase gene derived from *Listeria seeligeri,* codon-optimized for *E. coli,* from GenScript. The synthetic DNA was treated with SalI and HindIII, and the obtained DNA fragment and a treated product obtained by treating the pUC18-ppat-pno produced in Example 1 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner. Here, kat is the abbreviated name of a catalase.

The nucleotide sequence of the DNA fragment introduced into the plasmid was confirmed to be the nucleotide sequence of SEQ ID NO:11 according to a usual method of determining a nucleotide sequence. The obtained plasmid was named pUC 18-ppat-pno-kat.

### Example 3: Production of aspC-pno Expression Strain

A DNA fragment including a pyridoxamine-oxaloacetate transaminase gene derived from *Escherichia coli* W3110 was amplified by PCR with an oligonucleotide having the nucleotide sequence of SEQ ID NO:16 and an oligonucleotide having the nucleotide sequence of SEQ ID NO:17 as primers using the genomic DNA of *Eschrichia coli* W3110 as a template. The amplified DNA fragment was treated with EcoRI and BamHI, and the obtained DNA fragment and a treated product obtained by treating the pUC18-pno produced in Example 1 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:8 according to a usual method of determining a nucleotide sequence. The obtained plasmid was named pUC 18-aspC-pno. Here, aspC is the abbreviated name of pyridoxamine-oxaloacetate transaminase.

### Example 4: Production of aspC-pno-kat Expression Strain

A synthetic DNA having the nucleotide sequence of SEQ ID NO:11 was obtained by custom synthesis of the nucleotide sequence of a catalase gene derived from *Listeria seeligeri,* codon-optimized for *E. coli,* from GenScript. The synthetic DNA was treated with SalI and HindIII, and the obtained DNA fragment and a treated product obtained by treating the pUC18-aspC-pno produced in Example 3 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner. Here, kat is the abbreviated name of a catalase.

The nucleotide sequence of the DNA fragment introduced into the plasmid was confirmed to be the nucleotide sequence of SEQ ID NO:11 according to a usual method of determining a nucleotide sequence. The obtained plasmid was named aspC-pno-kat.

### Test 1: Production of Pyridoxamine Using Pyridoxine as Raw Material

DH5α transformed with each of pUC18 and the plasmids produced in Comparative Example 1 and Example 1 was inoculated into 100 mL of LB medium including 100 µg/ mL of ampicillin in a 500 mL baffled Erlenmeyer flask and cultured with shaking at 30°C for 24 hours. The culture solution was centrifuged at 8,000 rpm for 20 minutes, and bacterial cells obtained as a precipitate were suspended in 800 µL of water to prepare a bacterial cell suspension.

Pyridoxine hydrochloride and L-alanine were dissolved in water to prepare a substrate solution including pyridoxine hydrochloride (200 mM) and L-alanine (800 mM). The pH of the substrate solution was adjusted to pH 8.0 with sodium hydroxide. 100 µL of substrate solution and 300 µL of bacterial cell suspension were mixed in a 2.0 mL tube, and the mixture was shaken at 1,000 rpm and 37°C for 6 hours while the tube was uncapped. A part of the reaction liquid was collected, and analyzed under the analysis conditions described above. The reaction yield obtained as a result is set forth in Table 1. The yield set forth in Table 1 represents the ratio of the molar amount of obtained pyridoxamine dihydrochloride to the molar amount of pyridoxine hydrochloride in the substrate solution.

**Table 1**

| Plasmid | Yield |
|---|---|
| pUC18 | 0% |
| pUC18-pno | 23% |
| pUC18-ppat-pno | 84% |

As set forth in Table 1, pyridoxamine dihydrochloride was produced with high production efficiency in a case in which both of the gene encoding pyridoxine oxidase and the gene encoding pyridoxamine synthetase were extracellularly introduced. In the transformant including the plasmid produced in Comparative Example 1, it is assumed that a spontaneous reaction between pyridoxal and an amino group donor occurred. As described above, in the case of this spontaneous reaction, it is impossible to achieve high pyridoxamine production efficiency due to formation of a by-product. Also in the results of Table 1, when a transformant including the plasmid produced in Comparative Example 1 was used, high pyridoxamine production efficiency was not achieved.

### Test 2: Production of Pyridoxamine Using Pyridoxine as Raw Material

DH5α transformed with each of pUC18 and the plasmid produced in Example 3 was inoculated into 100 mL of LB medium including 100 µg/ mL of ampicillin in a 500 mL baffled Erlenmeyer flask and cultured with shaking at 30°C for 24 hours. The culture solution was centrifuged at 8,000 rpm for 20 minutes, and bacterial cells obtained as a precipitate were suspended in 800 µL of water to prepare a bacterial cell suspension.

Pyridoxine hydrochloride and monosodium L-glutamate monohydrate were dissolved in water to prepare a substrate solution including pyridoxine hydrochloride (200 mM) and monosodium L-glutamate monohydrate (800 mM). The pH of the substrate solution was adjusted to pH 8.0 with sodium hydroxide. 300 µL of substrate solution and 900 µL of bacterial cell suspension were mixed in a 2.0 mL tube, and the mixture was shaken at 1,000 rpm and 37°C for 24 hours while the tube was uncapped. Apart of the reaction liquid was collected, and analyzed under the analysis conditions described above. The reaction yield obtained as a result is set forth in Table 2. The yield set forth in Table 2 represents the ratio of the molar amount of obtained pyridoxamine dihydrochloride to the molar amount of pyridoxine hydrochloride in the substrate solution.

**Table 2**

| Plasmid | Yield |
|---|---|
| pUC18 | 0% |
| pUC18-acpC-pno | 60% |

As set forth in Table 2, pyridoxamine dihydrochloride was produced with high production efficiency in a case in which the gene encoding pyridoxine oxidase and the gene encoding pyridoxamine synthetase were extracellularly introduced.

### Test 3: Production of Pyridoxamine Using Pyridoxine as Raw Material (Examination of Oxygen Concentration)

DH5α transformed with the plasmid (pUC18-ppat-pno) produced in Example 1 was inoculated into 100 mL of LB medium including 100 µg/ mL of ampicillin in a 500 mL baffled Erlenmeyer flask, and cultured with shaking at 30°C for 24 hours. The culture solution was centrifuged at 8,000 rpm for 20 minutes, and bacterial cells obtained as a precipitate were suspended in 800 µL of water to prepare a bacterial cell suspension.

Pyridoxine hydrochloride and L-alanine were dissolved in water to prepare a substrate solution including pyridoxine hydrochloride (200 mM) and L-alanine (800 mM). The pH of the substrate solution was adjusted to pH 8.0 with sodium hydroxide. 100 µL of substrate solution and 300 µL of bacterial cell suspension were mixed in a 2.0 mL tube. The reaction was allowed to proceed with shaking at 1,000 rpm and 37°C for 24 hours under a condition in which the tube was uncapped, under a condition in which the tube was capped, or under a condition in which the tube was capped after purged with nitrogen . A part of the reaction liquid was collected, and analyzed under the analysis conditions described above. The reaction yield obtained as a result is set forth in Table 3. The yield set forth in Table 3 represents the ratio of the molar amount of obtained pyridoxamine dihydrochloride to the molar amount of pyridoxine hydrochloride in the substrate solution.

**Table 3**

| Plasmid | Yield |
|---|---|
| In presence of air (Open system) | 84% |
| In presence of air (Closed system) | 21% |
| Purged with nitrogen (Closed system) | 2% |

As set forth in Table 3, pyridoxamine or a salt thereof was produced at high yield under a condition in which oxygen supply was not limited than a condition in which oxygen supply was limited, in a case in which the recombinant microorganism into which the gene encoding pyridoxine oxidase and the gene encoding pyridoxamine synthetase had been extracellularly introduced was used to produce pyridoxamine or a salt thereof,.

### Test 4: Production of Pyridoxamine Using Pyridoxine as Raw Material (Examination of Effect by Hydrogen Peroxide-Degrading Enzyme)

DH5α transformed with each of the plasmid (pUC18-ppat-pno) produced in Example 1 and the plasmid (pUC18-ppat-pno-kat) produced in Example 2 was inoculated into 100 mL of LB medium including 100 µg/ mL of ampicillin in a 500 mL baffled Erlenmeyer 1 flask, and cultured with shaking at 30°C for 24 hours. The culture solution was centrifuged at 8,000 rpm for 20 minutes, and bacterial cells obtained as a precipitate were suspended in 800 µL of water to prepare a bacterial cell suspension.

Pyridoxine hydrochloride and L-alanine were dissolved in water to prepare a substrate solution including pyridoxine hydrochloride (200 mM) and L-alanine (800 mM). The pH of the substrate solution was adjusted to pH 8.0 with sodium hydroxide. 100 µL of substrate solution and 300 µL of bacterial cell suspension were mixed in a 2.0 mL tube. The reaction was allowed to react with shaking at 1,000 rpm for 24 hours at 37°C while the tube was capped. A part of the reaction liquid was collected, and analyzed under the analysis conditions described above. Table 4 shows the relative value (ratio of PM production rate) of the amount of pyridoxamine dihydrochloride produced using DH5α transformed with pUC18-ppat-pno-kat, with the amount of pyridoxamine dihydrochloride produced using DH5α transformed with pUC 18-ppat-pno set to 1.0.

**Table 4**

| Plasmid | Ratio of PM Production Rate |
|---|---|
| pUC18-ppat-pno | 1.0 |
| pUC18-ppat-pno-kat | 1.4 |

As set forth in Table 4, it is understood that in a case in which the recombinant microorganism according to the disclosure further includes the gene encoding a hydrogen peroxide-degrading enzyme, the production efficiency of pyridoxamine or a salt thereof under a condition in which oxygen supply is limited is further improved.

### Test 5: Production of Pyridoxamine Using Pyridoxine as Raw Material (Examination of Effect by Hydrogen Peroxide-Degrading Enzyme)

DH5α transformed with each of the plasmid (pUC18-aspC-pno) produced in Example 3 and the plasmid (pUC18-aspC-pno-kat) produced in Example 4 was inoculated into 100 mL of LB medium including 100 µg/mL of ampicillin in a 500 mL baffled Erlenmeyer flask and cultured with shaking at 30°C for 24 hours. The culture solution was centrifuged at 8,000 rpm for 20 minutes, and bacterial cells obtained as a precipitate were suspended in 800 µL of water to prepare a bacterial cell suspension.

Pyridoxine hydrochloride and monosodium L-glutamate monohydrate were dissolved in water to prepare a substrate solution including pyridoxine hydrochloride (200 mM) and monosodium L-glutamate monohydrate (800 mM). The pH of the substrate solution was adjusted to pH 8.0 with sodium hydroxide. 100 µL of the substrate solution and 300 µL of the bacterial cell suspension were mixed in a 2.0 mL tube. The reaction was allowed to proceed with shaking at 1,000 rpm for 24 hours at 37°C while the tube was capped. A part of the reaction liquid was collected, and analyzed under the analysis conditions described above. Table 5 shows the relative value (PM production rate ratio) of the amount of pyridoxamine dihydrochloride produced using DH5α transformed with pUC18-aspC-pno-kat, with the amount of pyridoxamine dihydrochloride produced using DH5α transformed with pUC 18-aspC-pno set to 1.0.

**Table 5**

| Plasmid | Ratio PM Production Rate |
|---|---|
| pUC18-aspC-pno | 1.0 |
| pUC18-aspC-pno-kat | 1.6 |

As set forth in Table 5, it is revealed that in a case in which the recombinant microorganism according to the disclosure further includes the gene encoding a hydrogen peroxide-degrading enzyme, the production efficiency of pyridoxamine or a salt thereof under a condition in which oxygen supply is limited is further improved.

As described above, according to the method of producing pyridoxamine or a salt thereof according to the disclosure, it is shown that pyridoxamine or a salt thereof can be inexpensively produced from pyridoxine or a salt thereof with high production efficiency.

### Test 6 (reference): Examination of Pyridoxine Concentration of pno

DH5α transformed with the plasmid (pUC18-pno) produced in Comparative Example 1 was inoculated into 2 mL of LB medium including 100 µg/ mL of ampicillin in a 500 mL test tube, and cultured with shaking at 37°C for 24 hours. The culture solution was centrifuged at 13,000 rpm for 3 minutes, and bacterial cells obtained as a precipitate were suspended in 1,000 µL of water to prepare a bacterial cell suspension.

Pyridoxine hydrochloride was dissolved in water to prepare a substrate solution including pyridoxine hydrochloride (500 mM). The substrate solution was adjusted to pH 8.0 with sodium hydroxide. 1,000 µL of reaction liquid was prepared in a 2.0 mL tube by nixing a predetermined amount of substrate solution, 100 µL of Tris-HCl (1 M), 100 µL of bacterial cell suspension, and water so that the concentration of pyridoxine hydrochloride in each reaction liquid was shown in Table 6. The reaction was allowed to proceed with shaking at 1,000 rpm and 37°C for 24 hours while the tube was capped. A part of the reaction liquid was collected, centrifuged, and the produced pyridoxal hydrochloride was quantified by measuring the absorbance at 415 nm. The amount of PL production at each pyridoxine hydrochloride concentration set forth in Table 6 is expressed in a relative value (ratio of PL production) to the amount of pyridoxal hydrochloride at a pyridoxine hydrochloride concentration of 0.8 mM.

**Table 6**

| Pyridoxine hydrochloride (mM) | Ratio of PL Production |
|---|---|
| 0.8 | 1.00 |
| 4.2 | 1.53 |
| 8.4 | 1.50 |
| 42.1 | 0.98 |
| 84.2 | 0.75 |
| 168.3 | 0.61 |

As set forth in Table 6, in a case in which the concentration of pyridoxine hydrochloride in the reaction liquid was increased to above a certain value, the PL production ratio was rather decreased. In other words, inhibition of the enzymatic activity of pyridoxine oxidase was observed.

### Test 7: Production of Pyridoxamine Using Pyridoxine as Raw Material (Raw Material Added All at Once)

DH5α transformed with the plasmid (pUC18-ppat-pno) produced in Example 1 was inoculated into 400 mL of LB medium including 100 µg/mL of ampicillin in a 2,000 mL baffled Erlenmeyer flask and cultured with shaking at 30°C for 24 hours. The culture solution was centrifuged at 8,000 rpm for 20 minutes, and bacterial cells obtained as a precipitate were suspended in 10 mL of water to prepare a bacterial cell suspension.

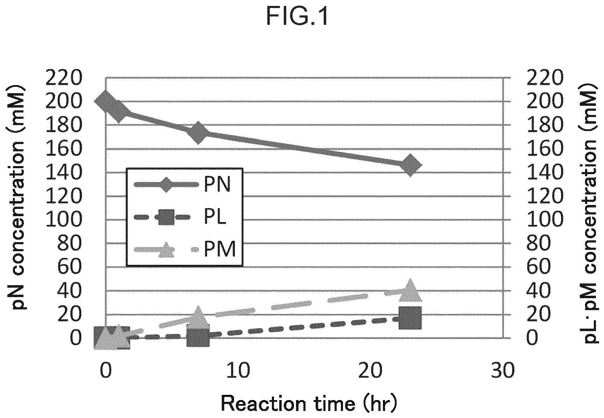

Pyridoxine hydrochloride was dissolved in water and adjusted to pH 8.0 with sodium hydroxide to prepare a substrate solution (1) including pyridoxine hydrochloride (500 mM). L-alanine was dissolved in water and adjusted to pH 8.0 with sodium hydroxide to prepare a substrate solution (2) including L-alanine (1,000 mM). Into a 100 mL baffled Erlenmeyer flask, 4.0 mL of cell suspension, 4.0 mL of the substrate solution (1), and 2.0 mL of the substrate solution (2) were added, which was allowed to react with shaking at 200 rpm for 24 hours at 37°C. A part of the reaction liquid was collected, and the concentration of pyridoxamine dihydrochloride was analyzed under the analysis conditions described above. The results are shown in Fig 1. In Figs 1 to 3, PN represents the concentration of pyridoxine hydrochloride, PL represents the concentration of pyridoxal hydrochloride, and PM represents the concentration of pyridoxamine dihydrochloride. Further, hr represents a unit of time "hour".

As shown in Fig. 1, production of pyridoxamine dihydrochloride proceeded even when high concentrations of pyridoxine hydrochloride and L-alanine were all added from the beginning.

### Test 8: Production of Pyridoxamine Using Pyridoxine as Raw Material (Pyridoxine Hydrochloride and L-Alanine Added in Several Batches)

DH5α transformed with the plasmid (pUC18-ppat-pno) produced in Example 1 was inoculated into 400 mL of LB medium including 100 µg/ mL of ampicillin in a 2,000 mL baffled Erlenmeyer flask and cultured with shaking at 30°C for 24 hours. The culture solution was centrifuged at 8,000 rpm for 20 minutes, and bacterial cells obtained as a precipitate were suspended in 10 mL of water to prepare a bacterial cell suspension.

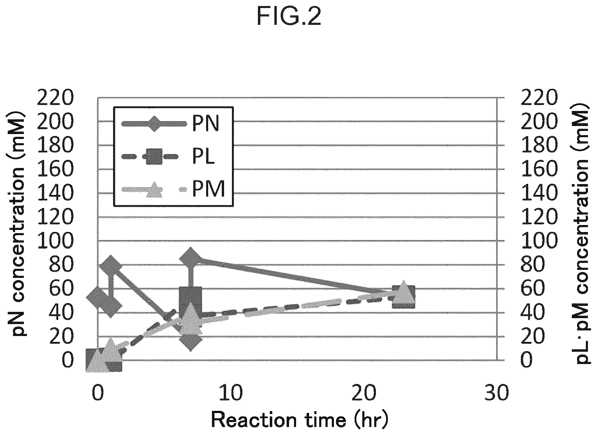

Pyridoxine hydrochloride was dissolved in water and adjusted to pH 8.0 with sodium hydroxide to prepare a substrate solution (1) including pyridoxine hydrochloride (500 mM). L-alanine was dissolved in water and adjusted to pH 8.0 with sodium hydroxide to prepare a substrate solution (2) including L-alanine (1,000 mM). Into a 100 mL baffled Erlenmeyer flask, 4.0 mL of cell suspension, 0.5 mL of the substrate solution (1), and 0.25 mL of the substrate solution (2) were added, which was allowed to react with shaking at 200 rpm at 37°C. One hour after the initiation of the reaction, 0.5 mL of the substrate solution (1) and 0.25 mL of the substrate solution were further added. Seven hours after the initiation of the reaction, 0.5 mL of the substrate solution (1) and 0.25 mL of the substrate solution (2) were further added. The reaction was continued until 24 hours after the initiation of the reaction. A part of the reaction liquid was collected, and the concentration of pyridoxamine dihydrochloride was analyzed under the analysis conditions described above. The results are shown in Fig 2. In this test, equimolar amounts of pyridoxine hydrochloride and L-alanine were added each time.

As shown in Fig. 2, the production efficiency of pyridoxamine dihydrochloride was further improved when a relatively large amount of pyridoxine hydrochloride was added in several batches rather than adding the whole amount from the beginning. It is assumed that the concentration of pyridoxine can be maintained relatively low due to the addition in several batches, and the pyridoxine oxidase activity can be more easily exhibited.

### Test 9: Production of Pyridoxamine Using Pyridoxine as Raw Material (Pyridoxine Hydrochloride and L-Alanine Added in Several Batched and L-alanine Added at Once)

DH5α transformed with the plasmid (pUC18-ppat-pno) produced in Example 1 was inoculated into 400 mL of LB medium including 100 µg/mL of ampicillin in a 2,000 mL baffled Erlenmeyer flask and cultured with shaking at 30°C for 24 hours. The culture solution was centrifuged at 8,000 rpm for 20 minutes, and bacterial cells obtained as a precipitate was suspended in 10 mL of water to prepare a bacterial cell suspension.

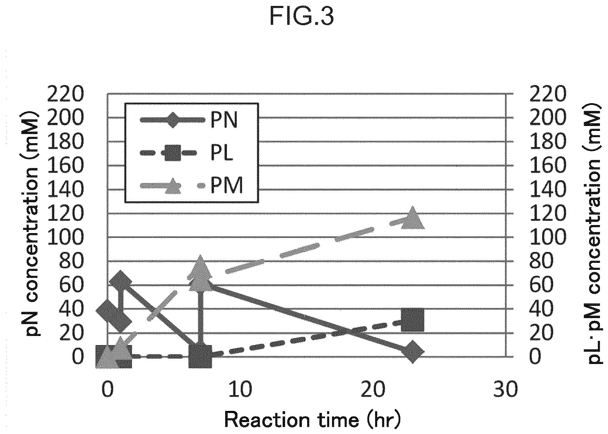

Pyridoxine hydrochloride was dissolved in water and adjusted to pH 8.0 with sodium hydroxide to prepare a substrate solution (1) including pyridoxine hydrochloride (500 mM). L-alanine was dissolved in water and adjusted to pH 8.0 with sodium hydroxide to prepare a substrate solution (2) including L-alanine (1,000 mM). Into a 100 mL baffled Erlenmeyer flask, 4.0 mL of cell suspension, 0.5 mL of the substrate solution (1), and 2.0 mL of the substrate solution (2) were added, which was allowed to react with shaking at 200 rpm at 37°C. One hour after the initiation of the reaction, 0.5 mL of the substrate solution (1) was further added. Seven hours after the initiation of the reaction, 0.5 mL of the substrate solution (1) was further added. The reaction was continued until 24 hours after the initiation of the reaction. A part of the reaction liquid was collected, and the concentration of pyridoxamine dihydrochloride was analyzed under the analysis conditions described above. The results are shown in Fig 3. In this test, the molar concentration of L-alanine was always maintained higher than the molar concentration of pyridoxine hydrochloride.

As can be seen from the comparison of Fig. 2 with Fig. 3, in a case in which the concentration of L-alanine was maintained higher than the concentration of pyridoxine hydrochloride, the production efficiency of pyridoxamine dihydrochloride was further improved even with a relatively large the amount of pyridoxine hydrochloride. It is assumed that due to the presence of L-alanine at a higher concentration than pyridoxine hydrochloride, the reaction equilibrium was more advantageously shifted in favor of the pyridoxamine generation.

### Reference Example 1: Production of ppat Expression Strain

A synthetic DNA having the nucleotide sequence of SEQ ID NO:10 was obtained by custom synthesis of the nucleotide sequence of a pyridoxamine-pyruvate aminotransferase gene derived from *Mesorhizobium loti* MAFF303099, codon-optimized for *E. coli,* from GenScript. A DNA fragment including a target gene was amplified by PCR with an oligonucleotide having the nucleotide sequence of SEQ ID NO:51 and an oligonucleotide having the nucleotide sequence of SEQ ID NO:52 as primers using the synthetic DNA as a template. The amplified DNA fragment was treated with EcoRI and BamHI, and the obtained DNA fragment and a treated product of PUC18 (manufactured by Takara Bio Inc.) with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:10 according to a usual method of determining a nucleotide sequence. The obtained plasmid was named pUC 18-ppat. Here, ppat is the abbreviated name of a pyridoxamine-pyruvate aminotransferase gene.

### Reference Example 2: Production of ppat-plr Expression Stain

A synthetic DNA having the nucleotide sequence of SEQ ID NO:53 was obtained by custom synthesis of the nucleotide sequence of a pyridoxal reductase gene derived from *Saccharomyces cerevisiae,* codon-optimized for *E. coli,* from GenScript. A DNA fragment including a target gene was amplified by PCR with an oligonucleotide having the nucleotide sequence of SEQ ID NO:54 and an oligonucleotide having the nucleotide sequence of SEQ ID NO:55 as primers using the synthetic DNA as a template. The amplified DNA fragment was treated with SalI and HindIII, and the obtained DNA fragment and a treated product obtained by treating the pUC18-ppat produced in Reference Example 1 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:53 according to a usual method of determining a nucleotide sequence. The obtained plasmid was named pUC 18-ppat-plr. Here, plr is the abbreviated name of a pyridoxal reductase gene (corresponding to pyridoxine dehydrogenase).

### Reference Example 3: Production of ppat-adh Expression Strain

A synthetic DNA having the nucleotide sequence of SEQ ID NO:56 was obtained by custom synthesis of the nucleotide sequence of an alanine dehydrogenase gene derived from *Shewanella sp.* AC10, into which a mutation as D198A in an encoded amino acid sequence was introduced, and which was codon-optimized for *E. coli,* from GenScript. A DNA fragment including a target gene was amplified by PCR with an oligonucleotide having the nucleotide sequence of SEQ ID NO:57 and an oligonucleotide having the nucleotide sequence of SEQ ID NO:58 as primers using the synthetic DNA as a template. The amplified DNA fragment was treated with BamHI and SalI, and the obtained DNA fragment and a treated product obtained by treating the pUC18-ppat produced in Reference Example 1 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:56 according to a usual method of determining a nucleotide sequence. The obtained plasmid was named pUC 18-ppat-adh. Here, adh is the abbreviated name of an alanine dehydrogenase gene.

### Reference Example 4: Production of ppat-adh-plr Expression Strain

A synthetic DNA having the nucleotide sequence of SEQ ID NO:56 was obtained by custom synthesis of the nucleotide sequence of an alanine dehydrogenase gene derived from *Shewanella sp.* AC10, into which a mutation as D198A in an encoded amino acid sequence was introduced, and which was codon-optimized for *E. coli,* from GenScript. A DNA fragment including a target gene was amplified by PCR with an oligonucleotide having the nucleotide sequence of SEQ ID NO:57 and an oligonucleotide having the nucleotide sequence of SEQ ID NO:58 as primers using the synthetic DNA as a template. The amplified DNA fragment was treated with BamHI and SalI, and the obtained DNA fragment and a treated product obtained by treating the pUC18-ppat-plr produced in Reference Example 2 with BamHI and SalI were ligated using Ligation High (manufactured by TOYOBO Co., Ltd.). *Escherichia coli* DH5α (manufactured by TOYOBO Co., Ltd.) was transformed with this ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:56 according to a usual method of determining a nucleotide sequence. The obtained plasmid was named pUC 18-ppat-adh-plr.

### Reference Example 5: Production of adh-plr Expression Plasmid

A pyridoxal reductase gene (plr) fragment recovered by treating pUC18-ppat-plr produced in Reference Example 2 with SalI and HindIII, an alanine dehydrogenase gene (adh) fragment recovered by treating pUC-ppat-adh produced in Reference Example 3 with BamHI and SalI, and a treated product of pUC18 with BamHI and HindIII were ligated using Ligation High (TOYOBO Co., Ltd.). *Escherichia coli* DH5α (manufactured by TOYOBO Co., Ltd.) was transformed with the ligation product. The transformant was cultured on LB agar medium, and a strain with a target plasmid was selected from ampicillin resistant strains. The plasmid was extracted from the transformant obtained in such a manner, and the obtained plasmid was named pUC18-adh-plr.

### Reference Example 6: Production of Msppat-adh-plr Expression Plasmid

A synthetic DNA having the nucleotide sequence of SEQ ID NO:32 was obtained by custom synthesis of a gene in which the nucleotide sequence of a pyridoxamine-pyruvate aminotransferase gene derived from *Mesorhizobium sp.* YR577 was designed to be codon-optimized for *E. coli,* and in which EcoRI was introduced into the 5' end and BamHI was introduced into the 3', from eurofins. A DNA fragment obtained by treating the synthetic DNA with EcoRI/BamHI, and a DNA fragment obtained by treating pUC18-adh-plr produced in Reference Example 5 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:32 according to a usual method of determining a nucleotide sequence. The obtained plasmid was named pUC 18-Msppat-adh-plr.

### Reference Example 7: Production of Psppat-adh-plr Expression Plasmid

A synthetic DNA having the nucleotide sequence of SEQ ID NO:33 was obtained by custom synthesis of a gene in which the nucleotide sequence of a pyridoxamine-pyruvate aminotransferase gene derived from *Pseudaminobacter salicylatoxidan* was designed to be codon-optimized for *E. coli,* and in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Reference Example 5 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:33 according to a usual method of determining a nucleotide sequence. The obtained plasmid was named pUC 18-Psppat-adh-plr.

### Reference Example 8: Production of Blppat-adh-plr Expression Plasmid

A synthetic DNA having the nucleotide sequence of SEQ ID NO:34 was obtained by custom synthesis of a gene in which the nucleotide sequence of a pyridoxamine-pyruvate aminotransferase gene derived from *Bauldia litoralis* was designed to be codon-optimized for *E. coli,* and in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Reference Example 5 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:34 according to a usual method of determining a nucleotide sequence. The obtained plasmid was named pUC 18-Blppat-adh-plr.

### Reference Example 9: Production of Ssppat-adh-plr Expression Plasmid

A synthetic DNA having the nucleotide sequence of SEQ ID NO:35 was obtained by custom synthesis of a gene in which the nucleotide sequence of a pyridoxamine-pyruvate aminotransferase gene derived from *Skermanella stibiiresistens* was designed to be codon-optimized for *E. coli,* and in which EcoRI was introduced into the 5' end and BamHI was introduced into the 3' end, from eurofins. A DNA fragment obtained by treating the synthetic DNA with EcoRI/BamHI, and a DNA fragment obtained by treating of pUC18-adh-plr produced in Reference Example 5 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:35 according to a usual method of determining a nucleotide sequence. The obtained plasmid was named pUC 18-Ssppat-adh-plr.

### Reference Example 10: Production of Rsppat-adh-plr Expression Plasmid

A synthetic DNA having the nucleotide sequence of SEQ ID NO:36 was obtained by custom synthesis of a gene in which the nucleotide sequence of a pyridoxamine-pyruvate aminotransferase gene derived from *Rhizobium sp.* AC44/96 was designed to be codon-optimized for *E. coli,* and in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Reference Example 5 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:36 according to a usual method of determining a nucleotide sequence. The obtained plasmid was named pUC 18-Rsppat-adh-plr.

### Reference Example 11: Production of Etppat-adh-plr Expression Plasmid

A synthetic DNA having the nucleotide sequence of SEQ ID NO:37 was obtained by custom synthesis of a gene in which the nucleotide sequence of a pyridoxamine-pyruvate aminotransferase gene derived from *Erwinia toletana* was designed to be codon-optimized for *E. coli,* and in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Reference Example 5 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:37 according to a usual method of determining a nucleotide sequence. The obtained plasmid was named pUC 18-Etppat-adh-plr.

### Reference Example 12: Production of Hgppat-adh-plr Expression Plasmid

A synthetic DNA having the nucleotide sequence of SEQ ID NO:38 was obtained by custom synthesis of a gene in which the nucleotide sequence of a pyridoxamine-pyruvate aminotransferase gene derived from *Herbiconiux ginsengi* was designed to be codon-optimized for *E. coli,* and in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Reference Example 5 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:38 according to a usual method of determining a nucleotide sequence. The obtained plasmid was named pUC 18-Hgppat-adh-plr.

### Test 10: Production of Pyridoxamine Using Pyridoxine as Raw Material

DH5α transformed with each of the plasmids produced in Reference Examples 6 to 12 was inoculated into 100 mL of LB medium including 100 µg/ mL of ampicillin in a 500 mL baffled Erlenmeyer flask and cultured with shaking at 30°C for 24 hours. 40 mL of culture solution was taken, and centrifuged at 5,000 rpm for 5 minutes, and bacterial cells obtained as a precipitate were suspended in 0.9 mL of water to prepare a bacterial cell suspension.

Pyridoxine hydrochloride and L-alanine were dissolved in water to prepare a substrate solution including pyridoxine hydrochloride (500 mM) and L-alanine (500 mM). The pH of the substrate solution was adjusted to pH 8.0 with 25% aqueous ammonia. 400 µL of substrate solution, 500 µL of water, and 100 µL of bacterial cell suspension were mixed, and allowed to react at 37°C for 1 hour. Apart of the reaction liquid was collected, and analyzed under the analysis conditions described above. The results are set forth in Table 7. The amount of produced pyridoxamine set forth in Table 7 is expressed in a relative amount, with the molar amount of pyridoxamine dihydrochloride produced by the ppat-adh-plr expression strain produced in Reference Example 4 set to 100.

**Table 7**

| Introduced plasmid | Amount of Produced Pyridoxamine (Relative Value) |
|---|---|
| pUC18-ppat-adh-plr | 100 |
| pUC18-Msppat-adh-plr | 46 |
| pUC18-Psppat-adh-plr | 113 |
| pUC18-Blppat-adh-plr | 34 |
| pUC18-Ssppat-adh-plr | 77 |
| pUC18-Rsppat-adh-plr | 34 |
| pUC18-Etppat-adh-plr | 109 |
| pUC18-Hgppat-adh-plr | 75 |

As set forth in Table 7, pyridoxamine dihydrochloride was able to be also produced from pyridoxine hydrochloride by introducing a pyridoxamine-pyruvate aminotransferase gene derived from *Mesorhizobium sp.* YR577, a pyridoxamine-pyruvate aminotransferase gene derived from *Pseudaminobacter salicylatoxidans,* a pyridoxamine-pyruvate aminotransferase gene derived from *Bauldia litoralis,* a pyridoxamine-pyruvate aminotransferase gene derived from *Skermanella stibiiresistens,* a pyridoxamine-pyruvate aminotransferase gene derived from *Rhizobium sp.* AC44/96, a pyridoxamine-pyruvate aminotransferase gene derived from *Erwinia toletana,* or a pyridoxamine-pyruvate aminotransferase gene derived from *Herbiconiux ginsengi,* as a gene encoding a pyridoxamine-pyruvate aminotransferase, instead of the pyridoxamine-pyruvate aminotransferase gene derived from *Mesorhizobium loti* MAFF303099 used in Reference Example 4. Although these Reference Examples are not examples using pyridoxine oxidase but examples using pyridoxine dehydrogenase, these Reference Examples show that these pyridoxamine-pyruvate aminotransferases can function as the pyridoxamine synthetase in the disclosure.

The disclosure of Japanese Patent Application No. 2017-095572 filed on May 12, 2017, is hereby incorporated by reference in its entirety.

All publications, patent applications, and technical standards mentioned in this specification are herein incorporated by reference to the same extent as if each individual document, patent application, or technical standard was specifically and individually indicated to be incorporated by reference.

## Claims

1. A recombinant microorganism, comprising:
a gene encoding a pyridoxine oxidase, and a gene encoding a pyridoxamine synthetase having an enzymatic activity of synthesizing pyridoxamine from pyridoxal,
wherein each of the gene encoding the pyridoxine oxidase and the gene encoding the pyridoxamine synthetase is introduced from outside of a bacterial cell, or is endogenous to the bacterial cell and has an enhanced expression.

2. The recombinant microorganism according to claim 1, wherein the pyridoxamine synthetase is a pyridoxamine-pyruvate transaminase, a pyridoxamine-oxaloacetate transaminase, an aspartate transaminase, or a pyridoxamine phosphate transaminase.

3. The recombinant microorganism according to claim 1 or 2, wherein the pyridoxine oxidase is represented by enzyme number EC 1.1.3.12.

4. The recombinant microorganism according to any one of claims 1 to 3, wherein the gene encoding the pyridoxine oxidase is derived from *Microbacterium luteolum.*

5. The recombinant microorganism according to any one of claims 1 to 4, wherein the gene encoding a pyridoxine oxidase:
(a) has a nucleotide sequence of SEQ ID NO:5;
(b) has a nucleotide sequence that hybridizes with DNA having a nucleotide sequence complementary to the nucleotide sequence of SEQ ID NO:5 under a stringent condition, and that encodes a protein having pyridoxine oxidase activity;
(c) has a nucleotide sequence encoding a protein that has an amino acid sequence of SEQ ID NO:1; or
(d) has a nucleotide sequence encoding a protein that has an amino acid sequence having 80% or more sequence identity with the amino acid sequence of SEQ ID NO:1 and that has pyridoxine oxidase activity.

6. The recombinant microorganism according to any one of claims 1 to 5, wherein the pyridoxamine synthetase comprises at least one of the following partial amino acid sequence (c), partial amino acid sequence (d), partial amino acid sequence (e), partial amino acid sequence (f), partial amino acid sequence (g), or partial amino acid sequence (h), and has an enzymatic activity of synthesizing pyridoxamine from pyridoxal:
(c) X₈X₉X₁₀X₁₁X₁₂X₁₃ (SEQ ID NO:39)
wherein X₈ represents L, M, I or V,
X₉ represents H or Q,
X₁₀ represents G, C or A,
X₁₁ represents E or D,
X₁₂ represents P or A, and
X₁₃ represents V, I, L or A;
(d) X₁₄X₁₅TPSGTX₁₆X₁₇ (SEQ ID NO:40)
wherein X₁₄ represents H or S,
X₁₅ represents D or E,
X₁₆ represents I, V, or L, and
X₁₇ represents N or T;
(e) X₁₈DX₁₉VSX₂₀X₂₁ (SEQ ID NO:41)
wherein X₁₈ represents V, I, or A,
X₁₉ represents A, T, or S,
X₂₀ represents S, A, or G, and
X₂₁ represents F, W, or V;
(f) X₂₂X₂₃X₂₄KCX₂₅GX₂₆X₂₇P (SEQ ID NO:42)
wherein X₂₂ represents G or S,
X₂₃ represents P, S, or A,
X₂₄ represents N, G, S, A, or Q,
X₂₅ represents L or M,
X₂₆ represents A, S, C, or G, and
X₂₇ represents P, T, S, or A;
(g) X₂₈X₂₉X₃₀X₃₁SX₃₂GX₃₃X₃₄ (SEQ ID NO:43)
wherein X₂₈ represents G or D,
X₂₉ represents V or I,
X₃₀ represents V, T, A, S, M, I, or L,
X₃₁ represents F, M, L, I, or V,
X₃₂ represents S, G, A, T, I, L, or H,
X₃₃ represents R, M, or Q, and
X₃₄ represents G, R, A, D, H, or K; and
(h) X₃₅X₃₆RX₃₇X₃₈HMGX₃₉X₄₀A (SEQ ID NO:44)
wherein X₃₅ represents L or V,
X₃₆ represents T, I, V, or L,
X₃₇ represents I, V, or L,
X₃₈ represents G or S,
X₃₉ represents P, A, or R, and
X₄₀ represents T, V, or S.

7. The recombinant microorganism according to any one of claims 1 to 6, wherein the pyridoxamine synthetase is represented by enzyme number EC 2.6.1.30.

8. The recombinant microorganism according to any one of claims 1 to 7, wherein the gene encoding a pyridoxamine synthetase is derived from *Mesorhizobium loti.*

9. The recombinant microorganism according to any one of claims 1 to 8, wherein the gene encoding a pyridoxamine synthetase:
(a) has a nucleotide sequence of any one of SEQ ID NO:6 or SEQ ID NO:25 to SEQ ID NO:31, or
has a region between an 18th nucleotide and a 3' end in a nucleotide sequence of SEQ ID NO:10, or a region between an 18th nucleotide and a 3' end in a nucleotide sequence of any one of SEQ ID NO:32 to SEQ ID NO:38;
(b) has a nucleotide sequence that hybridizes with DNA having a nucleotide sequence complementary to a nucleotide sequence of any one of SEQ ID NO:6 or SEQ ID NO:25 to SEQ ID NO:31, or with DNA having a nucleotide sequence complementary to a region between an 18th nucleotide and a 3' end in a nucleotide sequence of SEQ ID NO:10 or a region between an 18th nucleotide and a 3' end in a nucleotide sequence of any one of SEQ ID NO:32 to SEQ ID NO:38 under a stringent condition, and that encodes a protein having an enzymatic activity of synthesizing pyridoxamine from pyridoxal;
(c) has a nucleotide sequence encoding a protein that has an amino acid sequence of any one of SEQ ID NO:2 or SEQ ID NO:18 to SEQ ID NO:24; or
(d) has a nucleotide sequence encoding a protein that has an amino acid sequence having 80% or more sequence identity with at least one amino acid sequence selected from the group consisting of SEQ ID NO:2 and SEQ ID NO:18 to SEQ ID NO:24, and that has an enzymatic activity of synthesizing pyridoxamine from pyridoxal.

10. The recombinant microorganism according to any one of claims 1 to 5, wherein the pyridoxamine synthetase is represented by enzyme number EC 2.6.1.31 or EC 2.6.1.1.

11. The recombinant microorganism according to any one of claims 1 to 5 or claim 10, wherein the gene encoding a pyridoxamine synthetase is derived from *Escherichia coli.*

12. The recombinant microorganism according to any one of claims 1 to 5 or claims 10 to 11, wherein the gene encoding the pyridoxamine synthetase:
(a) has a nucleotide sequence of SEQ ID NO:8;
(b) has a nucleotide sequence that hybridizes with DNA having a nucleotide sequence complementary to the nucleotide sequence of SEQ ID NO:8 under a stringent condition, and that encodes a protein having an enzymatic activity of synthesizing pyridoxamine from pyridoxal;
(c) has a nucleotide sequence encoding a protein that has an amino acid sequence of SEQ ID NO:4; or
(d) has a nucleotide sequence encoding a protein that has an amino acid sequence having 80% or more sequence identity with the amino acid sequence of SEQ ID NO:4, and that has an enzymatic activity of synthesizing pyridoxamine from pyridoxal.

13. The recombinant microorganism according to any one of claims 1 to 11, further comprising a gene encoding a hydrogen peroxide-degrading enzyme that has an enzymatic activity of generating oxygen from hydrogen peroxide.

14. The recombinant microorganism according to claim 13, wherein the gene encoding the hydrogen peroxide-degrading enzyme is introduced from outside of a bacterial cell, or is endogenous to a bacterial cell and has an enhanced expression.

15. The recombinant microorganism according to claim 13 or 14, wherein the hydrogen peroxide-degrading enzyme is represented by enzyme number EC 1.11.1.6.

16. The recombinant microorganism according to any one of claims 13 to 15, wherein the gene encoding a hydrogen peroxide-degrading enzyme:
(a) has a nucleotide sequence of SEQ ID NO:7;
(b) hybridizes with DNA having a nucleotide sequence complementary to the nucleotide sequence of SEQ ID NO:7 under a stringent condition, and has an enzymatic activity of generating oxygen from hydrogen peroxide;
(c) has a nucleotide sequence encoding a protein that has an amino acid sequence of SEQ ID NO:3; or
(d) has a nucleotide sequence encoding a protein that has an amino acid sequence having 80% or more sequence identity with the amino acid sequence of SEQ ID NO:3, and that has an enzymatic activity of generating oxygen from hydrogen peroxide.

17. The recombinant microorganism according to any one of claims 1 to 16, comprising a recombinant *E. coli.*

18. A method of producing pyridoxamine or a salt thereof, the method comprising bringing the recombinant microorganism according to any one of claims 1 to 17, a culture of the recombinant microorganism, or a treated product of the recombinant microorganism or the culture, into contact with pyridoxine or a salt thereof to produce pyridoxamine or a salt thereof in the presence of oxygen.

19. The production method according to claim 18, wherein the recombinant microorganism, the culture of the recombinant microorganism, or the treated product of the recombinant microorganism or the culture, comprises the pyridoxine oxidase and the pyridoxamine synthetase.

20. The production method according to claim 19, wherein the recombinant microorganism, the culture of the recombinant microorganism, or the treated product of the recombinant microorganism or the culture, further comprises a hydrogen peroxide-degrading enzyme.

21. The production method according to any one of claims 18 to 20, wherein the treated product of the recombinant microorganism or the culture is a product treated by treatment comprising one or more selected from the group consisting of heat treatment, cooling treatment, mechanical destruction of a cell, ultrasonic treatment, freeze-thaw treatment, drying treatment, pressurized or reduced pressure treatment, osmotic pressure treatment, cell autolysis, surfactant treatment, enzyme treatment, cell separation treatment, purification treatment, and extraction treatment.

22. The production method according to any one of claims 18 to 21, the method comprising either or both of the following (A) and (B):
(A) adding pyridoxine or a salt thereof continuously or in several batches to a solution including the recombinant microorganism, the culture of the recombinant microorganism, or the treated product of the recombinant microorganism or the culture; and
(B) controlling a molar concentration of an amino acid consumed by the pyridoxamine synthetase so as to be 1 or more times a molar concentration of pyridoxine or a salt thereof, in a solution including the recombinant microorganism, the culture of the recombinant microorganism, or the treated product of the recombinant microorganism or the culture.

23. The production method according to claim 22, wherein the amino acid consumed by the pyridoxamine synthetase is L-alanine, D-alanine, L-glutamic acid, or D-glutamic acid.


SEQUENCE LISTING

<110>  Mitsui Chemicals Inc.
       Renascience Co., Ltd

<120>  Recombinant Microorganism and Method of Producing Pyridoxamine or
        Salt thereof Using the Recombinant Microorganism

<130>  MT-F03226-00

<150>  JP2017-095572
<151>  2017-05-12

<160>  58

<170>  PatentIn version 3.5

<210>  1
<211>  507
<212>  PRT
<213>  Microbacterium luteolum

<400>  1

Met Ala Gln Tyr Asp Val Ala Ile Ile Gly Ala Gly Ser Ala Gly Ala
1               5                   10                  15

Leu Ile Ala Ala Arg Leu Ser Glu Asp Pro Ala Arg Asn Val Leu Leu
            20                  25                  30

Ile Glu Ala Gly Gly Arg Pro Ser Asp Pro Asp Ile Leu Lys Pro Ser
        35                  40                  45

Met Trp Pro Ala Ile Gln His Arg Ser Tyr Asp Trp Asp Tyr Lys Thr
    50                  55                  60

Thr Pro Gln Glu Gly Ala Ala Gly Arg Ser Phe Ala Trp Ala Arg Gly
65                  70                  75                  80

Lys Gly Leu Gly Gly Ser Ser Leu Leu His Ala Met Gly Tyr Met Arg
                85                  90                  95

Gly His Pro Ala Asp Phe Ala Ala Trp Ala Glu Ala Thr Gly Asp Glu
            100                 105                 110

Arg Trp Ser Trp Glu Gly Leu Leu Pro Ser Phe Met Ala Asn Glu Asp
        115                 120                 125

His Val Ser Gly Gly Asp Gly Ile His Gly Lys Asp Gly Pro Met Pro
    130                 135                 140

Val Trp Ile Pro Asp Asp Glu Val Ser Pro Leu Thr Gln Ala Phe Met
145                 150                 155                 160

Thr Ala Gly Asn Ala Leu Gly Leu Pro Arg Ile Pro Asp His Asn Thr
                165                 170                 175

Gly Gln Met Ile Gly Val Thr Pro Asn Ser Leu Met Ile Arg Asp Gly
            180                 185                 190

Arg Arg Val Thr Val Ala Glu Ala Trp Leu Thr Pro Glu Val Cys Ala
        195                 200                 205

Arg Pro Asn Leu Thr Ile Met Thr Gly Thr Leu Thr Arg Arg Leu Lys
    210                 215                 220

Leu Glu Lys Ser His Val Ser Ala Ile Glu Leu Ala Gly Pro Glu Gly
225                 230                 235                 240

Leu Ala Thr Val Thr Ala Ser Glu Ile Ile Leu Ser Ala Gly Ser Leu
                245                 250                 255

Glu Ser Pro Ala Leu Leu Met Arg Ser Gly Ile Gly Arg Glu Asn Val
            260                 265                 270

Leu Arg Glu Ala Gly Val Thr Cys Arg Val Lys Ala Pro Glu Leu Gly
        275                 280                 285

Leu Asn Leu Met Asp His Leu Leu Gly Ala Gly Asn Leu Tyr Ala Thr
    290                 295                 300

Lys Lys His Leu Pro Pro Ser Arg Leu Gln His Ser Glu Ser Met Ala
305                 310                 315                 320

Tyr Met Arg Ala Gly Asp Phe Ser Ala Gly Gly Gln Pro Glu Ile Val
                325                 330                 335

Val Gly Cys Gly Val Ala Pro Ile Val Ser Glu Ser Phe Thr Ala Pro
            340                 345                 350

Ala Pro Gly Asn Ala Tyr Ser Phe Leu Phe Gly Val Thr His Pro Thr
        355                 360                 365

Ser Arg Gly Glu Ile Arg Ile Thr Gly Asp Ala Pro Asp Ser Pro Leu
    370                 375                 380

Ile Ile Asp Pro Arg Tyr Leu Gln Thr Gln Asn Asp Arg Asn Leu Phe
385                 390                 395                 400

Arg Ala Ala Leu Gly Ala Ala Arg Glu Ile Gly His Arg Pro Glu Leu
                405                 410                 415

Ala Glu Trp Arg Asp His Glu Ile Leu Pro Lys Ser Leu Ala Ala Ser
            420                 425                 430

Gln Asp Ile Asp Thr Phe Ile Ala Lys Ala Val Ile Thr His His His
        435                 440                 445

Pro Ser Gly Thr Cys Arg Met Gly Lys Asp Glu Met Ser Val Val Asp
    450                 455                 460

Ala Asp Leu Arg Leu Arg Gly Leu Asp Asn Leu Tyr Val Val Asp Gly
465                 470                 475                 480

Ser Val Leu Pro Ser Leu Thr Ala Gly Pro Ile His Ala Ala Val Gln
                485                 490                 495

Ala Ile Ala Glu Asn Phe Thr Thr Gly Phe Lys
            500                 505

<210> 2
<211> 393
<212> PRT
<213> Mesorhizobium loti

<400> 2

Met Met Arg Tyr Pro Glu His Ala Asp Pro Val Ile Thr Leu Thr Ala
1               5                   10                  15

Gly Pro Val Asn Ala Tyr Pro Glu Val Leu Arg Gly Leu Gly Arg Thr
            20                  25                  30

Val Leu Tyr Asp Tyr Asp Pro Ala Phe Gln Leu Leu Tyr Glu Lys Val
        35                  40                  45

Val Asp Lys Ala Gln Lys Ala Met Arg Leu Ser Asn Lys Pro Val Ile
    50                  55                  60

Leu His Gly Glu Pro Val Leu Gly Leu Glu Ala Ala Ala Ala Ser Leu
65                  70                  75                  80

Ile Ser Pro Asp Asp Val Val Leu Asn Leu Ala Ser Gly Val Tyr Gly
                85                  90                  95

Lys Gly Phe Gly Tyr Trp Ala Lys Arg Tyr Ser Pro His Leu Leu Glu
            100                 105                 110

Ile Glu Val Pro Tyr Asn Glu Ala Ile Asp Pro Gln Ala Val Ala Asp
        115                 120                 125

Met Leu Lys Ala His Pro Glu Ile Thr Val Val Ser Val Cys His His
    130                 135                 140

Asp Thr Pro Ser Gly Thr Ile Asn Pro Ile Asp Ala Ile Gly Ala Leu
145                 150                 155                 160

Val Ser Ala His Gly Ala Tyr Leu Ile Val Asp Ala Val Ser Ser Phe
                165                 170                 175

Gly Gly Met Lys Thr His Pro Glu Asp Cys Lys Ala Asp Ile Tyr Val
            180                 185                 190

Thr Gly Pro Asn Lys Cys Leu Gly Ala Pro Pro Gly Leu Thr Met Met
        195                 200                 205

Gly Val Ser Glu Arg Ala Trp Ala Lys Met Lys Ala Asn Pro Leu Ala
    210                 215                 220

Pro Arg Ala Ser Met Leu Ser Ile Val Asp Trp Glu Asn Ala Trp Ser
225                 230                 235                 240

Arg Asp Lys Pro Phe Pro Phe Thr Pro Ser Val Ser Glu Ile Asn Gly
                245                 250                 255

Leu Asp Val Ala Leu Asp Leu Tyr Leu Asn Glu Gly Pro Glu Ala Val
            260                 265                 270

Trp Ala Arg His Ala Leu Thr Ala Lys Ala Met Arg Ala Gly Val Thr
        275                 280                 285

Ala Met Gly Leu Ser Val Trp Ala Ala Ser Asp Ser Ile Ala Ser Pro
    290                 295                 300

Thr Thr Thr Ala Val Arg Thr Pro Asp Gly Val Asp Glu Lys Ala Leu
305                 310                 315                 320

Arg Gln Ala Ala Arg Ala Arg Tyr Gly Val Val Phe Ser Ser Gly Arg
                325                 330                 335

Gly Glu Thr Leu Gly Lys Leu Thr Arg Ile Gly His Met Gly Pro Thr
            340                 345                 350

Ala Gln Pro Ile Tyr Ala Ile Ala Ala Leu Thr Ala Leu Gly Gly Ala
        355                 360                 365

Met Asn Ala Ala Gly Arg Lys Leu Ala Ile Gly Lys Gly Ile Glu Ala

370                 375                 380

Ala Leu Ala Val Ile Asp Ala Asp Ala
385                 390

<210>  3
<211>  488
<212>  PRT
<213>  Listeria seeligeri

<400>  3

Met Thr Asp Arg Arg Asn Leu Thr Thr Asn Gln Gly Val Pro Ile Gly
1               5                   10                  15

Asp Asn Gln Asn Ser Met Thr Ala Gly Leu Lys Gly Pro Thr Leu Leu
            20                  25                  30

Glu Asp Tyr Val Leu Ile Glu Lys Leu Ala His Phe Asp Arg Glu Arg
        35                  40                  45

Val Pro Glu Arg Val Val His Ala Arg Gly Ala Gly Ala His Gly Lys
    50                  55                  60

Phe Val Thr Lys Lys Ser Met Lys Lys Tyr Thr Lys Ala Gln Phe Leu
65                  70                  75                  80

Gln Glu Glu Gly Thr Glu Thr Glu Val Phe Ala Arg Phe Ser Thr Val
                85                  90                  95

Ile His Gly Gln His Ser Pro Glu Thr Leu Arg Asp Pro Arg Gly Phe
            100                 105                 110

Ser Val Lys Phe Tyr Thr Glu Glu Gly Asn Tyr Asp Phe Val Gly Asn
        115                 120                 125

Asn Leu Pro Val Phe Phe Ile Arg Asp Ala Ile Lys Phe Pro Asp Val
    130                 135                 140

Ile His Ser Leu Lys Pro Asp Pro Arg Thr Asn Ile Gln Asp Gly Asn
145                 150                 155                 160

Arg Tyr Trp Asp Phe Phe Ser Leu Thr Pro Glu Ala Thr Thr Met Ile
                165                 170                 175

Thr Tyr Leu Phe Ser Asp Glu Gly Thr Pro Ala Ser Tyr Arg Glu Ile
            180                 185                 190

Arg Gly Ser Ser Val His Ala Phe Lys Trp Ile Asn Glu Glu Gly Lys

195                 200                 205

Thr Val Tyr Val Lys Leu Arg Trp Val Pro Lys Ala Gly Ile Val Asn
    210                 215                 220

Leu Ser Thr Asp Gln Ala Ala Gln Ile Gln Ala Lys Glu Phe Asn His
225                 230                 235                 240

Ala Ser Arg Asp Leu Tyr Glu Ala Ile Glu Asn Gly Asp Tyr Pro Glu
                245                 250                 255

Trp Asp Leu Tyr Val Gln Val Leu Asp Pro Lys Asp Leu Asp Asn Tyr
            260                 265                 270

Asp Phe Asn Pro Leu Asp Ala Thr Lys Asp Trp Phe Glu Asp Val Phe
        275                 280                 285

Pro Tyr Glu His Val Gly Thr Met Thr Leu Asn Arg Asn Pro Asp Asn
    290                 295                 300

Ile Phe Ala Glu Thr Glu Ser Val Gly Phe Asn Pro Gly Val Leu Val
305                 310                 315                 320

Pro Gly Met Leu Pro Ser Glu Asp Arg Val Leu Gln Gly Arg Leu Phe
                325                 330                 335

Ser Tyr Ser Asp Thr Gln Arg His Arg Val Gly Pro Asn Tyr Leu Gln
            340                 345                 350

Leu Pro Ile Asn Ser Pro Lys Thr Pro Val Asp Asn Asn Gln Arg Asp
        355                 360                 365

Gly Gln Met Pro Phe Lys Gln Gln Thr Ser Ser Ile Asn Tyr Glu Pro
    370                 375                 380

Asn Ser Tyr Asp Thr Glu Pro Lys Glu Asn Pro Ala Tyr Ile Glu Pro
385                 390                 395                 400

Glu Gln Glu Ile Arg Gly Asp Ile Ser Gly Arg Leu Val Ala Glu Lys
                405                 410                 415

Pro Asn Asn Phe Gly His Ala Lys Glu Val Trp Lys Arg Tyr Ser Asp
            420                 425                 430

Ala Glu Arg Ala Ala Leu Val Lys Asn Ile Val Asp Asp Trp Glu Gly
        435                 440                 445

Val Arg Glu Asp Ile Lys Ile Arg Asn Leu Arg Asn Phe Tyr Gln Val
    450                 455                 460

Glu Pro Glu Phe Ala Glu Arg Val Ala Ala Gly Thr Gly Ile Asn Leu
465                 470                 475                 480

Ala Glu His Val Ile Asp Leu Lys
                485

<210>  4
<211>  396
<212>  PRT
<213>  Escherichia coli

<400>  4

Met Phe Glu Asn Ile Thr Ala Ala Pro Ala Asp Pro Ile Leu Gly Leu
1               5                   10                  15

Ala Asp Leu Phe Arg Ala Asp Glu Arg Pro Gly Lys Ile Asn Leu Gly
            20                  25                  30

Ile Gly Val Tyr Lys Asp Glu Thr Gly Lys Thr Pro Val Leu Thr Ser
        35                  40                  45

Val Lys Lys Ala Glu Gln Tyr Leu Leu Glu Asn Glu Thr Thr Lys Asn
    50                  55                  60

Tyr Leu Gly Ile Asp Gly Ile Pro Glu Phe Gly Arg Cys Thr Gln Glu
65                  70                  75                  80

Leu Leu Phe Gly Lys Gly Ser Ala Leu Ile Asn Asp Lys Arg Ala Arg
                85                  90                  95

Thr Ala Gln Thr Pro Gly Gly Thr Gly Ala Leu Arg Val Ala Ala Asp
            100                 105                 110

Phe Leu Ala Lys Asn Thr Ser Val Lys Arg Val Trp Val Ser Asn Pro
        115                 120                 125

Ser Trp Pro Asn His Lys Ser Val Phe Asn Ser Ala Gly Leu Glu Val
    130                 135                 140

Arg Glu Tyr Ala Tyr Tyr Asp Ala Glu Asn His Thr Leu Asp Phe Asp
145                 150                 155                 160

Ala Leu Ile Asn Ser Leu Asn Glu Ala Gln Ala Gly Asp Val Val Leu
                165                 170                 175

Phe His Gly Cys Cys His Asn Pro Thr Gly Ile Asp Pro Thr Leu Glu
            180                 185                 190

Gln Trp Gln Thr Leu Ala Gln Leu Ser Val Glu Lys Gly Trp Leu Pro
        195                 200                 205

Leu Phe Asp Phe Ala Tyr Gln Gly Phe Ala Arg Gly Leu Glu Glu Asp
    210                 215                 220

Ala Glu Gly Leu Arg Ala Phe Ala Ala Met His Lys Glu Leu Ile Val
225                 230                 235                 240

Ala Ser Ser Tyr Ser Lys Asn Phe Gly Leu Tyr Asn Glu Arg Val Gly
                245                 250                 255

Ala Cys Thr Leu Val Ala Ala Asp Ser Glu Thr Val Asp Arg Ala Phe
            260                 265                 270

Ser Gln Met Lys Ala Ala Ile Arg Ala Asn Tyr Ser Asn Pro Pro Ala
        275                 280                 285

His Gly Ala Ser Val Val Ala Thr Ile Leu Ser Asn Asp Ala Leu Arg
    290                 295                 300

Ala Ile Trp Glu Gln Glu Leu Thr Asp Met Arg Gln Arg Ile Gln Arg
305                 310                 315                 320

Met Arg Gln Leu Phe Val Asn Thr Leu Gln Glu Lys Gly Ala Asn Arg
                325                 330                 335

Asp Phe Ser Phe Ile Ile Lys Gln Asn Gly Met Phe Ser Phe Ser Gly
            340                 345                 350

Leu Thr Lys Glu Gln Val Leu Arg Leu Arg Glu Glu Phe Gly Val Tyr
        355                 360                 365

Ala Val Ala Ser Gly Arg Val Asn Val Ala Gly Met Thr Pro Asp Asn
    370                 375                 380

Met Ala Pro Leu Cys Glu Ala Ile Val Ala Val Leu
385                 390                 395

<210>  5
<211>  1524
<212>  DNA
<213>  Microbacterium luteolum

<400>  5
atggctcagt atgatgtggc gatcattggt gctggctctg ccggcgccct gatagcagcg        60

cgtctaagcg aagatccggc ccgtaatgtt ctgctaatcg aagctggtgg gagaccatca 120

gacccggaca ttttaaagcc ctctatgtgg cctgcgatcc agcatcgcag ctatgattgg 180

gattacaaaa cgacacctca ggaaggtgcc gctggcagaa gttttgcctg ggctcgcggc 240

aaaggtcttg gtggatcaag cctgctccac gctatgggct atatgcgtgg gcacccggca 300

gatttcgctg catgggcaga agccacgggc gatgagcgct ggagctggga aggtttattg 360

ccatctttca tggcaaatga agaccatgtg tcgggcggcg atggtattca cggcaaagac 420

ggtccgatgc ccgtctggat accagacgac gaagtcagtc cactgacaca ggcattcatg 480

acagcaggca atgcactcgg cctgccgcgt atcccggacc acaataccgg ccaaatgatt 540

ggggtcacac ccaattccct gatgatccgt gatgggcgtc gcgtcaccgt tgctgaagca 600

tggctcacgc ccgaagtttg cgcgcgtccg aacctgacga tcatgacagg cacgttgacc 660

cgccgcctta aactggaaaa atcacacgtt tcagcaattg aactcgcagg cccggaagga 720

ctagctaccg tcacggcaag cgaaatcatt ttgtccgctg gttcattgga aagccccgca 780

cttttaatgc gctctggcat agggcgcgaa aatgtactcc gcgaagccgg tgtcacttgc 840

cgggtcaaag cgccagaact ggggcttaac ctgatggatc atctgcttgg ggcgggtaat 900

ctttatgcca ctaaaaagca tttgcctccg tcccgtctgc aacattcgga aagcatggcg 960

tatatgcgtg ccggagattt ctcagccggt ggccagccgg aaattgtcgt tggctgcggc 1020

gtggctccaa tcgtttccga gagctttact gcacctgctc cgggtaacgc ctattccttc 1080

ctttttggtg ttactcaccc tacaagtcga ggcgaaatac gcataaccgg agatgcaccg 1140

gacagcccac ttataattga ccctcgatat cttcagaccc aaaatgatag aaacctgttt 1200

cgagcagcgc tcggtgcggc acgtgaaatc ggacatcgcc ctgaattagc tgaatggcgt 1260

gaccacgaaa tactccccaa gtcgcttgca gcaagccaag acatcgacac ttttattgcg 1320

aaggcagtga tcacacatca ccacccttcc gggacatgcc gtatgggtaa ggatgaaatg 1380

tctgtcgttg atgctgatct gcgtctgcgc ggattggaca acctgtatgt cgtcgatggt 1440

tcagttttgc caagcctgac tgcgggccct atccacgccg ccgtacaggc aatcgcagaa 1500

aatttcacaa caggattcaa ataa 1524

<210> 6
<211> 1182
<212> DNA
<213> Mesorhizobium loti

<400> 6
atgatgcgct atcccgaaca tgccgatccg gtcatcaccc tgaccgcggg gccggtgaac 60

gcctatccag aagtgttgcg cggcctcggc cgtacggtgc tttatgatta cgatcctgca 120

ttccagctcc tttacgagaa ggtggtcgac aaggcgcaga aggcgatgcg gctgtcgaac 180

aagccggtca tcctgcatgg cgagccggtg ctcggcctcg aagcggcggc ggcgtcgctg 240

atctcgcccg acgacgttgt gctcaatctt gcctcgggcg tctacggcaa gggctttggc 300

tattgggcga agcggtactc gccgcatctg ctcgagatcg aagtgcccta taacgaggcg 360

atcgatccgc aggcggtcgc cgacatgctc aaggcgcatc cggagatcac cgttgtgtcg 420

gtctgtcatc acgacacgcc gtcgggcacc atcaatccga tcgacgccat cggcgcgctg 480

gtctcggcgc atggcgccta tctgatcgtc gacgcggtct cgtcctttgg cggcatgaag 540

acgcatcccg aggattgcaa agccgatatt tatgtcaccg gccccaacaa atgcctgggc 600

gcccctcccg gcctcaccat gatgggcgtc agcgagcggg cctgggccaa gatgaaggcc 660

aaccccctgg cgccgcgcgc atcgatgctg agcattgtcg actgggaaaa tgcctggtcg 720

agggacaagc cgtttccgtt cacgccgtcg gtctcggaga tcaacgggct cgacgtcgcg 780

ctcgatcttt acctcaatga gggaccggag gcggtatggg cgcgccatgc gctcaccgcc 840

aaggccatgc gagcgggcgt caccgcgatg ggcctgtcgg tctgggccgc cagcgacagc 900

atcgcgtcgc cgaccaccac cgccgtccgc acccccgatg gtgtcgacga gaaggcgctt 960

cgccaggccg cgcgtgcccg ttacggcgtg gtgttttcgt cggggcgggg cgagacgttg 1020

gggaagctga cgcgcatcgg ccatatgggg ccgaccgccc agccgatcta cgcgatcgcg 1080

gcgctgacgg cacttggcgg cgccatgaac gcggccggcc ggaaactcgc aatcggcaaa 1140

ggcatcgagg cggcgctggc cgtaatcgac gccgacgcct ga 1182

<210> 7
<211> 1467
<212> DNA
<213> Listeria seeligeri

<400> 7
atgaccgata gaagaaactt aacgacgaat caaggagtgc cgattgggga caaccaaaat 60

tcgatgacag cgggattaaa aggaccaact ttgttagaag attatgtgtt aattgagaaa 120

ttggcgcatt ttgatagaga acgtgttcca gaacgtgtgg tgcatgctcg tggtgctggt 180

gcgcacggca aatttgtaac gaaaaaaagc atgaaaaaat atacgaaagc acaattttta 240

caagaagaag gaacagagac agaggttttt gcgcgttttt ctacagtaat ccatggtcaa 300

cattcaccag aaacgcttcg tgatccacgt ggtttttcgg ttaaatttta tacggaagaa 360

ggtaactatg actttgtcgg taacaactta cctgtattct ttatccgtga tgctatcaag 420

tttcctgacg taattcactc cttgaagcca gatccacgta ctaacattca agatggcaat 480

cgttactggg acttctttag tttaactccg gaagcgacga cgatgattac ttatttattt 540

agcgatgagg ggactccggc atcttaccgc gaaattcgtg gttcaagcgt acatgccttt 600

aaatggataa acgaagaagg taagactgtt tatgtaaaac tacgctgggt tccaaaagca 660

ggaatcgtca atctttcaac tgatcaagca gcacaaattc aagcaaaaga atttaaccat 720

gctagtcgcg atttgtatga agcaattgag aatggtgatt atccagagtg ggatttatat 780

gtgcaagtgc tagatccaaa agacttggat aattacgact tcaatccgct tgatgcaacc 840

aaagactggt ttgaagatgt atttccatac gagcatgttg gaacaatgac attaaatcgt 900

aatccggata atatttttgc tgaaacagaa tcagttggct ttaatccagg tgtgcttgtg 960

ccggggatgt taccttctga ggaccgtgta ctacaggggc gattgttctc ttactctgat 1020

acgcaaagac accgcgttgg acctaactac ttacaattac caatcaacag cccaaaaact 1080

cctgttgata acaaccaacg tgatggacag atgccgttta aacagcaaac aagttcgatt 1140

aattatgaac caaatagtta tgatacagaa ccaaaagaaa accctgcata tatcgagcct 1200

gagcaagaaa ttcgtgggga tatctctggc cgactagtgg cagaaaagcc aaataacttt 1260

ggtcatgcta aagaagtttg gaagcgttac tcagatgcag aacgtgcggc tcttgtgaaa 1320

aatattgtag acgattggga aggtgtgcgc gaagatatta agattcgcaa cttgcgcaat 1380

ttctatcaag tagagccgga atttgcagaa cgtgtggctg ctggaactgg aattaacctt 1440

gctgaacatg tgatagattt aaaataa 1467

<210> 8
<211> 1191
<212> DNA
<213> Escherichia coli

<400> 8
atgtttgaga acattaccgc cgctcctgcc gacccgattc tgggcctggc cgatctgttt 60

cgtgccgatg aacgtcccgg caaaattaac ctcgggattg gtgtctataa agatgagacg 120

ggcaaaaccc cggtactgac cagcgtgaaa aaggctgaac agtatctgct cgaaaatgaa 180

accaccaaaa attacctcgg cattgacggc atccctgaat ttggtcgctg cactcaggaa 240

ctgctgtttg gtaaaggtag cgccctgatc aatgacaaac gtgctcgcac ggcacagact 300

ccggggggca ctggcgcact acgcgtggct gccgatttcc tggcaaaaaa taccagcgtt 360

aagcgtgtgt gggtgagcaa cccaagctgg ccgaaccata agagcgtctt taactctgca 420

ggtctggaag ttcgtgaata cgcttattat gatgcggaaa atcacactct tgacttcgat 480

gcactgatta acagcctgaa tgaagctcag gctggcgacg tagtgctgtt ccatggctgc 540

tgccataacc caaccggtat cgaccctacg ctggaacaat ggcaaacact ggcacaactc 600

tccgttgaga aaggctggtt accgctgttt gacttcgctt accagggttt tgcccgtggt 660

ctggaagaag atgctgaagg actgcgcgct ttcgcggcta tgcataaaga gctgattgtt 720

gccagttcct actctaaaaa ctttggcctg tacaacgagc gtgttggcgc ttgtactctg 780

gttgctgccg acagtgaaac cgttgatcgc gcattcagcc aaatgaaagc ggcgattcgc 840

gctaactact ctaacccacc agcacacggc gcttctgttg ttgccaccat cctgagcaac 900

gatgcgttac gtgcgatttg ggaacaagag ctgactgata tgcgccagcg tattcagcgt 960

atgcgtcagt tgttcgtcaa tacgctgcag gaaaaaggcg caaaccgcga cttcagcttt 1020

atcatcaaac agaacggcat gttctccttc agtggcctga caaaagaaca agtgctgcgt 1080

ctgcgcgaag agtttggcgt atatgcggtt gcttctggtc gcgtaaatgt ggccgggatg 1140

acaccagata acatggctcc gctgtgcgaa gcgattgtgg cagtgctgta a 1191

<210> 9
<211> 1542
<212> DNA
<213> Artificial

<220>
<223> Artificial Fragment for Pyridoxine Oxygenase

<400> 9
acaaaaagga taaaacaaat ggctcaatat gatgtagcta taataggagc agggagtgct 60

ggagcattaa tagctgcgcg tctgagcgaa gatccggcgc gtaacgtgct gctgattgaa 120

gcgggtggcc gtccgagcga cccggatatc ctgaagccga gcatgtggcc ggcgattcag 180

caccgtagct acgactggga ttataagacc accccgcaag agggtgcggc gggccgtagc 240

ttcgcgtggg cgcgtggtaa aggtctgggt ggcagcagcc tgctgcatgc gatgggctac 300

atgcgtggtc acccggcgga ttttgcggcg tgggcggagg cgaccggtga cgaacgttgg 360

agctgggagg gtctgctgcc gagctttatg gcgaacgaag accacgtgag cggtggcgat 420

ggtattcacg gcaaagacgg tccgatgccg gtgtggattc cggacgatga agttagcccg 480

ctgacccagg cgtttatgac cgcgggtaac gcgctgggtc tgccgcgtat cccggatcac 540

aacaccggcc aaatgattgg tgtgaccccg aacagcctga tgatccgtga cggccgtcgt 600

gtgaccgttg cggaagcgtg gctgaccccg gaagtgtgcg cgcgtccgaa cctgaccatt 660

atgaccggta ccctgacccg tcgtctgaag ctggagaaga gccacgttag cgcgattgaa 720

ctggcgggtc cggagggtct ggcgaccgtt accgcgagcg aaatcattct gagcgcgggt 780

agcctggaga gcccggcgct gctgatgcgt agcggtattg gccgtgaaaa cgtgctgcgt 840

gaagcgggcg tgacctgccg tgttaaggcg ccggaactgg gtctgaacct gatggatcac 900

ctgctgggtg cgggcaacct gtacgcgacc aagaaacacc tgccgccgag ccgtctgcag 960

cacagcgaga gcatggcgta tatgcgtgcg ggtgacttca gcgcgggtgg ccaaccggaa 1020

attgtggttg gttgcggcgt ggcgccgatc gttagcgaga gctttaccgc gccggcgccg 1080

ggtaacgcgt acagcttcct gtttggtgtt acccacccga ccagccgtgg cgaaatccgt 1140

attaccggtg acgcgccgga tagcccgctg atcattgatc cgcgttatct gcagacccaa 1200

aacgaccgta acctgttccg tgcggcgctg ggtgcggcgc gtgaaattgg tcaccgtccg 1260

gagctggcgg aatggcgtga tcacgagatt ctgccgaaga gcctggcggc gagccaggac 1320

atcgatacct ttattgcgaa agcggtgatt acccatcatc acccgagcgg tacctgccgt 1380

atgggtaaag acgagatgag cgtggttgac gcggatctgc gtctgcgtgg cctggataac 1440

ctgtatgtgg ttgacggtag cgttctgccg agcctgaccg cgggtccgat tcacgcggcg 1500

gttcaggcga ttgcggaaaa ctttaccacc ggcttcaagt aa 1542

<210> 10
<211> 1199
<212> DNA
<213> Artificial

<220>
<223> Artificial Fragment for Pyridoxamine-Pyruvate Transaminase

<400> 10
acaaaaagga taaaacaatg atgaggtatc ccgaacacgc tgatccagta ataactttaa 60

cagcaggacc cgtaaacgcg tatccggaag ttctgcgtgg tctgggccgt accgttctgt 120

acgactatga tccggcgttc cagctgctgt acgagaaggt ggttgataag gcgcaaaaag 180

cgatgcgtct gagcaacaaa ccggtgattc tgcacggcga accggttctg ggtctggaag 240

cggcggcggc gagcctgatc agcccggacg atgtggttct gaacctggcg agcggcgtgt 300

acggcaaggg ctttggttac tgggcgaaac gttatagccc gcacctgctg gagattgaag 360

tgccgtataa cgaggcgatt gacccgcagg cggttgcgga tatgctgaag gcgcacccgg 420

aaatcaccgt ggttagcgtg tgccaccacg acaccccgag cggtaccatc aacccgattg 480

acgcgattgg tgcgctggtt agcgcgcacg gtgcgtacct gattgtggac gcggttagca 540

gcttcggtgg catgaagacc cacccggagg actgcaaagc ggatatctat gtgaccggtc 600

cgaacaaatg cctgggtgct ccgccgggtc tgaccatgat gggtgttagc gaacgtgcgt 660

gggcgaagat gaaagcgaac ccgctggcgc cgcgtgcgag catgctgagc attgtggact 720

gggagaacgc gtggagccgt gataagccgt tcccgtttac cccgagcgtg agcgaaatca 780

acggcctgga cgttgcgctg gatctgtatc tgaacgaagg tccggaagcg gtttgggcgc 840

gtcatgcgct gaccgcgaag gcgatgcgtg cgggcgtgac cgcgatgggt ctgagcgttt 900

gggcggcgag cgatagcatt gcgagcccga ccaccaccgc ggtgcgtacc ccggatggtg 960

ttgatgaaaa ggcgctgcgt caggcggcgc gtgcgcgtta cggcgtggtt tttagcagcg 1020

gccgtggtga aaccctgggt aaactgaccc gtattggcca catgggtccg accgcgcaac 1080

cgatttatgc gattgcggcg ctgaccgcgc tgggtggcgc gatgaacgcg gcgggtcgta 1140

agctggcgat tggcaaaggt atcgaagcgg cgctggcggt tatcgacgcg gatgcgtga 1199

<210>  11
<211>  1485
<212>  DNA
<213>  Artificial

<220>
<223>  Artificial Fragment for Catalase

<400>  11
acaaaaagga taaaacaaat gacagatagg agaaacctaa caactaacca aggagttccc      60

ataggggata atcaaaacag catgaccgcg ggcctgaaag gcccgaccct gctggaagat     120

tacgtgctga tcgagaagct ggcgcacttc gaccgtgaac gtgttccgga gcgtgttgtt     180

catgcgcgtg gtgcgggtgc gcacggcaag ttcgttacca agaaaagcat gaagaaatat     240

accaaagcgc agtttctgca agaggaaggc accgagaccg aagtgttcgc gcgttttagc     300

accgttattc acggtcaaca cagcccggaa accctgcgtg atccgcgtgg cttcagcgtt     360

aaattttaca ccgaggaagg taactacgac ttcgtgggca acaacctgcc ggttttcttt     420

atccgtgatg cgattaagtt tccggacgtg atccacagcc tgaaaccgga tccgcgtacc     480

aacattcagg atggtaaccg ttactgggac ttctttagcc tgaccccgga ggcgaccacc     540

atgatcacct acctgttcag cgacgaaggt accccggcga gctatcgtga gatccgtggc     600

agcagcgttc acgcgtttaa gtggattaac gaggaaggta aaaccgtgta cgttaaactg     660

cgttgggtgc cgaaggcggg catcgttaac ctgagcaccg atcaagcggc gcagattcaa     720

gcgaaagagt tcaaccacgc gagccgtgac ctgtatgaag cgattgagaa cggtgattac     780

ccggagtggg acctgtatgt gcaggttctg gatccgaagg acctggataa ctacgacttt     840

aacccgctgg acgcgaccaa agattggttc gaagacgtgt ttccgtatga gcacgttggc     900

accatgaccc tgaaccgtaa cccggacaac atcttcgcgg agaccgaaag cgtgggtttt     960

aacccgggcg tgctggttcc gggtatgctg ccgagcgaag atcgtgtgct gcagggccgt    1020

ctgttcagct acagcgacac ccaacgtcac cgtgttggtc cgaactatct gcagctgccg    1080

attaacagcc cgaagacccc ggtggataac aaccagcgtg acggccaaat gccgtttaaa    1140

cagcaaacca gcagcatcaa ctacgaaccg aacagctatg ataccgaacc gaaggagaac    1200

ccggcgtaca ttgagccgga acaagagatc cgtggtgaca ttagcggccg tctggtggcg    1260

gagaaaccga acaacttcgg tcacgcgaag gaagtttgga aacgttacag cgatgcggag    1320

cgtgcggcgc tggtgaagaa catcgttgac gattgggaag gcgtgcgtga ggacatcaaa    1380

attcgtaacc tgcgtaactt ctatcaggtt gaaccggagt ttgcggagcg tgttgcggcg    1440

ggcaccggca tcaatctggc ggagcatgtt atcgacctga aataa                    1485


<210>  12
<211>  29
<212>  DNA

<213>  Artificial

<220>
<223>  Primer Sequence

<400>  12
cgcggatcca caaaaaggat aaaacaatg                                          29


<210>  13
<211>  31
<212>  DNA
<213>  Artificial

<220>
<223>  Primer Sequence

<400>  13
acgcgtcgac ttacttgaag ccggtggtaa a                                       31


<210>  14
<211>  29
<212>  DNA
<213>  Artificial

<220>
<223>  Primer Sequence

<400>  14
ccggaattca caaaaaggat aaaacaatg                                          29


<210>  15
<211>  28
<212>  DNA
<213>  Artificial

<220>
<223>  Primer Sequence

<400>  15
cgcggatcct cacgcatccg cgtcgata                                           28


<210>  16
<211>  45
<212>  DNA
<213>  Artificial

<220>
<223>  Primer Sequence

<400>  16
ggaattcaca aaaaggataa aacaatgttt gagaacatta ccgcc                        45


<210>  17
<211>  29
<212>  DNA
<213>  Artificial

<220>

<223>  Primer Sequence

<400>  17
cgcggatcct tacagcactg ccacaatcg                                          29


<210>  18
<211>  392
<212>  PRT
<213>  Mesorhizobium sp. YR577

<400>  18

Met Arg Tyr Pro Asp His Thr Glu Pro Val Ile Thr Leu Thr Ala Gly
1               5                   10                  15

Pro Val Asn Ala Tyr Pro Asp Val Leu Arg Gly Leu Gly Arg Thr Val
            20                  25                  30

Leu Tyr Asp Tyr Asp Pro Ala Phe Gln Leu Phe Tyr Glu Lys Val Ile
        35                  40                  45

Gly Lys Ala Gln Lys Ala Met Arg Leu Ser Asn Lys Pro Val Val Leu
    50                  55                  60

His Gly Glu Pro Val Met Gly Leu Glu Ala Ala Ala Ala Ser Leu Ile
65                  70                  75                  80

Ser Arg Asp Asp Val Val Leu Asn Leu Ala Ser Gly Val Tyr Gly Lys
                85                  90                  95

Gly Phe Gly Tyr Trp Ala Lys Arg Tyr Ser Pro Asn Leu Leu Glu Ile
            100                 105                 110

Glu Val Pro Tyr Asn Glu Ala Ile Asp Pro Gln Ser Val Ala Asp Met
        115                 120                 125

Leu Lys Ser His Pro Glu Ile Thr Val Val Ser Val Cys His His Asp
    130                 135                 140

Thr Pro Ser Gly Thr Ile Asn Pro Ile Asp Glu Ile Gly Ala Ile Val
145                 150                 155                 160

Ser Ala His Gly Gly Tyr Leu Ile Val Asp Ala Val Ser Ser Phe Gly
                165                 170                 175

Gly Met Lys Thr His Pro Glu Asp Cys Lys Ala Asp Ile Tyr Val Thr
            180                 185                 190

Gly Pro Asn Lys Cys Leu Gly Ala Pro Pro Gly Leu Thr Leu Met Ser
        195                 200                 205

Val Ser Asp Arg Ala Trp Thr Arg Met Lys Ala Asn Ser Ala Ala Pro
    210                 215                 220

Arg Ala Ser Val Leu Ser Ile Leu Asp Trp Glu His Ala Trp Ser Lys
225                 230                 235                 240

Glu Lys Pro Phe Pro Phe Thr Pro Ser Val Ala Glu Met Asn Gly Leu
                245                 250                 255

Asp Val Ala Leu Asp Leu Tyr Leu Asn Glu Gly Pro Glu Ala Val Trp
            260                 265                 270

Ala Arg His Ala Leu Thr Ala Lys Ala Thr Arg Ala Gly Ala Gln Ala
        275                 280                 285

Met Gly Leu Ser Ile Trp Ala Ala Asn Glu Lys Ile Ala Ser Pro Thr
    290                 295                 300

Thr Thr Ala Ile Arg Thr Pro Asp Gly Val Asp Glu Lys Ala Leu Arg
305                 310                 315                 320

Gln Ala Ala Arg Glu Arg Tyr Gly Val Val Phe Ser Ser Gly Arg Gly
                325                 330                 335

Glu Thr Leu Gly Lys Leu Thr Arg Ile Gly His Met Gly Pro Thr Ala
            340                 345                 350

Gln Pro Ile Tyr Ala Ile Ala Ala Leu Thr Ala Leu Gly Gly Ala Leu
        355                 360                 365

Asn Ala Ala Gly Arg Lys Leu Gln Val Gly Lys Gly Ile Glu Ala Ala
    370                 375                 380

Leu Ala Val Ile Asp Ala Asp Ala
385                 390

<210>  19
<211>  392
<212>  PRT
<213>  Pseudaminobacter salicylatoxidans

<400>  19

Met Arg Tyr Lys Asp Asn Ala Asp Pro Val Ile Thr Leu Thr Ser Gly
1               5                   10                  15

Pro Val Asn Ala Tyr Ala Asp Val Leu Arg Gly Leu Ser Arg Thr Val
            20                  25                  30

Leu Tyr Asp Tyr Asp Pro Ala Phe Gln Leu Phe Tyr Glu Asn Val Val
        35                  40                  45

Ala Lys Ala Gln Lys Ala Met Arg Leu Ser Thr Arg Pro Val Leu Leu
    50                  55                  60

Gln Gly Glu Pro Val Met Gly Leu Glu Ala Ala Ala Ala Ser Leu Ile
65                  70                  75                  80

Ser Ser Glu Asp Val Val Leu Asn Leu Ala Ser Gly Val Tyr Gly Lys
                85                  90                  95

Gly Phe Gly Tyr Trp Ala Lys Arg Tyr Ser Pro Asn Leu Leu Glu Ile
            100                 105                 110

Glu Thr Pro Tyr Asn Glu Ser Ile Asp Pro Gln Ala Val Ala Asp Met
        115                 120                 125

Leu Lys Lys His Pro Glu Ile Thr Val Val Ser Val Cys His His Asp
    130                 135                 140

Thr Pro Ser Gly Thr Ile Asn Pro Ile Asp Glu Ile Gly Ala Ile Val
145                 150                 155                 160

Ser Ala His Gly Ala Tyr Phe Ile Val Asp Ala Val Ser Ser Phe Gly
                165                 170                 175

Gly Met Lys Thr His Pro Glu Asp Cys Lys Ala Asp Leu Tyr Val Thr
            180                 185                 190

Gly Pro Asn Lys Cys Leu Gly Ala Pro Pro Gly Leu Thr Met Met Gly
        195                 200                 205

Val Ser Asp Arg Ala Trp Ala Arg Met Lys Ala Asn Pro Ser Ala Pro
    210                 215                 220

Arg Ala Ser Val Leu Ser Ile Leu Asp Trp Glu Asp Ala Trp Ser His
225                 230                 235                 240

Glu Lys Pro Phe Pro Phe Thr Pro Ser Val Ala Glu Ile Asn Gly Leu
                245                 250                 255

Asp Val Ala Leu Asp Leu Tyr Leu Asn Glu Gly Pro Glu Val Val Trp
            260                 265                 270

Ala Arg His Ala Leu Thr Ala Lys Ala Thr Arg Ala Gly Ala Leu Ala

275                 280                 285

Met Gly Leu Ser Leu Trp Ala Ala Ser Glu Lys Ile Ala Ser Pro Thr
    290                 295                 300

Thr Thr Ser Ile Arg Thr Pro Gln Gly Val Asp Glu Ala Ala Leu Arg
305                 310                 315                 320

Gln Ala Val Arg Glu Arg Tyr Gly Val Val Phe Ser Ser Gly Arg Gly
                325                 330                 335

Glu Thr Leu Gly Lys Leu Thr Arg Ile Gly His Met Gly Pro Thr Ala
            340                 345                 350

Gln Pro Ile Tyr Ala Val Ala Ala Leu Thr Ala Leu Gly Gly Ala Leu
        355                 360                 365

Asn Ala Met Gly Gln Lys Leu Ala Val Gly Arg Gly Ile Asp Ala Ala
    370                 375                 380

Leu Ala Val Ile Asp Ala Asp Ala
385                 390

<210>  20
<211>  392
<212>  PRT
<213>  Bauldia litoralis

<400>  20

Met Arg Tyr Ala Leu Asp Ala Asp Pro Val Ile Thr Leu Thr Ser Gly
1               5                   10                  15

Pro Val Asn Ala Tyr Pro Asp Val Leu Arg Ala Leu Ala Arg Thr Val
            20                  25                  30

Leu Tyr Asp Tyr Asp Pro Ala Phe Gln Leu His Tyr Glu Gln Val Ile
        35                  40                  45

Gly Lys Met Gln Thr Ala Met Arg Thr Ser Asn Arg Pro Val Ile Leu
    50                  55                  60

His Gly Glu Pro Val Leu Gly Leu Glu Ala Ala Ala Ala Ser Leu Ile
65                  70                  75                  80

Thr Ala Asp Asp Thr Val Leu Asn Leu Ala Ser Gly Val Tyr Gly Lys
                85                  90                  95

Gly Phe Gly Tyr Trp Ala Ala Arg Tyr Ser Pro Asn Gln Leu Glu Ile

            100                 105                 110

Glu Val Pro Tyr Asn Gln Ala Ile Asp Pro Gln Gln Val Ala Asp Met
        115                 120                 125

Leu Lys Ala Asn Pro Ala Ile Ser Val Val Ala Val Cys His His Asp
    130                 135                 140

Thr Pro Ser Gly Thr Ile Asn Pro Ile Asp Glu Ile Gly Ala Ile Val
145                 150                 155                 160

Ala Asp His Gly Ala Tyr Leu Ile Val Asp Ala Val Ser Ser Phe Gly
                165                 170                 175

Gly Met Lys Thr His Pro Glu Asp Cys Lys Ala Asp Ile Tyr Val Thr
            180                 185                 190

Gly Pro Asn Lys Cys Leu Gly Ala Pro Pro Ala Leu Thr Met Leu Gly
        195                 200                 205

Val Ser Glu Arg Ala Trp Gln Lys Met Lys Asp Asn Pro Ala Ala Pro
    210                 215                 220

Arg Ala Ser Met Leu Ser Ile Leu Asp Trp Glu Asn Ala Trp Ser Arg
225                 230                 235                 240

Asp Glu Ala Phe Pro Phe Thr Pro Ser Val Ala Glu Val Asn Gly Leu
                245                 250                 255

Asp Val Ala Leu Asp Leu Tyr Leu Ala Glu Gly Pro Glu Ala Val Trp
            260                 265                 270

Ala Arg His Ala Leu Thr Ala Ala Ala Thr Arg Ala Gly Ile Leu Ala
        275                 280                 285

Met Gly Leu Asp Leu Trp Ala Ala Asp Ala Ala Ile Ala Ser Pro Thr
    290                 295                 300

Thr Thr Ala Val Arg Thr Pro Glu Gly Ile Asp Glu Ala Lys Leu Arg
305                 310                 315                 320

Ala Thr Val Arg Glu Arg Tyr Gly Val Val Met Ser Ser Gly Arg Gly
                325                 330                 335

Glu Thr Leu Gly Lys Leu Thr Arg Ile Gly His Met Gly Pro Thr Ala
            340                 345                 350

Gln Pro Ile Tyr Ala Ile Ala Ala Leu Thr Ala Phe Gly Gly Ala Leu
        355                 360                 365

Gln Ala Leu Gly His Pro Ala Pro Val Gly Lys Ala Ile Asp Ala Ala
    370                 375                 380

Leu Ala Val Ile Asp Ala Ala Ala
385                 390

<210>  21
<211>  391
<212>  PRT
<213>  Skermanella stibiiresistens

<400>  21

Met Thr Val Glu Arg Ile Val Glu Pro Ala Phe Thr Leu Ser Ser Gly
1               5                   10                  15

Pro Val Asp Ala Tyr Pro Ala Val Leu Arg Ala Leu Ser Arg Thr Val
            20                  25                  30

Leu Tyr Asp Phe Asp Pro Val Phe Gln His Phe Tyr Glu Ala Val Thr
        35                  40                  45

Asp Lys Ala Lys Val Ala Leu Arg Thr Ala Ala Pro Pro Val Ile Leu
    50                  55                  60

His Gly Glu Pro Val Leu Gly Leu Glu Ala Ala Ala Ala Ser Leu Ile
65                  70                  75                  80

Ala Arg Asp Asp Val Val Leu Asn Leu Ala Ser Gly Val Tyr Gly Lys
                85                  90                  95

Gly Phe Gly Phe Trp Ala Lys Arg Tyr Cys Ala Glu Leu Val Glu Ile
            100                 105                 110

Glu Val Pro Tyr Asp Asp Ala Ile Asp Pro Ala Ala Val Glu Ala Ala
        115                 120                 125

Phe Arg Glu Arg Pro Asp Ile Arg Val Val Ser Val Cys His His Asp
    130                 135                 140

Thr Pro Ser Gly Thr Leu Asn Pro Ile Asp Glu Ile Gly Arg Ile Val
145                 150                 155                 160

Ala Ala His Gly Ala Tyr Leu Ile Val Asp Ala Val Ser Ser Phe Gly
                165                 170                 175

Gly Met Asp Val His Pro Glu Ala Cys Gln Ala Asp Ile Phe Val Thr
            180                 185                 190

Gly Pro Asn Lys Cys Leu Gly Cys Pro Pro Gly Leu Thr Leu Leu Ala
        195                 200                 205

Val Ser Gly Arg Ala Trp Asp Lys Met Lys Ala Asn Pro Asp Ala Pro
    210                 215                 220

Arg Ala Ser Ile Leu Ser Ile Leu Asp Trp Glu Asn Ala Trp Arg Arg
225                 230                 235                 240

Asp Gln Pro Phe Pro Phe Thr Pro Ser Ile Ser Glu Val Asn Ala Leu
                245                 250                 255

Asp Ala Ala Leu Asp Leu Tyr Leu Glu Glu Gly Pro Glu Ala Val Trp
            260                 265                 270

Arg Arg His Ala Leu Thr Ser Lys Ala Cys Arg Asp Gly Ile Arg Ala
        275                 280                 285

Met Gly Met Thr Ile Trp Pro Ala Arg Glu Ala Ile Ala Ser Pro Thr
    290                 295                 300

Thr Thr Ala Val Arg Val Pro Asp Gly Ile Asp Ala Asp Ala Leu Leu
305                 310                 315                 320

Ala Thr Ala Arg Asp Gly Phe Gly Val Ser Phe Ser Ser Gly Arg Gly
                325                 330                 335

Glu Thr Lys Gly Lys Leu Ile Arg Ile Gly His Met Gly Arg Thr Ala
            340                 345                 350

Gln Pro Leu Tyr Ala Val Val Gly Leu Ala Ala Leu Gly Gly Ala Leu
        355                 360                 365

Arg Arg Leu Gly Ala Asn Val Asp Ala Gly Ala Gly Met Glu Ala Ala
    370                 375                 380

Leu Ala Val Ile Ser Asn Gln
385                 390

<210>  22
<211>  394
<212>  PRT
<213>  Rhizobium sp. AC44/96

<400>  22

Met Asn Gln Thr Lys Ile Ala Asp Pro Ile Phe Thr Leu Thr Thr Gly
1               5                   10                  15

Pro Val Asp Ala Tyr Pro Ala Val Leu Arg Ala Leu Ser Arg Pro Val
            20                  25                  30

Leu Tyr Asp Tyr Asp Pro Ala Phe Leu Ala Phe Tyr Glu Ala Val Asn
        35                  40                  45

Ala Lys Val Gln Arg Ala Phe His Thr Lys Thr Pro Pro Val Ile Leu
    50                  55                  60

Gln Gly Glu Pro Val Leu Gly Leu Glu Ala Ala Ala Ala Ser Leu Ile
65                  70                  75                  80

Ala Lys Lys Asp Val Val Leu Asn Leu Val Ser Gly Val Tyr Gly Lys
                85                  90                  95

Gly Phe Gly Phe Trp Ala Ala Arg Tyr Ala Arg Glu Leu Val Glu Leu
            100                 105                 110

Glu Val Pro Tyr Asn Asp Val Leu Thr Ala Asp Ala Val Ala Asp Met
        115                 120                 125

Leu Lys Lys Arg Pro Asp Ile Ala Val Val Ala Leu Cys His His Asp
    130                 135                 140

Thr Pro Ser Gly Thr Val Asn Pro Val Ala Glu Ile Gly Ala Val Val
145                 150                 155                 160

Arg Ala Ala Gly Lys Leu Phe Ile Val Asp Ser Val Ser Ala Phe Gly
                165                 170                 175

Gly Met Asp Val Leu Pro Glu Thr Ala Cys Ala Asp Ile Phe Val Thr
            180                 185                 190

Gly Pro Asn Lys Cys Leu Gly Cys Pro Pro Gly Leu Ser Leu Leu His
        195                 200                 205

Val Ser Glu Ala Ala Trp Glu Lys Ile Ala Ala Asn Pro Asp Ala Pro
    210                 215                 220

Thr Ala Ser Met Leu Ser Ile Ser Asp Trp Lys Arg Ala His Glu Ala
225                 230                 235                 240

Gly Gln Pro Phe Pro Phe Thr Pro Ser Val Ser Glu Ile Asn Ala Leu
                245                 250                 255

Asp Ala Ala Met Asp Leu Tyr Leu Glu Glu Gly Glu Gln Ala Val Trp
        260                 265                 270

Ala Arg His Ala Leu Thr Ala Lys Ala Cys Arg Ala Gly Val Gln Ala
        275                 280                 285

Ala Gly Leu Lys Leu Trp Ala Ala Arg Glu Asp Ile Ala Ser Pro Thr
    290                 295                 300

Cys Thr Ala Val Ala Ile Pro Asp Gly Ile Asp Glu Ala Lys Leu Arg
305                 310                 315                 320

Ala Ser Ile Arg Asp Arg Tyr Gly Val Val Phe Ser Ser Gly Arg Ala
                325                 330                 335

Glu Thr Leu Gly Lys Leu Thr Arg Ile Gly His Met Gly Pro Thr Ala
            340                 345                 350

Arg Pro Thr Tyr Ser Leu Val Ser Val Ala Ala Ile Val Gly Gly Leu
        355                 360                 365

Lys Ala Ala Gly Val Lys Asp Leu Asp Val Glu Ala Gly Val Ala Ala
    370                 375                 380

Ala Met Lys Val Ile Asp Ser Ala Glu Lys
385                 390

<210>  23
<211>  390
<212>  PRT
<213>  Erwinia toletana

<400>  23

Met Met Arg Ser Ala Phe Ala Gln Pro Leu Phe Thr Leu Thr Thr Gly
1               5                   10                  15

Pro Val Asp Val Tyr Pro Ala Val Ser Arg Ala Leu Ser Met Pro Val
            20                  25                  30

Trp Tyr Asp Tyr Asp Pro Ala Phe Gln Asn Cys Phe Glu Arg Val Ser
        35                  40                  45

Leu Lys Ala Ala Gln Ala Leu Glu Ser Ala Thr Pro Pro Leu Ile Leu
    50                  55                  60

Gln Gly Glu Pro Val Leu Ala Leu Glu Ala Ala Ala Ala Ser Leu Leu
65                  70                  75                  80

Ser Ala Glu Asp Met Val Leu Asn Leu Val Ser Gly Val Tyr Ala Ala
                85                  90                  95

Gly Phe Ser Glu Trp Ala Arg Arg Tyr Ser Arg Gln Val Glu Glu Leu
            100                 105                 110

Arg Val Glu Phe Asn Gln Val Ile Asp Pro Val Ala Val Asp Ala Trp
        115                 120                 125

Leu Ser Arg His Pro Gln Ile Thr Val Val Val Val Cys His His Asp
    130                 135                 140

Thr Pro Ser Gly Thr Leu Asn Pro Leu Asp Glu Ile Gly Arg Val Val
145                 150                 155                 160

Lys Lys His Gly Lys Leu Leu Leu Val Asp Ala Val Ser Ser Phe Ala
                165                 170                 175

Gly Val Ala Val Thr Ala Ala Ser Cys Gln Ala Asp Leu Phe Ile Thr
            180                 185                 190

Gly Pro Asn Lys Cys Leu Gly Cys Pro Pro Gly Leu Ser Leu Val Ala
        195                 200                 205

Val Ser Asp Ala Ala Trp Arg Lys Ile Glu Ala Asn Asp Ala Ala Pro
    210                 215                 220

Arg Ala Ser Val Leu Ser Leu Leu Asp Trp Arg Asp Ala Trp Ser Ala
225                 230                 235                 240

Glu Lys Pro Phe Pro Tyr Thr Pro Ser Val Ala Glu Ile Asn Gly Leu
                245                 250                 255

Glu Ala Ala Leu Asp Gly Tyr Leu Gln Glu Gly Pro Ala Gln Val Trp
            260                 265                 270

Ala Arg His Arg Leu Thr Ala Gln Ala Phe Arg Ala Gly Ile Gln Ala
        275                 280                 285

Met Gly Leu Ser Leu Trp Ala Ala Asp Glu Ser Ile Ala Ser Pro Thr
    290                 295                 300

Ala Thr Ala Ile Arg Val Pro Asp Gly Val Asn Glu Ala Gln Trp Arg
305                 310                 315                 320

Leu Arg Ala Arg Glu Cys Tyr Gly Val Met Phe Ser Ser Gly Arg Gly
                325                 330                 335

Glu Thr Leu Gly Lys Val Leu Arg Val Gly His Met Gly Pro Thr Ala
            340                 345                 350

Gln Pro Met Phe Ala Ile Ile Ala Leu Thr Ala Leu Ala Gly Ala Leu
        355                 360                 365

Asn Gln Leu Arg Glu Pro Gln Leu Asp Ile Gly Ala Gly Val Ala Ala
    370                 375                 380

Ala Met Ala Val Ile Gly
385                 390

<210>  24
<211>  386
<212>  PRT
<213>  Herbiconiux ginsengi

<400>  24

Met Ala His Pro Asp Phe Thr Leu Ser Ala Gly Pro Val Thr Val Thr
1               5                   10                  15

Ala Arg Thr Leu Ala Gly Leu Gly Ser Pro Ile Leu Tyr His Tyr Asp
            20                  25                  30

Pro Glu Phe Leu Ala Thr Phe Arg Arg Thr Gln Gly Lys Val Gly Gln
        35                  40                  45

Met Phe Gln Thr Asp Asn Asp Ile Ile Leu Met Gln Gly Glu Ala Ile
    50                  55                  60

Val Gly Leu Glu Gly Ala Leu Arg Ser Leu Ile Thr Pro Gly Met His
65                  70                  75                  80

Val Leu Asn Leu Val Gln Gly Val Phe Gly Lys Gly Thr Gly Tyr Trp
                85                  90                  95

Ile Ala Asp Phe Gly Ala Val Leu His Glu Ile Glu Val Gly Tyr Asp
            100                 105                 110

Asp Ala Val Ser Pro Ala Gln Val Glu Glu Tyr Leu Asp Ala His Pro
        115                 120                 125

Glu Ile Gln Met Val Cys Leu Val Ala Ser Glu Thr Pro Ser Gly Thr
    130                 135                 140

Val Thr Asp Val Ala Ala Ile Gly Pro Met Cys Arg Asp Arg Gly Ile
145                 150                 155                 160

Leu Thr Tyr Ile Asp Thr Val Ser Gly Val Leu Gly Met Pro Trp Lys
                165                 170                 175

Thr Asp Glu Trp Gly Leu Asp Leu Cys Val Ala Gly Ala Gln Lys Cys
            180                 185                 190

Leu Gly Gly Pro Pro Gly Val Ala Leu Ile Ser Val Ser Gln Lys Ala
        195                 200                 205

Trp Asp Val Met Tyr Ala Asn Pro Ala Ala Pro Arg Asp Ser Tyr Leu
    210                 215                 220

Ser Leu Ile Asp Trp Lys Glu Lys Trp Leu Gly Glu Gly Arg Phe Pro
225                 230                 235                 240

Tyr Thr Pro Ser Val Ser Asp Met Asn Gly Leu Glu Ala Ala Leu Asp
                245                 250                 255

Gln Ala Leu Glu Glu Gly Ile Asp Ala Val Val Ala Arg His Glu Ala
            260                 265                 270

Ala Ala Ala Val Thr Arg Ala Gly Ala Arg Ala Met Gly Leu Glu Leu
        275                 280                 285

Trp Ala Lys Ser Glu Glu Ile Ala Ser Ala Cys Val Thr Ser Ile Arg
    290                 295                 300

Leu Pro Asp Asp Ile Asp Asn Ala Val Val Arg Asp His Ala Arg Glu
305                 310                 315                 320

Val Tyr Gly Val Met Leu Ser His Gly Gln Gly Ala Gly Asn Leu Val
                325                 330                 335

Arg Leu Ser His Met Gly Pro Thr Ala Gly Gly Leu His Ser Val Val
            340                 345                 350

Gly Leu Ala Ala Leu Gly Arg Thr Leu Ala Asp Leu Gly Met Ser Val
        355                 360                 365

Asp Ile Gly Ala Gly Leu Glu Ala Ala Leu Ala Leu Leu Ser Thr Gln
    370                 375                 380

Arg Arg
385

<210>  25

<211>  1179
<212>  DNA
<213>  Mesorhizobium sp. YR577

<400>  25
atgcgatatc ccgaccacac tgagccggtg atcacactca ccgccggtcc cgtcaacgcc      60

tatcccgatg tgctacgggg gctcgggcgc acggtgcttt acgactacga cccggcgttc     120

cagctcttct acgagaaggt catcggcaag gcgcagaagg ccatgcgtct gtcgaataag     180

ccggtcgtcc tgcatggcga gccggtgatg ggtctggagg ccgccgcggc gtcgctcatc     240

tctagggatg atgtggtgct caatctggca tcgggtgtct acggcaaggg gttcggctat     300

tgggcgaagc gttattcgcc gaacctgctg gagatcgagg ttccttacaa tgaggcgatc     360

gatccgcagt ccgtcgcgga catgctgaaa tcgcatcccg aaatcaccgt cgtttcggtc     420

tgccatcacg acacgccgtc aggcacgatc aacccgatcg atgagatcgg agcgatcgtt     480

tcggcgcatg gcggctatct gatcgtcgac gccgtttcgt ccttcggcgg catgaaaacg     540

catcccgagg attgcaaggc cgacatctat gtgaccgggc cgaacaagtg ccttggcgca     600

cctcccggac tgacgctgat gagcgtgagc gaccgtgcct ggaccaggat gaaagcgaac     660

tctgcagcac ctcgcgcatc ggttctgagc attctcgatt gggagcatgc atggtccaag     720

gaaaagccgt ttccgtttac gccgtccgtc gccgaaatga acggcctgga tgtcgcgctc     780

gacctttatc tcaacgaggg gccggaagcg gtgtgggcgc ggcacgccct gaccgccaag     840

gcgacacgcg ccggggctca ggcgatgggc ctgtcgatct gggctgcaaa tgaaaagatc     900

gcctcaccga caacaaccgc catccgcacg ccggacgggg tcgatgaaaa ggcgctgcgt     960

caggccgcgc gtgagcgtta cggcgtggtg ttttcttccg gccggggcga aacgctcggc    1020

aaactgacgc gcattggcca catggggcca acggcccagc cgatctacgc gatcgcagcg    1080

ctgacggctt tgggcggtgc gctgaatgcg gccggccgga agcttcaggt tggcaagggt    1140

atcgaggcag cactcgccgt gatcgatgcg gatgcctga                           1179

<210>  26
<211>  1179
<212>  DNA
<213>  Pseudaminobacter salicylatoxidans

<400>  26
atgcgatata aagacaatgc cgacccggtg atcacgctca cctcggggcc cgtcaacgcc      60

tatgcggatg tgctgcgcgg gctgagccgc actgtgctct acgactatga tccggcgttc     120

cagctcttct atgaaaacgt ggtggccaag gcgcagaagg ccatgcgtct ttccacccgg     180

ccggtgctgt tgcagggtga gccggttatg ggactggagg cagcggcagc ttcactgatt     240

tccagcgagg atgtcgtcct gaacctcgcc tctggcgtct atggcaaggg cttcggctac     300

tgggcaaagc gctattcgcc gaacctgctc gagatcgaaa ccccctataa cgaatccatc     360

gacccgcagg cggttgccga tatgcttaag aagcatccgg aaatcaccgt tgtctcggtg 420

tgccaccacg acacaccgtc gggcacgatc aacccgatcg acgaaattgg cgcgatcgta 480

tcggcgcacg gggcctattt catcgtcgat gccgtatctt cattcggagg catgaagacc 540

catcccgaag actgcaaggc cgacctctat gtcactggcc cgaacaagtg cctgggcgcg 600

cctccgggac tgaccatgat gggcgtcagc gacagggcct gggccaggat gaaagccaat 660

ccatccgcac cgcgcgcctc ggtgctgagc attctcgact gggaggatgc ctggtcgcac 720

gaaaagccgt ttccgttcac accctctgtg gcggagatca acggcctcga cgtcgcgctg 780

gacctctatc tgaacgaggg gccggaagtg gtgtgggcgc gccatgcgct gacggcaaaa 840

gccacgcgtg cgggcgcatt ggccatgggg ttgtcgctct gggcggctag cgagaagatt 900

gcctcgccca ccacgacgtc gatccgcacg ccgcaggggg tggacgaagc cgcgcttcgg 960

caggcggtgc gagagcgcta tggcgtagtg ttttcatccg gccgtggcga gacgttgggc 1020

aagcttaccc gcatcggcca catgggcccc acggcgcagc cgatttacgc agtcgcggcg 1080

ctgaccgcgc tgggtggcgc gttgaacgcg atgggccaga agctggccgt cggcagaggc 1140

atcgacgccg ccctcgcggt gatcgacgcc gatgcatga 1179

<210> 27
<211> 1179
<212> DNA
<213> Bauldia litoralis

<400> 27
atgcgctatg cactcgatgc cgatcccgtc atcacgctga cctcggggcc ggtcaacgcc 60

tatccggatg tcctgcgggc gttggcgcgc acggttcttt acgactacga cccggccttc 120

cagctccatt acgagcaggt gatcggcaag atgcagacgg cgatgcggac gtcgaaccgt 180

ccggtcatcc tgcatggcga gccggtgctc ggactcgagg cggcggcggc ctcgctgatc 240

accgccgacg atactgtgct caacctggcg tcgggcgtct acggcaaggg gttcggctac 300

tgggcagcgc gctattcacc caaccagctc gagatcgagg ttccctacaa ccaggcgatc 360

gacccgcagc aggtcgccga catgctgaag gccaatccgg ccatttcggt tgtcgccgtc 420

tgccatcacg acacgccgtc ggggaccatc aacccgatcg acgagatagg cgccatcgtc 480

gccgaccatg gcgcctacct gatcgtcgac gcggtgtcct ccttcggcgg gatgaagacc 540

cacccggagg attgcaaggc cgacatctac gtcaccggcc cgaacaaatg cctcggcgcg 600

ccgccggccc tgaccatgct cggcgtcagc gaacgggcgt ggcagaagat gaaggacaat 660

ccggccgcgc cgcgcgcctc gatgctcagc atcctcgact gggagaatgc ctggtcgcgc 720

gacgaggcct ttcccttcac gccgtcggtg gccgaggtca acggtctcga cgtcgcgctc 780

gacctctacc ttgccgaggg accggaggcc gtctgggcgc gccacgcgct gaccgccgcg 840

gcgacgcggg ccggcatcct ggcgatgggc ctcgacctgt gggcggcgga cgcggccatc      900

gcctcgccga cgacgaccgc ggtgcggacg ccggagggca tcgatgaagc gaagctgcgc      960

gcgacggtcc gcgaacgcta tggcgtggtg atgtcgtccg ggcgcggcga aacgctcggc     1020

aagctcacgc ggatcggcca catgggcccg acggcgcagc cgatctatgc gatcgccgcg     1080

ctgaccgcct ttggcggcgc tctccaggcg ctcggccatc ccgcgccggt cggcaaggcg     1140

atcgacgcgg cgctcgcggt catcgacgcc gccgcctga                            1179

<210>  28
<211>  1176
<212>  DNA
<213>  Skermanella stibiiresistens

<400>  28
atgacagtcg agcgtatcgt cgagcctgct ttcactctca gttccggtcc cgtcgacgcg       60

tatcccgccg tgctcagggc actgtcgcgg acggtgctct acgacttcga tcccgtgttc      120

cagcacttct acgaggcggt gacggataag gccaaggtgg cactccgcac cgcggcgcca      180

cccgtgatcc tccatggcga gccggtgctg gggctggagg ccgcggccgc ctcgctgatc      240

gcccgcgacg atgtcgtgct caacctagcg tccggcgtct atggcaaggg cttcggtttc      300

tgggccaaac ggtactgcgc cgagttggtc gagatcgagg tgccttatga cgacgcgatc      360

gacccggcgg cggtcgaggc cgccttcagg gaacgccccg acatccgtgt ggtctcggtc      420

tgccatcatg acacgccgtc tggcacgctg aacccaatcg acgagatcgg ccgtatcgtg      480

gcggcccatg gtgcctatct gatcgtggac gccgtttcct cgttcggggg gatggacgtc      540

catcccgagg cctgccaagc cgacatcttc gtgacagggc cgaacaagtg cctgggctgc      600

ccgcctggtc tgacgctgct ggcggtcagt ggccgcgcct gggacaagat gaaggccaat      660

ccggacgcgc cgcgcgcctc gatcctcagc atcctggatt gggagaacgc gtggcggcgt      720

gaccagccgt ttcccttcac cccatcgatt tccgaagtga acgcgctgga cgcggctctg      780

gacctgtacc tggaggaggg gccggaagcc gtctggcgcc gtcacgcgct cacatccaag      840

gcctgccgcg acggcatccg cgccatgggc atgacgatat ggcccgcgcg ggaggcgatc      900

gcctctccga ccaccacggc ggtgcgggtg cccgacggga tcgacgcgga tgccctgttg      960

gcgacggcgc gggatggctt cggcgtcagc ttctcgtccg ggcggggcga gaccaagggc     1020

aagctgatcc gcatcggaca catgggccgc accgcccaac cactctacgc cgtcgtcggg     1080

ctggcggcgc tgggcggtgc gttgcggcgg ctgggagcca atgtcgacgc cggcgccggc     1140

atggaggcgg ctctcgccgt gatctcaaac caataa                               1176

<210>  29
<211>  1185

<212>  DNA
<213>  Rhizobium sp. AC44/96

<400>  29
atgaaccaga cgaaaattgc cgatccaatc tttaccttga ccacggggcc ggtcgatgcc      60

tatccagcgg tgctgcgcgc cctgtcccgc ccggtgctct acgactacga tccggcgttc     120

cttgcgttct acgaagccgt caatgccaag gtacagcgcg ccttccacac gaaaacccca     180

cccgtgatcc tccagggcga gcccgtgctg gggctcgaag cggctgcggc ttcgctgatc     240

gccaagaaag acgtggtgct taacctcgtt tccggcgtct acggcaaggg gttcggcttt     300

tgggcagcgc gctatgcgcg ggagctggtc gaattggaag ttccctataa cgatgtcctg     360

acggccgatg cggtggccga catgttgaaa aagcgtccgg atatcgccgt ggtcgcgctc     420

tgccaccatg ataccccgtc aggcaccgtc aatcccgtgg ccgaaatcgg tgcagtggtg     480

agagcggcgg gaaagctctt catcgtcgat tccgtatcgg cctttggcgg catggacgtg     540

cttccggaga cggcttgcgc cgatattttc gtaaccggcc ccaataaatg tctcggctgc     600

ccgcccggtt tgtcgcttct gcacgtcagc gaggctgcct gggagaagat cgccgccaat     660

cccgatgccc cgacagcttc gatgctttcg atcagtgatt ggaaacgagc tcacgaggct     720

ggccaaccgt tcccgtttac cccctcggtt tccgaaatca acgccctcga cgccgccatg     780

gatctctatc tggaggaagg cgagcaggca gtatgggcgc gccatgcgct gacagccaag     840

gcatgccggg caggcgtgca agcagccggc ctgaagctct gggcggcacg ggaagacatc     900

gcctcgccaa cctgcactgc cgtggcaatc cccgatggga tcgacgaagc aaagctgcgg     960

gcgtcgatcc gtgatcgcta cggcgttgtc ttttcttctg gccgtgccga gacgctgggc    1020

aagcttacgc gcatcggcca tatgggcccg actgcacgtc cgacctattc actcgtctcg    1080

gttgcagcca tcgttggcgg gctcaaggcc gccggcgtca aggaccttga tgtggaggct    1140

ggagttgcgg cggcaatgaa ggtcatagac agtgcagaaa agtga                    1185


<210>  30
<211>  1173
<212>  DNA
<213>  Erwinia toletana

<400>  30
atgatgcgtt ccgcctttgc ccagccgtta tttaccctga ctaccggtcc ggttgatgtc      60

tatccggcgg tgtcgcgggc attatcgatg ccggtctggt acgactacga cccggctttt     120

cagaactgct ttgaacgcgt cagcctgaag gccgcacagg cgctggaaag cgccacgccg     180

ccgctgatct tacagggtga accggtactg gcgctggaag ccgcggcggc atcgctgctt     240

agcgctgagg atatggtgct gaatctggtt tccggcgtat atgccgccgg tttctctgag     300

tgggcgcggc gctattcgcg tcaggttgag gagctgcgcg ttgaatttaa tcaggtgatt     360

gatcctgtcg cggttgatgc atggcttagt cgtcatccgc agattaccgt ggtggtggtt 420

tgccaccatg acacgccgtc cggcacccta aacccactgg atgaaattgg gcgggtggtg 480

aaaaaacatg gcaaattgct gctggtggat gcggtttctt cgtttgccgg tgtggcggtg 540

acggcggcca gttgccaggc ggacctgttt attaccggcc ccaataaatg tctcggctgt 600

ccgccgggct taagcctggt ggcggtatct gacgctgcct ggcggaaaat cgaggccaat 660

gacgctgcgc cgcgtgcgtc ggtactcagc ctgctcgact ggcgtgatgc ctggtcagcg 720

gaaaaacctt ttccgtatac cccatcagtg gcggagatca acgggctgga ggcggcgctg 780

gacggttatt tgcaggaggg gccagcgcag gtctgggcgc gccacaggct aaccgcacag 840

gcatttcgcg ccgggattca ggcgatggga cttagcctgt gggcggcgga tgaaagcatt 900

gcttcaccaa cggcaactgc gatacgcgtg cccgacgggg taaatgaagc gcaatggcga 960

ctgcgcgcgc gtgagtgtta tggcgtgatg ttttcgtcag ggcgcggtga aacgctgggc 1020

aaggtattgc gtgtcggcca tatggggccg actgcgcaac cgatgtttgc gattattgcg 1080

ctgaccgcac tggcgggggc acttaatcag ctacgcgagc cgcagctgga tattggcgct 1140

ggcgtggcgg cggcaatggc ggtgattggc taa 1173

<210> 31
<211> 1161
<212> DNA
<213> Herbiconiux ginsengi

<400> 31
atggcacacc ccgacttcac gctctcggcc gggccggtca ccgtcaccgc ccgcacgctc 60

gcgggcctcg gctcgccgat cctctaccac tacgaccccg aattcctcgc gacgttccgg 120

cgcacgcagg gcaaggtggg gcagatgttc cagaccgaca acgacatcat cctcatgcag 180

ggtgaggcca tcgtcggtct cgagggcgcc ttgcgctcgc tgatcacgcc cgggatgcac 240

gtgctgaacc tcgtgcaggg cgtgttcggc aagggcaccg gctactggat cgccgacttc 300

ggcgccgtgc tgcacgagat cgaggtcggc tacgacgacg cggtctcgcc ggcacaggtc 360

gaggagtacc tcgacgcgca tccggagatc cagatggtgt gcctggtggc ctccgagacc 420

ccgtcgggca cggtgacgga tgtcgcggcg atcggcccga tgtgccggga ccgcggcatc 480

ctgacctaca tcgacactgt ctcgggcgtg ctcggcatgc cctggaagac cgacgagtgg 540

ggcctggacc tctgcgtcgc cggcgctcag aaatgcctcg ggggccctcc tggcgtggcg 600

ctcatctcgg tgagccagaa ggcgtgggac gtgatgtacg cgaacccggc cgccccgcgt 660

gactcctatc tctcgctgat cgactggaag gagaagtggc tcggtgaggg ccgctttccg 720

tacacgccgt cggtgagcga catgaacggg ctcgaagcgg cgctcgacca ggcgctggag 780

gaggggatcg acgcggtcgt cgcgcgtcac gaggccgccg ccgccgtgac ccgcgccggc 840

gcccgagcca tgggtctcga gctctgggcg aagagcgagg agatcgcgtc ggcctgcgtc 900

acctccatcc gcctgcccga cgacatcgac aacgccgtgg tgcgcgacca cgcccgcgag 960

gtctacgggg tgatgctctc gcacgggcag ggtgccggca acctcgtgcg gctctcgcac 1020

atgggcccga cagccggtgg cctccactcg gtggtgggcc tcgccgcgct cggccgcacc 1080

ctcgccgatc tgggcatgtc cgtcgacatc ggagcgggac tggaggcggc gctcgcgctg 1140

ctcagtaccc agcggcggtg a 1161

<210> 32
<211> 1196
<212> DNA
<213> Artificial Sequence

<220>
<223> Artificial Fragment for Pyridoxamine Pyruvate Transaminase

<400> 32
acaaaaagga taaaacaatg cgttatcctg atcacactga accggtgatt acattaaccg 60

caggtccagt taatgcctat cccgatgtgt tacgcggttt gggtcgcacc gtgctttatg 120

attacgaccc ggcttttcag ttattttatg agaaagtcat tggtaaagcg cagaaggcca 180

tgcgtctgag caataaaccg gttgtcctgc atggcgaacc ggtgatgggt ctggaggccg 240

cagctgcgtc attgatctct agagatgatg tggtacttaa tctggcaagc ggtgtttacg 300

gtaaagggtt cggctattgg gctaagcgtt attcgcctaa cctgttagaa attgaggttc 360

cttacaatga agcgatcgat ccgcagtccg tcgctgacat gctgaaaagt catcccgaaa 420

ttaccgtagt tagtgtctgt catcacgaca ctccatcagg tacgatcaat ccgattgatg 480

agatcggagc gattgtttcg gctcatggcg gatatctgat cgtagatgcc gttagttcat 540

tcggtggcat gaaaacacat cccgaagatt gcaaagccga catatatgtg accgggccaa 600

acaaatgtct tggcgcacct cccggactga cgctgatgag cgtgagcgac cgtgcctgga 660

ccagaatgaa agcgaactct gcagcacctc gcgcaagtgt tctgagcatt ttggattggg 720

agcatgcatg gtccaaggaa aaaccgtttc catttactcc gtctgtcgcc gaaatgaacg 780

gactggatgt tgctttagac ctttatttga acgaagggcc tgaagcggtg tgggctcggc 840

acgcactgac cgccaaagcg acacgcgccg gggctcaagc aatgggctta tcgatctggg 900

ctgcaaatga aaagattgcc tcaccgacaa caaccgcaat acgcacgcca gacggggtcg 960

atgaaaaagc gctgcgtcag gccgctcgtg agcgttacgg agtggtattt tcttccggcc 1020

ggggagaaac tttaggcaaa ctgacgcgca ttggtcacat ggggccaact gcgcagccga 1080

tctacgcgat agcagcgctg acggctttgg gcggtgcgct gaatgccgct ggacggaaac 1140

ttcaagttgg caagggtatt gaggcagcat tagccgtaat agatgcggat gcctaa 1196

<210> 33
<211> 1196
<212> DNA
<213> Artificial Sequence

<220>
<223> Artificial Fragment for Pyridoxamine Pyruvate Transaminase

<400> 33
acaaaaagga taaaacaatg cgttataaag ataatgccga tccggtgatt acactgacca 60

gtggtcctgt taatgcatat gcggatgtgc tgcgtggtct gagccgcact gttttatacg 120

actatgatcc agcatttcag ttattctatg aaaatgtggt ggccaaagca cagaaagcta 180

tgcgtctttc tacccggccg gtactgttgc agggtgaacc tgttatggga ctggaagcag 240

cggcagcttc actgatttcc agcgaagatg tcgttctgaa cttagcctct ggtgtctatg 300

gtaagggctt tggttactgg gcaaaacgct atagtccgaa tctgttagag attgaaaccc 360

cctataacga atctatcgac ccacaagctg ttgccgatat gcttaagaaa catccggaaa 420

ttaccgttgt ctcggtgtgt catcacgata cacctagtgg cacgattaat ccgatcgacg 480

aaattggtgc gatagtatca gcacacgggg cctattttat cgttgatgct gtatcttcat 540

tcggaggcat gaaaacccat cccgaagatt gtaaggccga cctttacgtt actggtccaa 600

acaaatgcct gggcgcgcct ccgggactga caatgatggg ggtcagcgat cgggcctggg 660

ccagaatgaa agctaatcca tccgcacctc gtgcctcggt gctgagcatt ttagactggg 720

aggatgcttg gagtcatgaa aaaccgtttc cattcacacc ttctgtagcg gagataaatg 780

gcttagatgt cgcgctggac ctttacctga acgaggggcc ggaagtggtg tgggcacgcc 840

atgcgctgac tgcaaaagcc acgcgtgcag gtgcattggc tatggggttg tcattatggg 900

cggctagcga gaagattgcc agtcccacca caacgtcgat acgcactcca cagggggttg 960

atgaagctgc acttcggcaa gcggtgcgtg agcgctatgg cgtagtgttt tcatccggtc 1020

gtggcgagac gttgggtaaa cttacccgca tcggacacat gggccccacg gcgcagccga 1080

tttacgcagt tgcggctctg actgcgctgg gcggggcttt gaacgcgatg ggacaaaagc 1140

tggccgtcgg cagaggaata gacgccgctt tagcggtaat cgacgccgat gcataa 1196

<210> 34
<211> 1196
<212> DNA
<213> Artificial Sequence

<220>
<223> Artificial Fragment for Pyridoxamine Pyruvate Transaminase

<400> 34
acaaaaagga taaaacaatg cgttatgcac tggatgccga tcctgttatt acactgacca 60

gcggtccggt taatgcatat ccggatgtgc tgcgtgcttt ggcgcgcaca gttctttatg 120

attacgaccc tgcttttcag ttacattatg aacaggtgat cggtaaaatg caaacggcaa 180

tgcggacaag caaccgtccg gtcattctgc atggcgaacc agtgctggga ttagaggcgg 240

ctgcggccag tctgattacc gcagacgata ctgtgttaaa tctggcatct ggtgtttacg 300

gcaaagggtt tggttattgg gcagcgcgtt attcacccaa ccagctggaa atcgaggttc 360

cctacaacca ggctattgat ccgcagcaag tcgccgatat gctgaaggct aatcctgcca 420

tttctgttgt tgcagtatgt catcacgaca ctccgtcagg gaccattaat ccaatcgatg 480

aaataggtgc cattgtcgcc gaccatggag cctatctgat tgttgatgcg gtgagttcct 540

tcggcgggat gaaaacccac ccggaggatt gcaaagcaga catctacgta acaggtccta 600

acaaatgttt aggcgcaccg ccagccctga ccatgttggg tgtcagcgaa agagcgtggc 660

agaagatgaa agataatccg gcagctcctc gcgcctcgat gttaagcatt cttgactggg 720

aaaatgcctg gtctcgtgat gaggcctttc ccttcacgcc gtcagtggct gaagttaacg 780

ggttagacgt agcgctggat ttataccttg ccgagggacc agaagcagtc tgggcacgcc 840

atgcgctgac cgccgctgcg actcgggctg gcatactggc aatgggtttg gacctgtggg 900

cggctgatgc ggccatcgca agtccgacaa cgaccgcagt gagaactcct gagggcattg 960

atgaagcgaa actgcgtgct acagtacgcg aacgttatgg agtggtgatg tcgtccgggc 1020

gcggcgaaac gttgggtaaa cttactcgga taggccacat gggaccaacg gcgcagccta 1080

tctatgcaat tgccgcgctg accgcttttg gtggcgcttt acaagcgttg ggtcatcccg 1140

ctccagtagg aaaggcaatc gacgcggctc ttgcggtcat agatgccgca gcctaa 1196

<210> 35
<211> 1193
<212> DNA
<213> Artificial Sequence

<220>
<223> Artificial Fragment for Pyridoxamine Pyruvate Transaminase

<400> 35
acaaaaagga taaaacaatg acagttgaac gtattgttga acctgctttt actttaagta 60

gcggtcctgt cgatgcgtat ccagccgtgt tacgcgcact gtcacgtacg gttttatacg 120

attttgatcc tgtgttccag cacttttatg aagcagttac tgataaagct aaagtggcac 180

ttcgtaccgc ggcaccacct gtaatcttgc atggcgaacc ggtgctgggt ttagaggccg 240

cggctgccag cctgattgca cgcgacgatg ttgtacttaa tttagcgtct ggtgtctatg 300

gcaagggttt cggtttttgg gccaaacgtt actgtgctga attggttgag attgaagtgc 360

cttatgatga cgcgatcgat ccggcggcgg tcgaggccgc attcagagaa cgccccgaca 420

ttcgtgtagt tagtgtctgc catcatgata cgccgtctgg cactctgaac ccaattgatg 480

aaatcggacg tatagtggca gcccatggtg cttatctgat tgtagacgcc gtttcttcgt 540

ttggtgggat ggatgttcat ccagaggctt gtcaagccga catctttgtg acagggccga 600

ataaatgcct gggctgtccg cctggtttga cgctgctggc ggtcagtggc cgcgcttggg 660

ataaaatgaa ggccaatccg gacgcgccga gagcatcaat tcttagcata ctggattggg 720

aaaacgcgtg gcggcgtgat cagccgtttc ccttcacccc aagtatttcc gaagtgaatg 780

cactggatgc ggctttagac ctgtatctgg aagaggggcc ggaagccgta tggcgccgtc 840

acgcacttac atcaaaagcc tgccgcgatg gcatccgcgc tatgggaatg actatatggc 900

ccgcgcgtga ggcaattgct tctccgacca ccacggcggt gcgggtaccc gacgggattg 960

atgcagatgc tctgttggcg acagcacgtg atggctttgg tgttagcttc tcgtccgggc 1020

ggggcgaaac caaaggaaag ctgatccgca taggacacat gggtcgcacc gcccagccat 1080

tatacgccgt tgtcgggtta gcagcgctgg gcggtgcgtt gagacggctg ggagctaatg 1140

tggacgccgg cgcaggaatg gaggcagctc ttgccgtcat ctcaaaccaa taa 1193

<210> 36
<211> 1202
<212> DNA
<213> Artificial Sequence

<220>
<223> Artificial Fragment for Pyridoxamine Pyruvate Transaminase

<400> 36
acaaaaagga taaaacaatg aatcagacga aaattgcaga tccaattttt accttgacca 60

caggtccggt tgatgcctat ccagcggtgc tgcgtgcact gtctcgcccg gtgttatatg 120

actacgatcc ggcgtttctt gcgttctatg aagccgtcaa tgctaaagta cagcgtgcct 180

ttcatacgaa aaccccacct gtgatcctgc agggtgaacc tgtgctgggg ttagaagcgg 240

ctgcggctag cctgattgcc aagaaagatg tggtgcttaa cctggtttct ggcgtttacg 300

gtaaagggtt cggcttttgg gcagcgcgct atgcgcgtga gctggtcgaa ttggaagttc 360

cctataacga tgtcctgaca gccgatgcgg tagctgacat gttgaaaaag cgtccagata 420

tcgccgtggt tgcgttatgt caccatgata ccccgtcagg taccgtcaat cctgtagccg 480

aaataggtgc agtggtgaga gcggcgggaa aactgtttat tgttgattcc gtaagtgcct 540

ttggtggcat ggacgtgtta ccggaaacgg cttgcgctga tattttcgta actggtccca 600

ataaatgttt aggctgcccg cctggtttgt ctcttctgca cgtcagcgaa gctgcctggg 660

agaagattgc agccaatccc gatgcaccaa cagcttcaat gcttagcatc agtgattgga 720

aaagagctca cgaggctggt caaccgttcc cgtttactcc ttcggtttcc gaaataaacg 780

ccctggacgc tgccatggat ttatatctgg aggaaggcga gcaggcagta tgggcgcgcc 840

atgcgctgac agccaaagca tgtcgggcag gagtgcaagc agccggcctg aagttatggg 900

cggcacggga agacatcgct tcaccaacct gcactgccgt ggcaattccc gatgggattg 960

acgaagcaaa actgcgggcg agtatacgtg atcgctacgg agttgtcttt tctagcggcc 1020

gtgctgagac gctgggtaag ttgacacgca tcgggcatat gggcccgact gcacgtccta 1080

cctattcatt agtctcggtt gcagccattg ttggagggct gaaagcagcc ggcgttaaag 1140

accttgatgt agaggctgga gttgcggcgg caatgaaggt catagacagt gcagaaaaat 1200

aa 1202

<210> 37
<211> 1190
<212> DNA
<213> Artificial Sequence

<220>
<223> Artificial Fragment for Pyridoxamine Pyruvate Transaminase

<400> 37
acaaaaagga taaaacaatg atgcgttccg cctttgccca gccgttattt accctgacta 60

caggtcctgt tgatgtctat ccggcggtga gtcgtgcatt atctatgcct gtctggtatg 120

attacgaccc ggcttttcag aactgctttg aacgcgtcag cctgaaggcc gcacaggctc 180

tggaaagtgc cacgccgcct ctgatcttac agggtgaacc cgtactggca ctggaagccg 240

cagcggcatc actgcttagc gctgaagata tggtgctgaa tctggtttcc ggcgtatatg 300

ccgccggttt ctctgagtgg gcgcggcgtt actctcgtca agttgaagag ctgcgcgttg 360

aatttaatca ggtgattgat cctgtcgctg ttgatgcatg gcttagtcgt catccgcaga 420

ttaccgtggt tgtggtttgc caccatgata cgccttccgg caccttaaac ccactggatg 480

aaattgggcg ggttgtgaaa aaacatggta aattgctgct tgtggatgcg gtttcttcgt 540

ttgccggtgt ggcagtcaca gcggccagtt gccaagctga cctgttcata accggcccca 600

ataaatgttt aggctgtccg ccaggattaa gcctggtggc ggtatctgat gctgcctggc 660

ggaaaatcga ggccaatgac gctgcaccgc gtgcgagcgt attatcgttg ttagactggc 720

gtgatgcctg gtcagctgaa aaaccttttc cctatacacc atcagtagcg gagatcaacg 780

gactggaagc agcgctggac ggttacttgc aggaggggcc agctcaggtc tgggcgcgcc 840

acagattaac cgcacaagca ttccgcgccg ggattcaggc aatgggactt agcttgtggg 900

cggctgatga aagtattgct tcaccaacgg caactgcgat acgcgttccc gacggagtaa 960

atgaagccca atggagactg cgcgcgcgtg agtgttatgg cgtgatgttt tcgtcagggc 1020

gcggtgaaac gctgggcaag gtattgcgtg tcggtcatat gggaccgact gcccaaccaa 1080

tgttcgcgat tatcgctctt acagcactgg cgggggcact taaccagtta agagagccgc 1140

aactggatat tggcgctggc gttgcagccg caatggctgt gataggttaa 1190

<210>  38
<211>  1178
<212>  DNA
<213>  Artificial Sequence

<220>
<223>  Artificial Fragment for Pyridoxamine Pyruvate Transaminase

<400>  38
acaaaaagga taaaacaatg gcacatcctg attttacact gagcgccggt ccggttaccg      60

ttaccgcacg cacgctggcg ggtttaggca gtccgattct gtatcattat gaccctgaat     120

ttttagcaac atttcgtcgc acgcagggta aagtggggca gatgttccaa accgataatg     180

acattatcct gatgcagggt gaagccattg ttggtcttga aggcgctttg cgttctctga     240

taacgcccgg gatgcatgtt ctgaacttag tgcagggagt atttggcaaa ggtaccggct     300

actggattgc cgatttcgga gctgtgctgc acgagatcga agttggctat gatgacgcgg     360

tctcaccggc acaagttgaa gagtatcttg atgcacatcc agaaattcag atggtttgtc     420

tggtggcctc cgagaccccg agtggtacag taacggatgt cgcggcaatt ggcccaatgt     480

gtcgggatcg tggaatcctg acttatatag atactgtttc tggcgtgttg ggtatgccct     540

ggaaaacaga cgaatggggc ctggatttat gcgtcgcagg agctcagaaa tgtctggggg     600

gccctcctgg tgttgcgctt atttcagtga gccaaaaggc atggatgtaa atgtacgcga     660

atccggccgc tccacgtgac tcatatttaa gtctgatcga ttggaaagag aaatggctgg     720

gtgaaggccg ctttccgtat acgccatctg tgagcgacat gaacggcttt gaagcagcgt     780

tagatcaggc actggaagag gggattgacg cggttgtcgc acgtcacgaa gccgctgccg     840

ctgtgacccg cgccggagct agagccatgg gtctggaact ttgggcgaag agcgaagaga     900

tagcatcggc ttgcgttact tccattcgtc tgcccgatga catcgataat gccgtggtac     960

gcgatcatgc tcgtgaagtc tacggggtga tgttatcaca cgggcagggt gccggcaact    1020

tggtacggtt atctcacatg ggtccgacag ctggtggcct gcattcggtg gtaggacttg    1080

ccgcgttagg ccgcaccttg gctgacctgg gaatgtccgt cgatataggt gcgggactgg    1140

aggcggcatt agcgctgttg agtactcaaa gacggtaa                            1178

<210>  39
<211>  6
<212>  PRT
<213>  Artificial Sequence

<220>
<223>  Specific Amino Acid Residues

<220>
<221>  misc_feature
<222>  (1)..(1)
<223>  Xaa is Leu, Met, Ile or Val

<220>
<221>  misc_feature
<222>  (2)..(2)
<223>  Xaa is His or Gln

<220>
<221>  misc_feature
<222>  (3)..(3)
<223>  Xaa is Gly, Cys or Ala

<220>
<221>  misc_feature
<222>  (4)..(4)
<223>  Xaa is Glu or Asp

<220>
<221>  misc_feature
<222>  (5)..(5)
<223>  Xaa is Pro or Ala

<220>
<221>  misc_feature
<222>  (6)..(6)
<223>  Xaa is Val, Ile, Leu or Ala

<400>  39

Xaa Xaa Xaa Xaa Xaa Xaa
1               5


<210>  40
<211>  9
<212>  PRT
<213>  Artificial Sequence

<220>
<223>  Specific Amino Acid Residues


<220>
<221>  misc_feature
<222>  (1)..(1)
<223>  Xaa is His or Ser

<220>
<221>  misc_feature
<222>  (2)..(2)
<223>  Xaa is Glu or Asp

<220>
<221>  misc_feature
<222>  (8)..(8)
<223>  Xaa is Ile, Val or Leu

<220>
<221>  misc_feature
<222>  (9)..(9)
<223>  Xaa is Asn or Thr

<400>  40

Xaa Xaa Thr Pro Ser Gly Thr Xaa Xaa
1               5

<210>  41
<211>  7
<212>  PRT
<213>  Artificial Sequence

<220>
<223>  Specific Amino Acid Residues

<220>
<221>  misc_feature
<222>  (1)..(1)
<223>  Xaa is Val, Ile or Ala

<220>
<221>  misc_feature
<222>  (3)..(3)
<223>  Xaa is Ala, Thr or Ser

<220>
<221>  misc_feature
<222>  (6)..(6)
<223>  Xaa is Ser, Ala or Gly

<220>
<221>  misc_feature
<222>  (7)..(7)
<223>  Xaa is Phe, Trp or Val

<400>  41

Xaa Asp Xaa Val Ser Xaa Xaa
1               5

<210>  42
<211>  10
<212>  PRT
<213>  Artificial Sequence

<220>
<223>  Specific Amino Acid Residues

<220>
<221>  misc_feature
<222>  (1)..(1)
<223>  Xaa is Gly or Ser

<220>
<221>  misc_feature
<222>  (2)..(2)
<223>  Xaa is Pro, Ser or Ala

<220>
<221>  misc_feature
<222>  (3)..(3)
<223>  Xaa is Asn, Gly, Ser, Ala or Gln

<220>
<221>  misc_feature
<222>  (6)..(6)
<223>  Xaa is Leu or Met

<220>
<221>  misc_feature
<222>  (8)..(8)
<223>  Xaa is Ala, Ser, Cys or Gly

<220>
<221>  misc_feature
<222>  (9)..(9)
<223>  Xaa is Pro, Thr, Ser or Ala

<400>  42

Xaa Xaa Xaa Lys Cys Xaa Gly Xaa Xaa Pro
1               5                   10


<210>  43
<211>  9
<212>  PRT
<213>  Artificial Sequence

<220>
<223>  Specific Amino Acid Residues


<220>
<221>  misc_feature
<222>  (1)..(1)
<223>  Xaa is Gly or Asp

<220>
<221>  misc_feature
<222>  (2)..(2)
<223>  Xaa is Val or Ile

<220>
<221>  misc_feature
<222>  (3)..(3)
<223>  Xaa is Val, Thr, Ala, Ser, Met, Ile or Leu

<220>
<221>  misc_feature
<222>  (4)..(4)
<223>  Xaa is Phe, Met, Leu, Ile or Val

<220>
<221>  misc_feature
<222>  (6)..(6)
<223>  Xaa is Ser, Gly, Ala, Thr, Ile, Leu or His

<220>
<221>  misc_feature
<222>  (8)..(8)
<223>  Xaa is Arg, Met or Gln

<220>
<221>  misc_feature
<222>  (9)..(9)

<223>  Xaa is Gly, Arg, Ala, Asp, His or Lys

<400>  43

Xaa Xaa Xaa Xaa Ser Xaa Gly Xaa Xaa
1               5

<210>  44
<211>  11
<212>  PRT
<213>  Artificial Sequence

<220>
<223>  Specific Amino Acid Residues

<220>
<221>  misc_feature
<222>  (1)..(1)
<223>  Xaa is Leu or Val

<220>
<221>  misc_feature
<222>  (2)..(2)
<223>  Xaa is Thr, Ile, Val or Leu

<220>
<221>  misc_feature
<222>  (4)..(4)
<223>  Xaa is Ile, Val or Leu

<220>
<221>  misc_feature
<222>  (5)..(5)
<223>  Xaa is Gly or Ser

<220>
<221>  misc_feature
<222>  (9)..(9)
<223>  Xaa is Pro, Ala or Arg

<220>
<221>  misc_feature
<222>  (10)..(10)
<223>  Xaa is Thr, Val or Ser

<400>  44

Xaa Xaa Arg Xaa Xaa His Met Gly Xaa Xaa Ala
1               5                   10

<210>  45
<211>  19
<212>  PRT
<213>  Artificial Sequence

<220>
<223>  Specific Amino Acid Residues

<220>

<221>  misc_feature
<222>  (1)..(1)
<223>  Xaa is Val, Leu, Ile or Met

<220>
<221>  misc_feature
<222>  (2)..(2)
<223>  Xaa is Ile, Leu or Val

<220>
<221>  misc_feature
<222>  (3)..(3)
<223>  Xaa is Leu, Met, Ile or Val

<220>
<221>  misc_feature
<222>  (4)..(4)
<223>  Xaa is His or Gln

<220>
<221>  misc_feature
<222>  (5)..(5)
<223>  Xaa is Gly, Cys or Ala

<220>
<221>  misc_feature
<222>  (6)..(6)
<223>  Xaa is Glu or Asp

<220>
<221>  misc_feature
<222>  (7)..(7)
<223>  Xaa is Pro or Ala

<220>
<221>  misc_feature
<222>  (8)..(8)
<223>  Xaa is Val, Ile, Ala or Leu

<220>
<221>  misc_feature
<222>  (9)..(9)
<223>  Xaa is Leu Met, Pro or Val

<220>
<221>  misc_feature
<222>  (10)..(10)
<223>  Xaa is Gly or Ala

<220>
<221>  misc_feature
<222>  (11)..(11)
<223>  Xaa is Leu or Ile

<220>
<221>  misc_feature
<222>  (12)..(12)
<223>  Xaa is Glu or Gln

<220>
<221>  misc_feature
<222>  (13)..(13)
<223>  Xaa is Ala or Gly

<220>
<221>  misc_feature
<222>  (14)..(14)
<223>  Xaa is Ala or Val

<220>
<221>  misc_feature
<222>  (15)..(15)
<223>  Xaa is Ala or Leu

<220>
<221>  misc_feature
<222>  (16)..(16)
<223>  Xaa is Ala, Leu, His or Tyr

<220>
<221>  misc_feature
<222>  (17)..(17)
<223>  Xaa is Ser, Gly or Ala

<220>
<221>  misc_feature
<222>  (18)..(18)
<223>  Xaa is Leu, Phe, Val or Ala

<220>
<221>  misc_feature
<222>  (19)..(19)
<223>  Xaa is Ile, Phe, Val or Leu

<400>  45

Xaa Xaa Xaa Xaa Xaa Xaa Xaa Xaa Xaa Xaa Xaa Xaa Xaa Xaa Xaa Xaa
1               5                   10                  15

Xaa Xaa Xaa

<210>  46
<211>  21
<212>  PRT
<213>  Artificial Sequence

<220>
<223>  Specific Amino Acid Residues

<220>
<221>  misc_feature
<222>  (1)..(1)
<223>  Xaa is Val, Ile, Leu or Met

<220>
<221>  misc_feature
<222>  (2)..(2)
<223>  Xaa is Val or Ile

<220>
<221>  misc_feature
<222>  (3)..(3)

<223>  Xaa is Ser, Ala, Val, Cys or Phe

<220>
<221>  misc_feature
<222>  (4)..(4)
<223>  Xaa is Val, Ile, Ala, Leu or Thr

<220>
<221>  misc_feature
<222>  (5)..(5)
<223>  Xaa is Cys or Val

<220>
<221>  misc_feature
<222>  (6)..(6)
<223>  Xaa is His, Asn or Ala

<220>
<221>  misc_feature
<222>  (7)..(7)
<223>  Xaa is His or Ser

<220>
<221>  misc_feature
<222>  (8)..(8)
<223>  Xaa is Asp or Glu

<220>
<221>  misc_feature
<222>  (14)..(14)
<223>  Xaa is Ile, Val or Leu

<220>
<221>  misc_feature
<222>  (15)..(15)
<223>  Xaa is Asn or Thr

<220>
<221>  misc_feature
<222>  (16)..(16)
<223>  Xaa is Pro or Asp

<220>
<221>  misc_feature
<222>  (17)..(17)
<223>  Xaa is Ile, Val, Leu or Ala

<220>
<221>  misc_feature
<222>  (18)..(18)
<223>  Xaa is Asp, Asn, Glu, Ala, Gly, Gln, Val, Arg or Pro

<220>
<221>  misc_feature
<222>  (19)..(19)
<223>  Xaa is Ala, Glu, Gln or Asp

<220>
<221>  misc_feature
<222>  (20)..(20)
<223>  Xaa is Ile or Leu

<220>

<221>  misc_feature
<222>  (21)..(21)
<223>  Xaa is Gly or Ala

<400>  46

Xaa Xaa Xaa Xaa Xaa Xaa Xaa Xaa Thr Pro Ser Gly Thr Xaa Xaa Xaa
1               5                   10                  15

Xaa Xaa Xaa Xaa Xaa
            20

<210>  47
<211>  15
<212>  PRT
<213>  Artificial Sequence

<220>
<223>  Specific Amino Acid Residues

<220>
<221>  misc_feature
<222>  (1)..(1)
<223>  Xaa is Gly, Asp or Ala

<220>
<221>  misc_feature
<222>  (2)..(2)
<223>  Xaa is Ala, Gly, Lys, Thr, Gln, Arg or Glu

<220>
<221>  misc_feature
<222>  (3)..(3)
<223>  Xaa is Tyr, Asn, Leu or Phe

<220>
<221>  misc_feature
<222>  (4)..(4)
<223>  Xaa is Leu, Phe, Met or Val

<220>
<221>  misc_feature
<222>  (5)..(5)
<223>  Xaa is Ile, Leu or Tyr

<220>
<221>  misc_feature
<222>  (6)..(6)
<223>  Xaa is Val, Ala or Ile

<220>
<221>  misc_feature
<222>  (8)..(8)
<223>  Xaa is Ala, Ser or Thr

<220>
<221>  misc_feature
<222>  (11)..(11)
<223>  Xaa is Ser, Ala or Gly

<220>
<221>  misc_feature
<222>  (12)..(12)
<223>  Xaa is Phe, Trp or Val

<220>
<221>  misc_feature
<222>  (13)..(13)
<223>  Xaa is Gly, Ala or Leu

<220>
<221>  misc_feature
<222>  (14)..(14)
<223>  Xaa is Gly or Ser

<220>
<221>  misc_feature
<222>  (15)..(15)
<223>  Xaa is Met, Val or Leu

<400>  47

Xaa Xaa Xaa Xaa Xaa Xaa Asp Xaa Val Ser Xaa Xaa Xaa Xaa Xaa
1               5                   10                  15


<210>  48
<211>  24
<212>  PRT
<213>  Artificial Sequence

<220>
<223>  Specific Amino Acid Residues


<220>
<221>  misc_feature
<222>  (1)..(1)
<223>  Xaa is Ala, Ser, Val or Ile

<220>
<221>  misc_feature
<222>  (2)..(2)
<223>  Xaa is Asp, Gly or Ala

<220>
<221>  misc_feature
<222>  (3)..(3)
<223>  Xaa is Ile, Leu, Phe, Val or Met

<220>
<221>  misc_feature
<222>  (4)..(4)
<223>  Xaa is Tyr, Phe, Leu or Cys

<220>
<221>  misc_feature
<222>  (5)..(5)
<223>  Xaa is Val or Ile

<220>
<221>  misc_feature
<222>  (6)..(6)

<223>  Xaa is Thr or Ala

<220>
<221>  misc_feature
<222>  (7)..(7)
<223>  Xaa is Gly or Ser

<220>
<221>  misc_feature
<222>  (8)..(8)
<223>  Xaa is Pro, Ser or Ala

<220>
<221>  misc_feature
<222>  (9)..(9)
<223>  Xaa is Asn, Gly, Ser, Gln or Ala

<220>
<221>  misc_feature
<222>  (12)..(12)
<223>  Xaa is Leu or Met

<220>
<221>  misc_feature
<222>  (14)..(14)
<223>  Xaa is Ala, Ser, Cys or Gly

<220>
<221>  misc_feature
<222>  (15)..(15)
<223>  Xaa is Pro, Thr, Ser or Ala

<220>
<221>  misc_feature
<222>  (17)..(17)
<223>  Xaa is Gly, Ala or Ser

<220>
<221>  misc_feature
<222>  (18)..(18)
<223>  Xaa is Leu or Val

<220>
<221>  misc_feature
<222>  (19)..(19)
<223>  Xaa is Thr, Ser or Ala

<220>
<221>  misc_feature
<222>  (20)..(20)
<223>  Xaa is Met, Ile, Leu, Val or Phe

<220>
<221>  misc_feature
<222>  (21)..(21)
<223>  Xaa is Met, Leu, Val, Ala or Ile

<220>
<221>  misc_feature
<222>  (22)..(22)
<223>  Xaa is Gly, Ala, His or Ser

<220>

<221>  misc_feature
<222>  (23)..(23)
<223>  Xaa is Val, Ile or Ala

<400>  48

Xaa Xaa Xaa Xaa Xaa Xaa Xaa Xaa Xaa Lys Cys Xaa Gly Xaa Xaa Pro
1               5                   10                  15

Xaa Xaa Xaa Xaa Xaa Xaa Xaa Ser
            20

<210>  49
<211>  10
<212>  PRT
<213>  Artificial Sequence

<220>
<223>  Specific Amino Acid Residues

<220>
<221>  misc_feature
<222>  (1)..(1)
<223>  Xaa is Tyr, Phe, His or Ser

<220>
<221>  misc_feature
<222>  (2)..(2)
<223>  Xaa is Gly or Asp

<220>
<221>  misc_feature
<222>  (3)..(3)
<223>  Xaa is Val or Ile

<220>
<221>  misc_feature
<222>  (4)..(4)
<223>  Xaa is Val, Thr, Ala, Ser, Met, Ile or Leu

<220>
<221>  misc_feature
<222>  (5)..(5)
<223>  Xaa is Phe, Met, Leu, Ile or Val

<220>
<221>  misc_feature
<222>  (7)..(7)
<223>  Xaa is Ser, Gly, Ala, Thr, Ile, Leu or His

<220>
<221>  misc_feature
<222>  (9)..(9)
<223>  Xaa is Arg, Met or Gln

<220>
<221>  misc_feature
<222>  (10)..(10)
<223>  Xaa is Gly, Arg, Ala, Asp, His or Lys

<400>  49

Xaa Xaa Xaa Xaa Xaa Ser Xaa Gly Xaa Xaa
1               5                   10


<210>  50
<211>  16
<212>  PRT
<213>  Artificial Sequence

<220>
<223>  Specific Amino Acid Residues


<220>
<221>  misc_feature
<222>  (1)..(1)
<223>  Xaa is Leu, Gln, Lys, Ala, Phe, Tyr or Trp

<220>
<221>  misc_feature
<222>  (2)..(2)
<223>  Xaa is Gly, Asn, His or Asp

<220>
<221>  misc_feature
<222>  (3)..(3)
<223>  Xaa is Lys, Arg or Asn

<220>
<221>  misc_feature
<222>  (4)..(4)
<223>  Xaa is Leu or Val

<220>
<221>  misc_feature
<222>  (5)..(5)
<223>  Xaa is Thr, Ile, Val or Leu

<220>
<221>  misc_feature
<222>  (7)..(7)
<223>  Xaa is Ile, Val or Leu

<220>
<221>  misc_feature
<222>  (8)..(8)
<223>  Xaa is Gly or Ser

<220>
<221>  misc_feature
<222>  (12)..(12)
<223>  Xaa is Pro, Ala or Arg

<220>
<221>  misc_feature
<222>  (13)..(13)
<223>  Xaa is Thr, Val or Ser

<220>
<221>  misc_feature
<222>  (15)..(15)

<223>  Xaa is Gln, Arg, Glu, Lys, His, Tyr or Gly

<220>
<221>  misc_feature
<222>  (16)..(16)
<223>  Xaa is Pro or Gly

<400>  50

Xaa Xaa Xaa Xaa Xaa Arg Xaa Xaa His Met Gly Xaa Xaa Ala Xaa Xaa
1               5                   10                  15


<210>  51
<211>  29
<212>  DNA
<213>  Artificial

<220>
<223>  Primer Sequence

<400>  51
ccggaattca caaaaaggat aaaacaatg                                    29


<210>  52
<211>  28
<212>  DNA
<213>  Artificial

<220>
<223>  Primer Sequence

<400>  52
cgcggatcct cacgcatccg cgtcgata                                     28


<210>  53
<211>  1055
<212>  DNA
<213>  Artificial

<220>
<223>  Artificial Fragment for Pyridoxine Dehydrogenase

<400>  53
acaaaaagga taaaacaatg tcagtagctg atctaaagaa caacatacac aaactagata      60

caggatatgg gctaatgagc ctgacctggc gtgcggaacc gatcccgcag agccaagcgt     120

tcgaggcgat gcaccgtgtg gttgagctga gccgtgaacg tggtcacaag gcgttcttta     180

acgtgggcga gttctacggc ccggacttta tcaacctgag ctacgttcac gatttctttg     240

cgaagtatcc ggacctgcgt aaagatgtgg ttattagctg caagggtggc gcggacaacg     300

cgaccctgac cccgcgtggc agccacgatg atgtggttca gagcgtgaaa aacagcgtta     360

gcgcgatcgg tggctatatc gacattttcg aggtggcgcg tattgatacc agcctgtgca     420

ccaaaggtga agtttacccg tatgagagct ttgaagcgct ggcggagatg atcagcgaag     480

gcgtgatcgg tggcattagc ctgagcgagg ttaacgagga acaaatccgt gcgattcaca     540

aggactgggg taaattcctg acctgcgttg aggtggaact gagcctgttt agcaacgata 600

tcctgcacaa cggtattgcg aagacctgcg cggaactggg cctgagcatc atttgctaca 660

gcccgctggg tcgtggcctg ctgaccggtc agctgaaaag caacgcggac atcccggagg 720

gcgatttccg taagagcctg aaacgtttta gcgacgaaag cctgaagaaa aacctgaccc 780

tggtgcgttt cctgcaagag gaaatcgttg ataagcgtcc gcagaacaac agcattaccc 840

tggcgcaact ggcgctgggt tgggtgaagc actggaacaa agttccggag tatagcggtg 900

cgaagtttat cccgattccg agcggcagca gcattagcaa agtgaacgag aacttcgacg 960

aacagaagac caaactgacc gatcaagaat ttaatgcgat taacaaatac ctgaccacct 1020

tccacaccgt tggcgaccgc tatgagatgg cgtaa 1055

<210> 54
<211> 34
<212> DNA
<213> Artificial

<220>
<223> Primer Sequence

<400> 54
acgcgtcgac acaaaaagga taaaacaatg tcag 34

<210> 55
<211> 29
<212> DNA
<213> Artificial

<220>
<223> Primer Sequence

<400> 55
cccaagcttt tacgccatct catagcggt 29

<210> 56
<211> 1133
<212> DNA
<213> Artificial

<220>
<223> Artificial Fragment for Alanine Dehydrogenase

<400> 56
acaaaaagga taaaacaatg ataataggag tacccacaga aataaagaac cacgaataca 60

gggttgggat ggtaccgagc agcgtgcgtg aactgaccat caagggccac gtggtttacg 120

ttcagagcga tgcgggcgtg ggtattggtt tcaccgacca agattatatc gatgcgggtg 180

cgagcattct ggcgaccgcg gcggaagtgt ttgcgaaaag cgacatgatc gttaaggtga 240

aagaaccgca ggcggtggaa cgtgcgatgc tgcgtcacga ccaaattctg ttcacctacc 300

tgcacctggc gccggatctg ccgcagaccg aggaactgat caccagcggt gcggtttgca 360

ttgcgtatga gaccgtgacc gatgatcgtg gtggcctgcc gctgctggcg ccgatgagcg 420

aggttgcggg tcgtatgagc atccaagcgg gtgcgcgtgc gctggagaag agcctgggtg 480

gtcgtggtat gctgctgggt ggcgttccgg gcgtggaacc ggcgaaggtg gttatcattg 540

gtggcggtat ggttggtacc aacgcggcgc agatggcggt gggtatgggt gcggatgtgg 600

ttgtgctggc gcgtagcatt gatgcgctgc gtcgtctgaa cgttcaattc ggcagcgcgg 660

tgaaagcgat ctacagcacc gcggacgcga ttgagcgtca cgttctggaa gcggatctgg 720

tgatcggcgg tgttctggtt ccgggtgcgg cggcgccgaa gctgattacc cgtgacatgg 780

ttaagcgtat gaaaccgggc agcgcgatcg ttgacgtggc gattgatcag ggcggttgcg 840

ttgaaaccag ccacgcgacc acccaccaag atccgaccta catcgtggac gatgttgtgc 900

actattgcgt tgcgaacatg ccgggtgcgg tggcgcgtac cagcaccttt gcgctgaaca 960

acgcgaccct gccgtacatc attaagctgg cgaaccaggg ctataaacaa gcgctgctga 1020

acgataagca cctgctgaac ggtctgaacg tgatgcacgg caaggttgtg tgcaaggaag 1080

tggcggaggc gctgaacctg gagtttaccg agccgaagag cctgctggcg taa 1133

<210> 57
<211> 29
<212> DNA
<213> Artificial

<220>
<223> Primer Sequence

<400> 57
cgcggatcca caaaaaggat aaaacaatg 29

<210> 58
<211> 30
<212> DNA
<213> Artificial

<220>
<223> Primer Sequence

<400> 58
acgcgtcgac ttacgccagc aggctcttcg 30